# EUROPEAN PATENT SPECIFICATION

(11) **EP 1 272 668 B1**
(45) Date of publication and mention of the grant of the patent: **14.02.2007**
(21) Application number: 01926549.5
(22) Date of filing: 02.04.2001
(51) Int. Cl.: C12Q 1/68, G01N 33/543

(54) **METHODS, COMPOSITIONS AND KITS FOR THE DETECTION AND MONITORING OF BREAST CANCER**
VERFAHREN, ZUSAMMENSETZUNGEN UND KITS ZUR IDENTIFIZIERUNG UND ÜBERWACHUNG VON BRUSTKREBS
METHODES, COMPOSITIONS ET NECESSAIRES CORRESPONDANTS SERVANT AU DEPISTAGE ET A LA SURVEILLANCE DU CANCER DU SEIN

(30) Priority: 03.04.2000 US 194241 P; 20.07.2000 US 219862 P; 27.07.2000 US 221300 P; 18.12.2000 US 256592 P
(43) Date of publication of application: 08.01.2003
(73) Proprietor: CORIXA CORPORATION, Hamilton, MT 59840-3131 (US)
(72) Inventor: HOUGHTON, Raymond, L., Bothell, WA 98021 (US); DILLON, Davin, C., Issaquah, WA 98027 (US); MOLESH, David, Alan, Kingston, WA 98346 (US); XU, Jiangchun, Bellevue, WA 98006 (US); ZEHENTNER, Barbara, Bainbridge Island, WA 98110 (US); PERSING, David, H., Redmond, WA 98053 (US)
(74) Representative: Walker, Ross Thomson
(86) International application number: PCT/US2001/010631
(87) International publication number: WO 2001/075171

(56) References cited:
- WO-A-98/04742
- WO-A-98/45328
- WO-A1-99/14230
- WO-A2-98/24928
- DE-A- 19 736 715
- DE-A1- 19 813 839
- XU ET AL: "Identification of differentially expressed genes in human breast tumor using substraction and microarray" CHEMICAL ABSTRACTS + INDEXES, AMERICAN CHEMICAL SOCIETY. COLUMBUS, US, vol. 40, March 1999 (1999-03), page 319 XP002160220 ISSN: 0009-2258
- XU JIANGCHUN ET AL: "Identification of differentially expressed genes in human prostate cancer using subtraction and microarray." CANCER RESEARCH, vol. 60, no. 6, 15 March 2000 (2000-03-15), pages 1677-1682, XP002198982 ISSN: 0008-5472
- JIANG YUQIU ET AL: "Discovery of differentially expressed genes in human breast cancer using subtracted cDNA libraries and cDNA microarrays." ONCOGENE, vol. 21, no. 14, 2002, pages 2270-2282, XP001075060 28 March, 2002 ISSN: 0950-9232
- ZEHENTNER B, DILLON DC, JIANG Y ET AL: "Application of a multigene reverse transcription PCR for the detection of mammaglobin and complimentary transcribed genes in breast cancer lymph nodes" CLINICAL CHEMISTRY, vol. 48, no. 8, August 2002 (2002-08), pages 1225-1231, US

## Description

### TECHNICAL FIELD OF THE INVENTION

The present invention relates generally to the field of cancer diagnostics. More specifically, the present invention relates to methods, compositions and kits for the detection of cancer that employ oligonucleotide hybridization and/or amplification to simultaneously detect two or more tissue-specific polynucleotides in a biological sample suspected of containing cancer cells.

### BACKGROUND OF THE INVENTION

Cancer remains a significant health problem throughout the world. The failure of conventional cancer treatment regimens can commonly be attributed, in part, to delayed disease diagnosis. Although significant advances have been made in the area of cancer diagnosis, there still remains a need for improved detection methodologies that permit early, reliable and sensitive determination of the presence of cancer cells.

Breast cancer is second only to lung cancer in mortality among women in the U.S., affecting more than 180,000 women each year and resulting in approximately 40,000-50,000 deaths annually. For women in North America, the lifetime odds of getting breast cancer are one in eight.

Management of the disease currently relies on a combination of early diagnosis (through routine breast screening procedures) and aggressive treatment, which may include one or more of a variety of treatments such as surgery, radiotherapy, chemotherapy and hormone therapy. The course of treatment for a particular breast cancer is often selected based on a variety of prognostic parameters, including analysis of specific tumor markers. *See, e.g.,* Porter-Jordan et al., *Breast Cancer* 8:73-100 (1994). The use of established markers often leads, however, to a result that is difficult to interpret; and the high mortality observed in breast cancer patients indicates that improvements are needed in the diagnosis of the disease.

The recent introduction of immunotherapeutic approaches to breast cancer treatment which are targeted to Her2/neu have provided significant motivation to identify additional breast cancer specific genes as targets for therapeutic antibodies and T-cell vaccines as well as for diagnosis of the disease. To this end, mammaglobin, has been identified as one of the most breast-specific genes discovered to date, being expressed in approximately 70-80% of breast cancers. Because of its highly tissue-specific distribution, detection of mammaglobin gene expression has been used to identify micrometastatic lesions in lymph node tissues and, more recently, to detect circulating breast cancer cells in peripheral blood of breast cancer patients with known primary and metastatic lesions.

Mammaglobin is a homologue of a rabbit uteroglobin and the rat steroid binding protein subunit C3 and is a low molecular weight protein that is highly glycosylated. Watson et al., *Cancer Res*. 56:860-5 (1996); Watson et al., *Cancer Res*. 59:3028-3031 (1999); Watson et al., *Oncogene* 16:817-24 (1998). In contrast to its homologs, mammaglobin has been reported to be breast specific and overexpression has been described in breast tumor biopsies (23%), primary and metastatic breast tumors (~75%) with reports of the detection of mammaglobin mRNA expression in 91% of lymph nodes from metastatic breast cancer patients. Leygue et al., *J. Pathol*. 189:28-33 (1999) and Min et al., *Cancer Res.* 58:4581-4584 (1998).

Since mammaglobin gene expression is not a universal feature of breast cancer, the detection of this gene alone may be insufficient to permit the reliable detection of all breast cancers. Accordingly, what is needed in the art is a methodology that employs the detection of two or more breast cancer specific genes in order to improve the sensitivity and reliability of detection of micrometastases, for example, in lymph nodes and bone marrow and/or for recognition of anchorage-independent cells in the peripheral circulation.

The present invention achieves these and other related objectives by providing methods that are useful for the identification of tissue-specific polynucleotides, in particular tumor-specific polynucleotides, as well as methods, compositions and kits for the detection and monitoring of cancer cells in a patient afflicted with the disease.

In a first aspect of the present invention there is provided a method for detecting the presence of a breast cancer cell, said method comprising the steps of:
(a) contacting a biological sample obtained from a patient with a first oligonucleotide that hybridizes to a first polynucleotide selected from the group consisting of mammaglobin and lipophilin B;
(b) contacting said biological sample with a second oligonucleotide that hybridizes to a second polynucleotide sequence comprising a GABAπ (B899P) polynucleotide sequence;
(c) detecting in said biological sample an amount of hybridized oligonucleotide of steps (a) and (b); and
(d) comparing the amount of polynucleotide that hybridizes to the oligonucleotides of steps (a) and (b) to a predetermined cut-off value, and therefrom determining the presence or absence of a cancer in the patient.

Preferably, said first polynucleotide is selected from the group consisting of polynucleotides depicted in SEQ ID NO:73, SEQ ID NO:74 and SEQ ID NO:76, or the complement thereof; and wherein said second polynucleotide comprises the polynucleotide set forth in SEQ ID NO: 75, or the complement thereof.

Advantageously, the method further comprises:
(e) contacting said biological sample with a third oligonucleotide that hybridizes to a third polynucleotide selected from the group consisting of polynucleotides depicted in SEQ ID NO:5, SEQ ID NO:6 and SEQ ID NO:7; or the complement thereof;
(f) contacting said biological sample with a fourth oligonucleotide that hybridizes to a fourth polynucleotide selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24; or the complement thereof and
   wherein step (c) comprises detecting in said biological sample an amount of hybridized oligonucleotide of steps (a), (b), (e), and (f).

Alternatively, the method further comprises:
(e) contacting said biological sample with a third oligonucleotide that hybridizes to a third polynucleotide selected from the group consisting of a polynucleotide depicted in SEQ ID NO:1, SEQ ID NO:3, SEQ ID NO:5, SEQ ID NO:6 and SEQ ID NO:7; or the complement thereof;
(f) contacting said biological sample with a fourth oligonucleotide that hybridizes to a fourth polynucleotide selected from the group consisting of a polynucleotide depicted in SEQ ID NO:11, or the complement thereof;
(g) contacting said biological sample with a fifth oligonucleotide that hybridizes to a fifth polynucleotide selected from the group consisting of a polynucleotide depicted in SEQ ID NO:13, 15 and 17; or the complement thereof;
(h) contacting said biological sample with a sixth oligonucleotide that hybridizes to a sixth polynucleotide selected from the group consisting of a polynucleotide depicted in SEQ ID NO:19, SEQ ID NO:20, SEQ ID NO:21, SEQ ID NO:22, SEQ ID NO:23 and SEQ ID NO:24; or the complement thereof;
(i) contacting said biological sample with a seventh oligonucleotide that hybridizes to a seventh polynucleotide depicted in SEQ ID NO:30 or the complement thereof;
(j) contacting said biological sample with an eighth oligonucleotide that hybridizes to an eighth polynucleotide depicted in SEQ ID NO:32 or the complement thereof; and
(k) contacting said biological sample with a ninth oligonucleotide that hybridizes to a polynucleotide depicted in SEQ ID NO:76 or the complement thereof;
wherein step (c) comprises detecting in said biological sample an amount of hybridized oligonucleotide of steps (a), (b), (e), (f), (g), (h), (i), (j), and (k).

In a second aspect of the present invention, there is provided a method for detecting the presence of a breast cancer cell, said method comprising the steps of:
(a) contacting a biological sample obtained from a patient with a first oligonucleotide pair said first pair comprising a first oligonucleotide and a second oligonucleotide wherein said first oligonucleotide and said second oligonucleotide hybridize to a first polynucleotide and the complement thereof, respectively, wherein said first polynucleotide comprises mammaglobin or lipophilin B;
(b) contacting said biological sample with a second oligonucleotide pair said second pair comprising a third oligonucleotide and a fourth oligonucleotide wherein said third and said fourth oligonucleotide hybridize to a second polynucleotide and the complement thereof, respectively, and wherein said second polynucleotide comprises GABAπ (B899P);
(c) amplifying said first polynucleotide and said second polynucleotide; and
(d) detecting said amplified first polynucleotide and said amplified second polynucleotide;
wherein the presence of said amplified first polynucleotide or said amplified second polynucleotide indicates the presence of a breast cancer cell in said patient.

Preferably, said first polynucleotide is selected from the group consisting of polynucleotides depicted in SEQ ID NO:73, SEQ ID NO:74 and SEQ ID NO:76; and wherein said second polynucleotide comprises the polynucleotide set forth in SEQ ID NO: 75. Advantageously, the method further comprises contacting said biological sample with a third oligonucleotide pair said third pair comprising a forward primer and a reverse primer that hybridize to a third polynucleotide and the complement thereof, respectively, wherein said third polynucleotide is selected from the group consisting of polynucleotides depicted in SEQ ID NO:5, SEQ ID NO:6 and SEQ ID NO:7; wherein step (d) further comprises amplifying said third polynucleotide; and wherein step (e) further comprises detecting said third polynucleotide. Further preferably, the method of further comprises contacting said biological sample with a fourth oligonucleotide pair said fourth pair comprising a forward primer and a reverse primer that hybridize to a fourth polynucleotide and the complement thereof, respectively, wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24; wherein step (d) further comprises amplifying said fourth polynucleotide; and wherein step (e) further comprises detecting said fourth polynucleotide.

More preferably, said oligonucleotides are selected from the group consisting of oligonucleotides depicted in SEQ ID N0s:33-38, 48-51, and 53-62.

Advantageously, step (e) comprises a step selected from the group consisting of detecting a radiolabel and detecting a fluorophore.

Preferably, said oligonucleotides are intron spanning oligonucleotides.

Advantageously, said intron spanning oligonucleotides are selected from the group consisting of oligonucleotides depicted in SEQ ID NOs:36-44 and 48-62.

Further preferably, detection of said amplified first, second, third, or fourth polynucleotide comprises contacting said amplified first, second, third or fourth polynucleotide with a labeled oligonucleotide probe that hybridizes, under moderately stringent conditions, to said first, second, third, or fourth polynucleotide. Preferably, said labeled oligonucleotide probe comprises a detectable moiety selected from the group consisting of a radiolabel and a fluorophore.

Preferably, the said step of amplifying is achieved by a polynucleotide amplification methodology selected from the group consisting of reverse transcription polymerase chain reaction (RT-PCR), inverse PCR, RACE, ligase chain reaction (LCR), Qbeta Replicase, isothermal amplification, strand displacement amplification (SDA), rolling chain reaction (RCR), cyclic probe reaction (CPR), transcription-based amplification systems (TAS), nucleic acid sequence based amplification (NASBA) and 3SR.

Preferably, the methods of the present invention further comprise a step of enriching said cancer cell from said biological sample prior to hybridizing said oligonucleotide primer(s). Further preferably, said step of enriching said cancer cell from said biological sample is achieved by a methodology selected from the group consisting of cell capture and cell depletion. Advantageously, cell capture is achieved by immunocapture, said immunocapture comprising the steps of:
(a) adsorbing an antibody to the surface of said cancer cells; and
(b) separating said antibody adsorbed cancer cells from the remainder of said biological sample. Preferably, said antibody is directed to an antigen selected from the group consisting of CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE and TCRαβ or is an antibody directed to a breast tumor antigen.

Further preferably, said antibody is a monoclonal antibody.

Advantageously, the said antibody is conjugated to magnetic beads or is formulated in a tetrameric antibody complex.

Preferably, cell depletion is achieved by a method comprising the steps of:
(a) cross-linking red cells and white cells, and
(b) fractionating said cross-linked red and white cells from the remainder of said biological sample.

Advantageously, in the methods of the present invention, said biological sample is selected from the group consisting of blood, serum, lymph node, bone marrow, sputum, urine and tumor biopsy sample. Preferably, said biological sample is selected from the group consisting of blood, a lymph node and bone marrow. Further preferably, said lymph node is a sentinel lymph node.

Further preferably, in the methods of the present invention, said step of detection comprises a step of fractionation.

In a further aspect of the present invention, there is provided a composition for detecting a breast cancer cell in a biological sample of a patient, said composition comprising:
(a) a first oligonucleotide; and
(b) a second oligonucleotide;
wherein said first oligonucleotide and said second oligonucleotide are at least 15 nucleotides in length and specifically hybridize to a first polynucleotide or the complement thereof and to a second polynucleotide or the complement thereof, respectively; wherein said first polynucleotide is selected from the group consisting of SEQ ID NO:73, SEQ ID NO:74 and SEQ ID NO:76; and wherein said second polynucleotide comprises the polynucleotide set forth in SEQ ID NO: 75.

Preferably, the composition further comprises a third and a fourth oligonucleotide wherein said third and said fourth oligonucleotide hybridize to a third polynucleotide or the complement thereof and a fourth polynucleotide or the complement thereof, respectively; wherein said third polynucleotide is selected from the group consisting of polynucleotides depicted in SEQ ID NO:5, SEQ ID NO:6 and SEQ ID NO:7; and wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24.

Further preferably, said oligonucleotides are selected from the group consisting of oligonucleotides as disclosed in SEQ ID NOs: 33-44, 48-62, and 72.

In another aspect of the present invention there is provided a composition for detecting a breast cancer cell in a biological sample of a patient, said composition comprising:
(a) a first oligonucleotide pair; and
(b) a second oligonucleotide pair;
wherein said first oligonucleotide pair and said second oligonucleotide pair are at least 10 nucleotides in length and specifically hybridize to a first polynucleotide or the complement thereof and to a second polynucleotide or the complement thereof, respectively; wherein said first polynucleotide is selected from the group consisting of SEQ ID NO:73, SEQ ID NO:74 and SEQ ID NO:76; and wherein said second polynucleotide comprises the polynucleotide set forth in SEQ ID NO: 75.

Preferably, the composition further comprises a third oligonucleotide pair and a fourth oligonucleotide pair wherein said third oligonucleotide pair and said fourth oligonucleotide pair hybridize to a third polynucleotide or the complement thereof and to a fourth polynucleotide or the complement thereof, respectively; wherein said third polynucleotide is selected from the group consisting of polynucleotides depicted in SEQ ID NO:5, SEQ ID NO:6 and SEQ ID NO:7; and wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24.

Advantageously, said oligonucleotides are selected from the group consisting of oligonucleotides as disclosed in SEQ ID NOs: 33-44, 48-62, and 72.

There is also provided a kit for the detection of breast cancer, including the compositions of the present invention.

The present disclosure provides methods for identifying one or more tissue-specific polynucleotides which methods comprise the steps of: (a) performing a genetic subtraction to identify a pool of polynucleotides from a tissue of interest; (b) performing a DNA microarray analysis to identify a first subset of said pool of polynucleotides of interest wherein each member polynucleotide of said first subset is at least two-fold over-expressed in said tissue of interest as compared to a control tissue; and (c) performing a quantitative polymerase chain reaction analysis on polynucleotides within said first subset to identify a second subset of polynucleotides that are at least two-fold over-expressed as compared to the control tissue. Preferred genetic subtractions are selected from the group consisting of differential display and cDNA subtraction and are described in further detail herein below.

The present disclosure provide methods of identifying a subset of polynucleotides showing concordant and/or complementary tissue-specific expression profiles in a tissue of interest. Such methods comprise the steps of, (a) performing an expression analysis selected from the group consisting of DNA microarray and quantitative PCR to identify a first polynucleotides that is at least two-fold over-expressed in a tissue of interest as compared to a control tissue; and (b) performing an expression analysis selected from the group consisting of DNA microarray and quantitative PCR to identify a first polynucleotides that is at least two-fold over-expressed in a tissue of interest as compared to a control tissue.

Further embodiments of the present disclosure provide methods for detecting the presence of a cancer cell in a patient. Such methods comprise the steps of: (a) obtaining a biological sample from the patient; (b) contacting biological sample with a first oligonucleotide pair wherein the members of the first oligonucleotide pair hybridize, under moderately stringent conditions, to a first polynucleotide and the complement thereof, respectively; (c) contacting the biological sample with a second oligonucleotide pair wherein the members of the second oligonucleotide pair hybridize, under moderately stringent conditions, to a second polynucleotide and the complement thereof, respectively and wherein the first polynucleotide is unrelated in nucleotide sequence to the second polynucleotide; (d) amplifying the first polynucleotide and the second polynucleotide; and (e) detecting the amplified first polynucleotide and the amplified second polynucleotide; wherein the presence of the amplified first polynucleotide or amplified second polynucleotide indicates the presence of a cancer cell in the patient

By some embodiments, detection of the amplified first and/or second polynucleotides may be preceded by a fractionation step such as, for example, gel electrophoresis. Alternatively or additionally, detection of the amplified first and/or second polynucleotides may be achieved by hybridization of a labeled oligonucleotide probe that hybridizes specifically, under moderately stringent conditions, to the first or second polynucleotide. Oligonucleotide labeling may be achieved by incorporating a radiolabeled nucleotide or by incorporating a fluorescent label.

In certain preferred embodiments, cells of a specific tissue type may be enriched from the biological sample prior to the steps of detection. Enrichment may be achieved by a methodology selected from the group consisting of cell capture and cell depletion. Exemplary cell capture methods include immunocapture and comprise the steps of: (a) adsorbing an antibody to a tissue-specific cell surface to cells said biological sample; (b) separating the antibody adsorbed tissue-specific cells from the remainder of the biological sample. Exemplary cell depletion may be achieved by cross-linking red cells and white cells followed by a subsequent fractionation step to remove the cross-linked cells.

Alternative embodiments of the present disclosure provide methods for determining the presence or absence of a cancer in a patient, comprising the steps of: (a) contacting a biological sample obtained from the patient with an oligonucleotide that hybridizes to a polynucleotide that encodes a breast tumor protein; (b) detecting in the sample a level of a polynucleotide (such as, for example, mRNA) that hybridizes to the oligonucleotide; and (c) comparing the level of polynucleotide that hybridizes to the oligonucleotide with a predetermined cut-off value, and therefrom determining the presence or absence of a cancer in the patient Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are disclosed for monitoring the progression of a cancer in a patient, comprising the steps of: (a) contacting a biological sample obtained from a patient with an oligonucleotide that hybridizes to a polynucleotide that encodes a breast tumor protein; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Certain embodiments of the present disclosure provide that the step of amplifying said first polynucleotide and said second polynucleotide is achieved by the polymerase chain reaction (PCR).

Within certain embodiments, the cancer cell to be detected may be selected from the group consisting of prostate cancer, breast cancer, colon cancer, ovarian cancer, lung cancer head & neck cancer, lymphoma, leukemia, melanoma, liver cancer, gastric cancer, kidney cancer, bladder cancer, pancreatic cancer and endometrial cancer. Still further embodiments of the present invention provide that the biological, sample is selected from the group consisting of blood, a lymph node and bone marrow. The lymph node may be a sentinel lymph node.

Within specific embodiments of the present disclosure it is provided that the first polynucleotide is selected from the group consisting of mammaglobin, lipophilin B, GABAπ (B899P), B726P, B511S, B533S, B305D and B311D. Other embodiments provide that the second polynucleotide is selected from the group consisting of mammaglobin, lipophilin B, GABAπ (B899P), B726P, B511S, B533S, B305D and B311D.

Alternate embodiments of the present invention provide methods for detecting the presence or absence of a cancer in a patient, comprising the steps of: (a) contacting a biological sample obtained from a patient with a first oligonucleotide that hybridizes to a polynucleotide selected from the group consisting of mammaglobin and lipophilin B; (b) contacting the biological sample with a second oligonucleotide that hybridizes to a polynucleotide sequence selected from the group consisting of GABAπ (B899P); (c) detecting in the sample an amount of a polynucleotide that hybridizes to at least one of the oligonucleotides; and (d) comparing the amount of polynucleotide that hybridizes to the oligonucleotide to a predetermined cut-off value, and therefrom determining the presence or absence of a cancer in the patient.

According to certain embodiments, oligonucleotides may be selected from those disclosed herein such as those presented in SEQ ID Nos:33-72. By other embodiments, the amount of polynucleotide that hybridizes to the oligonucleotide is determined using a polymerase chain reaction. Alternatively, the amount of polynucleotide that hybridizes to the oligonucleotide may be determined using a hybridization assay.

Still other embodiments of the present invention provide methods for determining the presence or absence of a cancer cell in a patient, comprising the steps of: (a) contacting a biological sample obtained from a patient with a first oligonucleotide that hybridizes to a polynucleotide selected from the group consisting of a polynucleotide depicted in SEQ ID NO:73 and SEQ ID NO:74 or complement thereof; (b) contacting the biological sample with a second oligonucleotide that hybridizes to a polynucleotide depicted in SEQ ID NO:75 or complement thereof; (c) contacting the biological sample with a third oligonucleotide that hybridizes to a polynucleotide selected from the group consisting of a polynucleotide depicted in SEQ ID NO:1, SEQ ID NO:3, SEQ ID NO:5, SEQ ID NO:6 and SEQ ID NO:7 or complement thereof; (d) contacting the biological sample with a fourth oligonucleotide that hybridizes to a polynucleotide selected from the group consisting of a polynucleotide depicted in SEQ ID NO:11 or complement thereof; (e) contacting the biological sample with a fifth oligonucleotide that hybridizes to a polynucleotide selected from the group consisting of a polynucleotide depicted in SEQ ID NO:13, 15 and 17 or complement thereof; (f) contacting the biological sample with a sixth oligonucleotide that hybridizes to a polynucleotide selected from the group consisting of a polynucleotide depicted in SEQ ID NO:19, SEQ ID NO:20, SEQ ID NO:21, SEQ ID NO:22, SEQ ID NO:23 and SEQ ID NO:24 or complement thereof; (g) contacting the biological sample with a seventh oligonucleotide that hybridizes to a polynucleotide depicted in SEQ ID NO:30 or complement thereof; (h) contacting the biological sample with an eighth oligonucleotide that hybridizes to a polynucleotide depicted in SEQ ID NO:32 or complement thereof; (i) contacting the biological sample with a ninth oligonucleotide that hybridizes to a polynucleotide depicted in SEQ ID NO:76 or complement thereof; (j) detecting in the sample a hybridized oligonucleotide of any one of steps (a) through (i); and (j) comparing the amount of polynucleotide that hybridizes to the oligonucleotide to a predetermined cut-off value, wherein the presence of a hybridized oligonucleotide in any one of steps (a) through (i) in excess of the pre-determined cut-off value indicates the presence of a cancer cell in the biological sample of said patient.

Other related embodiments of the present invention provide methods for determining the presence or absence of a cancer cell in a patient, comprising the steps of: (a) contacting a biological sample obtained from a patient with a first oligonucleotide and a second oligonucleotide wherein said first and second oligonucleotides hybridize under moderately stringent conditions to a first and a second polynucleotide selected from the group selected from the group consisting of SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ ID NO:22, SEQ ID NO:23, SEQ ID NO:24, SEQ ID NO:30, SEQ ID NO:32, and SEQ ID NO:76 and wherein said first polynucleotide is unrelated structurally to said second polynucleotide; (b) detecting in the sample said first and said second hybridized oligonucleotides; and (c) comparing the amount of polynucleotide that hybridizes to the oligonucleotide to a predetermined cut-off value, wherein the presence of a hybridized first oligonucleotide or a hybridized second oligonucleotide in excess of the pre-determined cut-off value indicates the presence of a cancer cell in the biological sample of said patient.

Other related embodiments of the present disclosure provide methods for determining the presence or absence of a cancer cell in a patient, comprising the steps of: (a) contacting a biological sample obtained from a patient with a first oligonucleotide and a second oligonucleotide wherein said first and second oligonucleotides hybridize under moderately stringent conditions to a first and a second polynucleotide are both tissue-specific polynucleotides of the cancer to be detected and wherein said first polynucleotide is unrelated structurally, to said second polynucleotide; (b) detecting in the sample said first and said second hybridized oligonucleotides; and (c) comparing the amount of polynucleotide that hybridizes to the oligonucleotide to a predetermined cut-off value, wherein the presence of a hybridized first oligonucleotide or a hybridized second oligonucleotide in excess of the pre-determined cut-off value indicates the presence of a cancer cell in the biological sample of said patient.

In other related aspects, the present invention further provides compositions useful in the methods disclosed herein. Exemplary compositions comprise two or more oligonucleotide primer pairs each one of which specifically hybridizes to a distinct polynucleotide. Exemplary oligonucleotide primers suitable for compositions of the present invention are disclosed herein by SEQ ID NOs: 33-71. Exemplary polynucleotides suitable for compositions of the present invention are disclosed in SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ, ID NO:22, SEQ ID NO:23, SEQ ID NO:24, SEQ ID NO:30, SEQ ID NO:32, and SEQ ID NO:76.

The present invention also provides kits that are suitable for performing the detection methods of the present invention. Exemplary kits comprise oligonucleotide primer pairs each one of which specifically hybridizes to a distinct polynucleotide. Within certain embodiments, kits according to the present invention may also comprise a nucleic acid polymerase and suitable buffer. Exemplary oligonucleotide primers suitable for kits of the present invention are disclosed herein by SEQ ID NOs: 33-71. Exemplary polynucleotides suitable for kits of the present invention are disclosed in SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ ID NO:22, SEQ ID NO:23, SEQ ID NO:24, SEQ ID NO:30, SEQ ID NO:32, and SEQ ID NO:76.

These and other aspects of the present invention will become apparent upon reference to the following detailed description and attached drawings.

### BRIEF DESCRIPTION OF THE DRAWINGS AND SEQUENCE IDENTIFIERS

Figure 1 shows the mRNA expression profiles for B311D, B533S and B726P as determined using quantitative PCR (Taqman^{™}). Abbreviations: B.T.: Breast tumor; B.M.: Bone marrow; B.R.: Breast reduction.
Figure 2 shows the relationship of B533S expression to pathological stage of tumor. Tissues from normal breast (8), benign breast disorders (3), and breast tumors stage I (5), stage II (6), stage III (7), stage IV (3) and metastases (1 lymph node and 3 pleural effusions) were tested in real-time PCR. The data is expressed as the mean copies/ng β-actin for each group tested and the line is the calculated trend line.
Figures 3A and 3B show the gene complementation of B305D C-form, B726P, GABAπ and mammaglobin in metastases and primary tumors, respectively. The cut-off for each of the genes was 6.57, 1.65, 4.58 and 3.56 copies/ng β-Actin based on the mean of the negative normal tissues plus 3 standard deviations.
Figure 4 shows the full-length cDNA sequence for mammeglobin.
Figure 5 shows the determined cDNA sequence of the open reading frame encoding a mammaglobin recombinant polypeptide expressed in *E*. *coli*.
Figure 6 shows the full-length cDNA sequence for GABAπ.
Figure 7 shows the mRNA expression levels for mammaglobin, GABAπ, B305D (C form) and B726P in breast tumor and normal samples determined using real-time PCR and the SYBR detection system. Abbreviations: BT: Breast tumor; BR: Breast reduction; A. PBMC: Activated peripheral blood mononuclear cells; R PBMC: resting PBMC; T. Gland: Thyroid gland; S. Cord: Spinal Cord; A. Gland: Adrenal gland; B. Marrow: Bone marrow; S. Muscle: Skeletal muscle.
Figure 8 is a bar graph showing a comparison between the LipophilinB alone and the LipophilinB-B899P-B305D-C-B726 multiplex assays tested on a panel of breast tumor samples. Abbreviations: BT: Breast tumor; BR: Breast reduction; SCID: severe combined immunodeficiency.
Figure 9 is a gel showing the unique band length of four amplification products of tumor genes of interest (mammaglobin, B305D, B899P, B726P) tested in a multiplex Real-time PCR assay.
Figure 10 shows a comparison of a multiplex assay using intron-exon border spanning primers (bottom panel) and those using non-optimized primers (top panel), to detect breast cancer cells in a panel of lymph node tissues.
SEQ ID NO: 1 is the determined cDNA sequence for a first splice variant of B305D isoform A.
SEQ ID NO: 2 is the amino acid sequence encoded by the sequence of SEQ ID NO: 1.
SEQ ID NO: 3 is the determined cDNA sequence for a second splice variant of B305D isoform A.
SEQ ID NO: 4 is the amino acid sequence encoded by the sequence of SEQ ID NO: 3.
SEQ ID NO: 5-7 are the determined cDNA sequences for three splice variants of B305D isoform C.
SEQ ID NO: 8-10 are the amino acid sequences encoded by the sequence of SEQ ID NO: 5-7, respectively.
SEQ ID NO: 11 is the determined cDNA sequence for B311D.
SEQ ID NO: 12 is the amino acid sequence encoded by the sequence of SEQ ID NO: 11.
SEQ ID NO: 13 is the determined cDNA sequence of a first splice variant of B726P.
SEQ ID NO: 14 is the amino acid sequence encoded by the sequence of SEQ ID NO: 13.
SEQ ID NO: 15 is the determined cDNA sequence of a second splice variant of B726P.
SEQ ID NO: 16 is the amino acid sequence encoded by the sequence of SEQ ID NO: 15.
SEQ ID NO: 17 is the determined cDNA sequence of a third splice variant of B726P.
SEQ ID NO: 18 is the amino acid sequence encoded by the sequence of SEQ ID NO: 17.
SEQ ID NO: 19-24 are the determined cDNA sequences of further splice variants of B726P.
SEQ ID NO: 25-29 are the amino acid sequences encoded by SEQ ID NO: 19-24, respectively.
SEQ ID NO: 30 is the determined cDNA sequence for B511S.
SEQ ID NO: 31 is the amino acid sequence encoded by SEQ ID NO: 30.
SEQ ID NO: 32 is the determined cDNA sequence for B533S.
SEQ ID NO:33 is the DNA sequence of Lipophilin B forward primer.
SEQ ID NO:34 is the DNA sequence of Lipophilin B reverse primer.
SEQ ID NO:35 is the DNA sequence of Lipophilin B probe.
SEQ ID NO:36 is the DNA sequence of GABA (B899P) forward primer.
SEQ ID NO:37 is the DNA sequence of GABA (B899P) reverse primer.
SEQ ID NO:38 is the DNA sequence of GABA (B899P) probe.
SEQ ID NO:39 is the DNA sequence of B305D (C form) forward primer.
SEQ ID NO:40 is the DNA sequence of B305D (C form) reverse primer.
SEQ ID NO:41 is the DNA sequence of B305D (C form) probe.
SEQ ID NO:42 is the DNA sequence of B726P forward primer.
SEQ ID NO:43 is the DNA sequence of B726P reverse primer.
SEQ ID NO:44 is the DNA sequence of B726P probe.
SEQ ID NO:45 is the DNA sequence of Actin forward primer.
SEQ ID NO:46 is the DNA sequence of Actin reverse primer.
SEQ ID NO:47 is the DNA sequence of Actin probe.
SEQ ID NO:48 is the DNA sequence of Mammaglobin forward primer.
SEQ ID NO:49 is the DNA sequence of Mammaglobin reverse primer.
SEQ ID NO:50 is the DNA sequence of Mammaglobin probe.
SEQ ID NO:51 is the DNA sequence of a second GABA (B899P) reverse primer.
SEQ ID NO:52 is the DNA sequence of a second B726P forward primer.
SEQ ID NO:53 is the DNA sequence of a GABA B899P-INT forward primer.
SEQ ID NO:54 is the DNA sequence of a GABA B899P-INT reverse primer.
SEQ ID NO:55 is the DNA sequence of a GABA B899P-INT Taqman probe.
SEQ ID NO:56 is the DNA sequence of a B305D-INT forward primer.
SEQ ID NO:57 is the DNA sequence of a B305D-INT reverse primer.
SEQ ID NO:58 is the DNA sequence of a B305D-INT Taqman probe.
SEQ ID NO:59 is the DNA sequence of a B726-INT forward primer.
SEQ ID NO:60 is the DNA sequence of a B726-INT reverse primer.
SEQ ID NO:61 is the DNA sequence of a B726-INT Taqman probe.
SEQ ID NO:62 is the DNA sequence of a GABA B899P Taqman probe.
SEQ ID NO:63 is the DNA sequence of a B311D forward primer.
SEQ ID NO:64 is the DNA sequence of a B311 D reverse primer.
SEQ ID NO:65 is the DNA sequence of a B311 D Taqman probe.
SEQ ID NO:66 is the DNA sequence of a B533S forward primer.
SEQ ID NO:67 is the DNA sequence of a B533S reverse primer.
SEQ ID NO:68 is the DNA sequence of a B533S Taqman probe.
SEQ ID NO:69 is the DNA sequence of a B511S forward primer.
SEQ ID NO:70 is the DNA sequence of a B511S reverse primer.
SEQ ID NO:71 is the DNA sequence of a B511S Taqman probe.
SEQ ID NO:72 is the DNA sequence of a GABAπ reverse primer.
SEQ ID NO:73 is the full-length cDNA sequence for mammaglobin.
SEQ ID NO:74 is the determined cDNA sequence of the open reading frame encoding a mammaglobin recombinant polypeptide expressed in *E. coli.*
SEQ ID NO:75 is the full-length cDNA sequence for GABAπ.
SEQ ID NO:76 is the full-length cDNA sequence for lipophilin B.
SEQ ID NO:77 is the amino acid sequence encoded by the sequence of SEQ ID NO:76.

### DETAILED DESCRIPTION OF THE INVENTION

As noted above, the present invention is directed generally to methods that are suitable for the identification of tissue-specific polynucleotides as well as to methods, compositions and kits that are suitable for the diagnosis and monitoring of breast cancer.

### Identification of Tissue-specific Polynucleotides

Certain embodiments of the present invention provide methods, compositions and kits for the detection of a breast cancer cell within a biological sample. These methods comprise the step of detecting one or more tissue-specific polynucleotide(s) from a patient's biological sample the over-expression of which polynucleotides indicates the presence of a cancer cell within the patient's biological sample. Accordingly; the present invention also provides methods that are suitable for the identification of tissue-specific polynucleotides. As used herein, the phrases . "tissue-specific polynucleotides" or "tumor-specific polynucleotides" are meant to include all polynucleotides that are at least two-fold over-expressed as compared to one or more control tissues. As discussed in further detail herein below, over-expression of a given polynucleotide may be assessed, for example, by microarray and/or quantitative real-time polymerase chain reaction (Real-time PCR^{™}) methodologies.

Exemplary methods for detecting tissue-specific polynucleotides may comprise the steps of: (a) performing a genetic subtraction to identify a pool of polynucleotides from a tissue of interest; (b) performing a DNA microarray analysis to identify a first subset of said pool of polynucleotides of interest wherein each member polynucleotide of said first subset is at least two-fold over-expressed in said tissue of interest as compared to a control tissue; and (c) performing a quantitative polymerase chain reaction analysis on polynucleotides within said first subset to identify a second subset of polynucleotides that are at least two-fold over-expressed as compared to said control tissue.

### Polynucleotides Generally

As used herein, the term "polynucleotide" refers generally to either DNA or RNA molecules. Polynucleotides may be naturally occurring as normally found in a biological sample such as blood, serum, lymph node, bone marrow, sputum, urine and tumor biopsy samples. Alternatively, polynucleotides may be derived synthetically by, for example, a nucleic acid polymerization reaction. As will be recognized by the skilled artisan, polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e.* an endogenous sequence that encodes a tumor protein, such as a breast tumor protein, or a portion thereof) or may comprise a variant, or a biological or antigenic functional equivalent of such a sequence. Polynucleotide variants may contain one or more substitutions, additions, deletions and/or insertions, as further described below. The term "variants" also encompasses homologous genes of xenogenic origin.

When comparing polynucleotide or polypeptide sequences, two sequences are said to be "identical" if the sequence of nucleotides or amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) *CABIOS 5*:151-153; Myers, E.W. and Muller W. (1988) *CABIOS 4*:11-17; Robinson, E.D. (1971) *Comb. Theor 11*:105; Santou, N. Nes, M. (1987) *Mol. Biol. Evol. 4*:406-425; Sneath, P.H.A. and Sokal, R.R. (1973) *Numerical Taxonomy - the Principles and Practice of Numerical Taxonomy,* Freeman Press, San Francisco, CA; Wilbur, W.J. and Lipman, D.J. (1983) *Proc. Natl. Acad, Sci. USA 80*:726-730.

Alternatively, optimal alignment of sequences for comparison may be conducted by the local identity algorithm of Smith and Waterman (1981) *Add APL. Math 2*:482, by the identity alignment algorithm of Needleman and Wunsch (1970) *J. Mol. Biol.* 48:443, by the search for similarity methods of Pearson and Lipman (1988) *Proc. Natl. Acad Sci. USA* 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul *et al.* (1977) *Nucl. Acids Res.* 25:3389-3402 and Altschul *et al*. (1990) *J. Mol. Biol*. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) *Proc. Natl. Acad Sci. USA* 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (*i*.*e*., the window size) and multiplying the results by 100 to yield the percentage of sequence identity.

Therefore, the present invention encompasses polynucleotide and polypeptide sequences having substantial identity to the sequences disclosed herein, for example those comprising at least 50% sequence identity, preferably at least 55%, 60%, 65%, 70%, 75%, 80%, 85%, 90%, 95%, 96%, 97%, 98%, or 99% or higher, sequence identity compared to a polynucleotide or polypeptide sequence of this invention using the methods described herein, (*e.g.*, BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

In additional embodiments, the present invention provides isolated polynucleotides and polypeptides comprising various lengths of contiguous stretches of sequence identical to or complementary to one or more of the sequences disclosed herein. For example, polynucleotides are provided by this invention that comprise at least about 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc*.; 21, 22, 23, *etc.;* 30, 31, 32, *etc.;* 50, 51, 52, 53, *etc.;* 100, 101, 102, 103, *etc.;* 150, 151, 152, 153, *etc*.; including all integers through 200-500; 500-1,000, and the like.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative DNA segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

In other embodiments, the present invention is directed to polynucleotides that are capable of hybridizing under moderately stringent conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

Moreover, it will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

### Microarray Analyses

Polynucleotides that are suitable for detection according to the methods of the present invention may be identified, as described in more detail below, by screening a microarray of cDNAs for tissue and/or tumor-associated expression (*e*.*g*., expression that is at least two-fold greater in a tumor than in normal tissue, as determined using a representative assay provided herein). Such screens may be performed, for example, using a Synteni microarray (Palo Alto, CA) according to the manufacturer's instructions (and essentially as described by Schena *et al., Proc. Natl. Acad Sci. USA* 93:10614-10619 (1996) and Heller *et al., Proc. Natl. Acad. Sci. USA* 94:2150-2155 (1997)).

Microarray is an effective method for evaluating large numbers of genes but due to its limited sensitivity it may not accurately determine the absolute tissue distribution of low abundance genes or may underestimate the degree of overexpression of more abundant genes due to signal saturation. For those genes showing overexpression by microarray expression profiling, further analysis was performed using quantitative RT-PCR based on Taqman^{™} probe detection, which comprises a greater dynamic range of sensitivity. Several different panels of normal and tumor tissues, distant metastases and cell lines were used for this purpose.

### Quantitative Real-time Polymerase Chain Reaction

Suitable polynucleotides according to the present invention may be further characterized or, alternatively, originally identified by employing a quantitative PCR methodology such as, for example, the Real-time PCR methodology. By this methodology, tissue and/or tumor samples, such as, *e.g.*, metastatic tumor samples, may be tested along side the corresponding normal tissue sample and/or a panel of unrelated normal tissue samples.

Real-time PCR (*see* Gibson et al., *Genome Research 6*:995-1001, 1996; Heid et al., *Genome Research 6*:986-994, 1996) is a technique that evaluates the level of PCR product accumulation during amplification. This technique permits quantitative evaluation of mRNA levels in multiple samples. Briefly, mRNA is extracted from tumor and normal tissue and cDNA is prepared using standard techniques.

Real-time PCR may, for example, be performed either on the ABI 7700 Prism or on a GeneAmp® 5700 sequence detection system (PE Biosystems, Foster City, CA). The 7700 system uses a forward and a reverse primer in combination with a specific probe with a 5' fluorescent reporter dye at one end and a 3' quencher dye at the other end (Taqman™). When the Real-time PCR is performed using Taq DNA polymerase with 5'-3' nuclease activity, the probe is cleaved and begins to fluoresce allowing the reaction to be monitored by the increase in fluorescence (Real-time). The 5700 system uses SYBR® green, a fluorescent dye, that only binds to double stranded DNA, and the same forward and reverse primers as the 7700 instrument. Matching primers and fluorescent probes may be designed according to the primer express program (PE Biosystems, Foster City, CA). Optimal concentrations of primers and probes are initially determined by those of ordinary skill in the art. Control (*e.g*., β-actin) primers and probes may be obtained commercially from, for example, Perkin Elmer/Applied Biosystems (Foster City, CA).

To quantitate the amount of specific RNA in a sample, a standard curve is generated using a plasmid containing the gene of interest. Standard curves are generated using the Ct values determined in the real-time PCR, which are related to the initial cDNA concentration used in the assay. Standard dilutions ranging from 10-10⁶ copies of the gene of interest are generally sufficient. In addition, a standard curve is generated for the control sequence. This permits standardization of initial RNA content of a tissue sample to the amount of control for comparison purposes.

In accordance with the above, and as described further below, the present invention provides the illustrative breast tissue- and/or tumor-specific polynucleotides mammaglobin, lipophilin B, GABAπ (B899P), B726P, B511S, B533S, B305D and B311D having sequences set forth in SEQ ID NO: 1, 3, 5-7, 11, 13, 15, 17, 19-24, 30, 32, and 73-76 illustrative polypeptides encoded thereby having amino acid sequences set forth in SEQ ID NO: 2, 4, 8-10, 12, 14, 16, 18, 25-29 and 31 and 77 that may be suitably employed in the detection of cancer, more specifically, breast cancer.

The methods disclosed herein will also permit the identification of additional and/or alternative polynucleotides that are suitable for the detection of a wide range of cancers including, but not limited to, prostate cancer, breast cancer, colon cancer, ovarian cancer, lung cancer head & neck cancer, lymphoma, leukemia, melanoma, liver cancer, gastric cancer, kidney cancer, bladder cancer, pancreatic cancer and endometrial cancer.

### Methodologies for the Detection of Cancer

In general, a cancer cell may be detected in a patient based on the presence of one or more polynucleotides within cells of a biological sample (for example, blood, lymph nodes, bone marrow, sera, sputum, urine and/or tumor biopsies) obtained from the patient. In other words, such polynucleotides may be used as markers to indicate the presence or absence of a cancer such as, *e.g.*, breast cancer.

As discussed in further detail herein, the present invention achieves these and other related objectives by providing a methodology for the simultaneous detection of more than one polynucleotide, the presence of which is diagnostic of the presence of cancer cells in a biological sample. Each of the various cancer detection methodologies disclosed herein have in common a step of hybridizing one or more oligonucleotide primers and/or probes, the hybridization of which is demonstrative of the presence of a tumor- and/or tissue-specific polynucleotide. Depending on the precise application contemplated, it may be preferred to employ one or more intron-spanning oligonucleotides that are inoperative against polynucleotide of genomic DNA and, thus, these oligonucleotides are effective in substantially reducing and/or eliminating the detection of genomic DNA in the biological sample.

Further disclosed herein are methods for enhancing the sensitivity of these detection methodologies by subjecting the biological samples to be tested to one or more cell capture and/or cell depletion methodologies.

By certain embodiments of the present invention, the presence of a cancer cell in a patient may be determined by employing the following steps: (a) obtaining a biological sample from said patient; (b) contacting said biological sample with a first oligonucleotide that hybridizes to a first polynucleotide said first polynucleotide selected from the group consisting of polynucleotides depicted in SEQ ID NO:73 and SEQ ID NO:74; (c) contacting said biological sample with a second oligonucleotide that hybridizes to a second polynucleotide selected from the group consisting of SEQ ID NO: 1, 3, 5-7, 11, 13, 15, 17, 19-24, 30, 32, and 75; (d) detecting in said sample an amount of a polynucleotide that hybridizes to at least one of the oligonucleotides; and (e) comparing the amount of the polynucleotide that hybridizes to said oligonucleotide to a predetermined cut-off value, and therefrom determining the presence or absence of a cancer in the patient.

Alternative embodiments of the present invention provide methods wherein the presence of a cancer cell in a patient is determined by employing the steps of: (a) obtaining a biological sample from said patient; (b) contacting said biological sample with a first oligonucleotide that hybridizes to a first polynucleotide said first polynucleotide depicted in SEQ ID NO:76; (c) contacting said biological sample with a second oligonucleotide that hybridizes to a second polynucleotide selected from the group consisting of SEQ ID NO: 1, 3, 5-7, 11, 13, 15, 17, 19-24, 30, 32, and 75; (d) detecting in said sample an amount of a polynucleotide that hybridizes to at least one of the oligonucleotides; and (e) comparing the amount of the polynucleotide that hybridizes to said oligonucleotide to a predetermined cut-off value, and therefrom determining the presence or absence of a cancer in the patient.

Other embodiments of the present invention provide methods for determining the presence or absence of a cancer in a patient. Such methods comprise the steps of: (a) obtaining a biological sample from said patient; (b) contacting said biological sample obtained from a patient with a first oligonucleotide that hybridizes to a polynucleotide sequence selected from the group consisting of polynucleotides depicted in SEQ ID NO:73, SEQ ID NO:74 and SEQ ID NO:76; (c) contacting said biological sample with a second oligonucleotide that hybridizes to a polynucleotide as depicted in SEQ ID NO:75; (d) contacting said biological sample with a third oligonucleotide that hybridizes to a polynucleotide selected from the group consisting of polynucleotides depicted in SEQ ID NO:5, SEQ ID NO:6 and SEQ ID NO:7; (e) contacting said biological sample with a fourth oligonucleotide that hybridizes to a polynucleotide selected from the group consisting of polynucleotides depicted in SEQ ID NO:13, SEQ ID NO:15, SEQ ID NO:17, SEQ ID NO:19, SEQ ID NO:20; SEQ ID NO:21, SEQ ID NO:22, SEQ ID NO:23 and SEQ ID NO:24; (f)detecting in said biological sample an amount of a polynucleotide that hybridizes to at least one of said oligonucleotides; and (g) comparing the amount of polynucleotide that hybridizes to the oligonucleotide to a predetermined cut-off value, and therefrom determining the presence or absence of a cancer in the patient.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a breast tumor protein that is at least 10 nucleotides, and preferably at least 20 nucleotides, in length. Preferably, oligonucleotide primers hybridize to a polynucleotide encoding a polypeptide described herein under moderately stringent conditions, as defined above. Oligonucleotide primers which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence recited in SEQ ID NO: 1, 3, 5-7, 11, 13, 15, 17, 19-24, 30, 32 and 73-76. Techniques for both PCR based assays and hybridization assays are well known in the art (*see,* for example, Mullis *et al., Cold Spring Harbor Symp. Quant. Biol., 51*:263, 1987; Erlich ed., *PCR Technology,* Stockton Press, NY, 1989).

The present invention also provides amplification-based methods for detecting the presence of a cancer cell in a patient. Exemplary methods comprise the steps of (a) obtaining a biological sample from a patient; (b) contacting the biological sample with a first oligonucleotide pair the first pair comprising a first oligonucleotide and a second oligonucleotide wherein the first oligonucleotide and the second oligonucleotide hybridize to a first polynucleotide and the complement thereof, respectively; (c) contacting the biological sample with a second oligonucleotide pair the second pair comprising a third oligonucleotide and a fourth oligonucleotide wherein the third and the fourth oligonucleotide hybridize to a second polynucleotide and the complement thereof, respectively, and wherein the first polynucleotide is unrelated in nucleotide sequence to the second polynucleotide; (d) amplifying the first polynucleotide and the second polynucleotide; and (e) detecting the amplified first polynucleotide and the amplified second polynucleotide; wherein the presence of the amplified first polynucleotide or the amplified second polynucleotide indicates the presence of a cancer cell in the patient.

Methods according to the present invention are suitable for identifying polynucleotides obtained from a wide variety of biological sample such as, for example, blood, serum, lymph node, bone marrow, sputum, urine and tumor biopsy sample. In certain preferred embodiments, the biological sample is either blood, a lymph node or bone marrow. In other embodiments of the present invention, the lymph node may be a sentinel lymph node.

It will be apparent that the present methods may be employed in the detection of a wide variety of cancers. Exemplary cancers include, but are not limited to, prostate cancer, breast cancer, colon cancer, ovarian cancer, lung cancer head & neck cancer, lymphoma, leukemia, melanoma, liver cancer, gastric cancer, kidney cancer, bladder cancer, pancreatic cancer and endometrial cancer.

Certain exemplary embodiments of the present invention provide methods wherein the polynucleotides to be detected are selected from the group consisting of mammaglobin, lipophilin B, GABAπ (B899P), B726P, B511S, B533S, B305D and B311D. Alternatively and/or additionally, polynucleotides to be detected may be selected from the group consisting of those depicted in SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ ID NO:22, SEQ ID NO:23, SEQ ID NO:24, SEQ ID NO:30, SEQ ID NO:32, and SEQ ID NO:76.

Suitable exemplary oligonucleotide probes and/or primers that may be used according to the methods of the present invention are disclosed herein by SEQ ID N0s:33-35 and 63-72. In certain preferred embodiments that eliminate the background detection of genomic DNA, the oligonucleotides may be intron spanning oligonucleotides. Exemplary intron spanning oligonucleotides suitable for the detection of various polynucleotides disclosed herein are depicted in SEQ ID NOs:36-62.

Depending on the precise application contemplated, the artisan may prefer to detect the tissue- and/or tumor-specific polynucleotides by detecting a radiolabel and detecting a fluorophore. More specifically, the oligonucleotide probe and/or primer may comprises a detectable moiety such as, for example, a radiolabel and/or a fluorophore.

Alternatively or additionally, methods of the present invention may also comprise a step of fractionation prior to detection of the tissue- and/or tumor-specific polynucleotides such as, for example, by gel electrophoresis.

In other embodiments, methods described herein may be used as to monitor the progression of cancer. By these embodiments, assays as provided for the diagnosis of a cancer may be performed over time, and the change in the level of reactive polypeptide(s) or polynucleotide(s) evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple breast tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### Cell Enrichment

In other aspects of the present invention, cell capture technologies may be used prior to polynucleotide detection to improve the sensitivity of the various detection methodologies disclosed herein.

Exemplary cell enrichment methodologies employ immunomagnetic beads that are coated with specific monoclonal antibodies to surface cell markers, or tetrameric antibody complexes, may be used to first enrich or positively select cancer cells in a sample. Various commercially available kits may be used, including Dynabeads® Epithelial Enrich (Dynal Biotech, Oslo, Norway), StemSep^{™} (StemCell Technologies, Inc., Vancouver, BC), and RosetteSep (StemCell Technologies). The skilled artisan will recognize that other readily available methodologies and kits may also be suitably employed to enrich or positively select desired cell populations.

Dynabeads® Epithelial Enrich contains magnetic beads coated with mAbs specific for two glycoprotein membrane antigens expressed on normal and neoplastic epithelial tissues. The coated beads may be added to a sample and the sample then applied to a magnet, thereby capturing the cells bound to the beads. The unwanted cells are washed away and the magnetically isolated cells eluted from the beads and used in further analyses.

RosetteSep can be used to enrich cells directly from a blood sample and consists of a cocktail of tetrameric antibodies that target a variety of unwanted cells and crosslinks them to glycophorin A on red blood cells (RBC) present in the sample, forming rosettes. When centrifuged over Ficoll, targeted cells pellet along with the free RBC.

The combination of antibodies in the depletion cocktail determines which cells will be removed and consequently which cells will be recovered. Antibodies that are available include, but are not limited to: CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE, and TCRαβ. Additionally, it is contemplated in the present invention that mAbs specific for breast tumor antigens, can be developed and used in a similar manner. For example, mAbs that bind to tumor-specific cell surface antigens may be conjugated to magnetic beads, or formulated in a tetrameric antibody complex, and used to enrich or positively select metastatic breast tumor cells from a sample.

Once a sample is enriched or positively selected, cells may be further analysed. For example, the cells may be lysed and RNA isolated. RNA may then be subjected to RT-PCR analysis using breast tumor-specific multiplex primers in a Real-time PCR assay as described herein.

In another aspect of the present invention, cell capture technologies may be used in conjunction with Real-time PCR to provide a more sensitive tool for detection of metastatic cells expressing breast tumor antigens. Detection of breast cancer cells in bone marrow samples, peripheral blood, and small needle aspiration samples is desirable for diagnosis and prognosis in breast cancer patients.

### Probes and Primers

As noted above and as described in further detail herein, certain methods, compositions and kits according to the present invention utilize two or more oligonucleotide primer pairs for the detection of cancer. The ability of such nucleic acid probes to specifically hybridize to a sequence of interest will enable them to be of use in detecting the presence of complementary sequences in a biological sample.

Alternatively, in other embodiments, the probes and/or primers of the present invention may be employed for detection via nucleic acid hybridization. As such, it is contemplated that nucleic acid segments that comprise a sequence region of at least about 15 nucleotide long contiguous sequence that has the same sequence as, or is complementary to, a 15 nucleotide long contiguous sequence of a polynucleotide to be detected will find particular utility. Longer contiguous identical or complementary sequences, *e.g.*, those of about 20, 30, 40, 50, 100, 200, 500, 1000 (including all intermediate lengths) and even up to full length sequences will also be of use in certain embodiments.

Oligonucleotide primers having sequence regions consisting of contiguous nucleotide stretches of 10-14, 15-20, 30, 50, or even of 100-200 nucleotides or so (including intermediate lengths as well), identical or complementary to a polynucleotide to be detected , are particularly contemplated as primers for use in amplification reactions such as, *e.g.*, the polymerase chain reaction (PCR^{™}).. This would allow a polynucleotide to be analyzed, both in diverse biological samples such as, for example, blood, lymph nodes and bone marrow.

The use of a primer of about 15-25 nucleotides in length allows the formation of a duplex molecule that is both stable and selective. Molecules having contiguous complementary sequences over stretches greater than 15 bases in length are generally preferred, though, in order to increase stability and selectivity of the hybrid, and thereby improve the quality and degree of specific hybrid molecules obtained. One will generally prefer to design primers having gene-complementary stretches of 15 to 25 contiguous nucleotides, or even longer where desired.

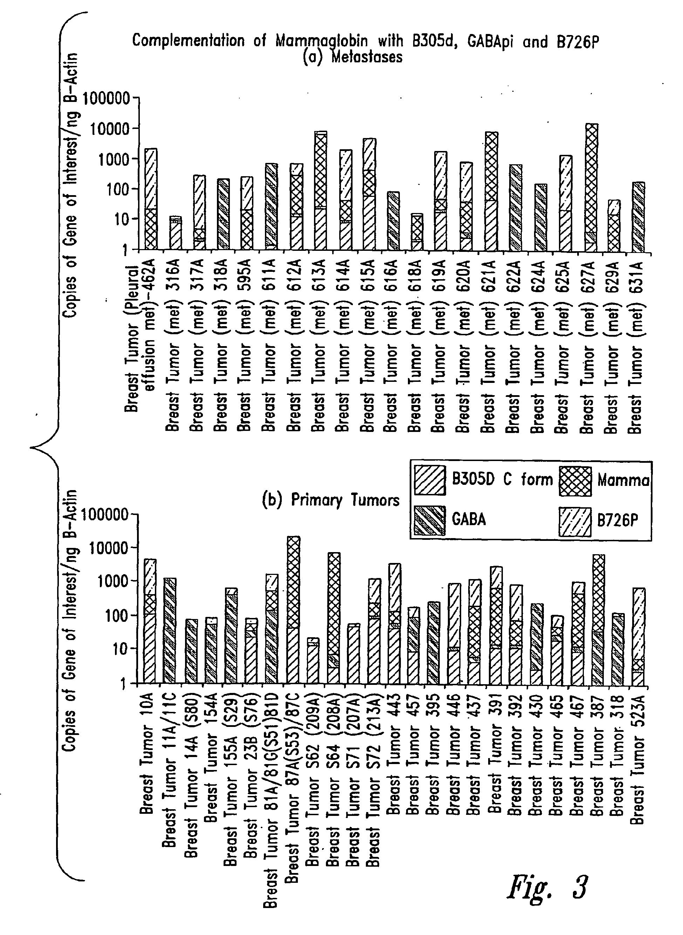

Primers may be selected from any portion of the polynucleotide to be detected. All that is required is to review the sequence, such as those exemplary polynucleotides set forth in SEQ ID NO: 1, 3, 5-7, 11, 13, 15, 17, 19-24, 30, 32, 73-75 (Figures 3-6, respectively) and SEQ ID NO:76 (lipophilin B) or to any continuous portion of the sequence, from about 15-25 nucleotides in length up to and including the full length sequence, that one wishes to utilize as a primer. The choice of primer sequences may be governed by various factors. For example, one may wish to employ primers from towards the termini of the total sequence. The exemplary primers disclosed herein may optionally be used for their ability to selectively form duplex molecules with complementary stretches of the entire polynucleotide of interest such as those set forth in SEQ ID NO: 1, 3, 5-7, 11, 13, 15, 17, 19-24, 30, 32, 73-75 (Figures 3-6, respectively), and SEQ ID NO:76 (lipophilin B).

The present invention further provides the nucleotide sequence of various exemplary oligonucleotide primers and probes, set forth in SEQ ID NOs: 33-71, that may be used, as described in further detail herein, according to the methods of the present invention for the detection of cancer.

Oligonucleotide primers according to the present invention may be readily prepared routinely by methods commonly available to the skilled artisan including, for example, directly synthesizing the primers by chemical means, as is commonly practiced using an automated oligonucleotide synthesizer. Depending on the application envisioned, one will typically desire to employ varying conditions of hybridization to achieve varying degrees of selectivity of probe towards target sequence. For applications requiring high selectivity, one will typically desire to employ relatively stringent conditions to form the hybrids, *e*.*g*., one will select relatively low salt and/or high temperature conditions, such as provided by a salt concentration of from about 0.02 M to about 0.15 M salt at temperatures of from about 50°C to about 70°C. Such selective conditions tolerate little, if any, mismatch between the probe and the template or target strand, and would be particularly suitable for isolating related sequences.

### Polynucleotide Amplification Techniques

Each of the specific embodiments outlined herein for the detection of cancer has in common the detection of a tissue- and/or tumor-specific polynucleotide via the hybridization of one or more oligonucleotide primers and/or probes. Depending on such factors as the relative number of cancer cells present in the biological sample and/or the level of polynucleotide expression within each cancer cell, it may be preferred to perform an amplification step prior to performing the steps of detection. For example, at least two oligonucleotide primers may be employed in a polymerase chain reaction (PCR) based assay to amplify a portion of a breast tumor cDNA derived from a biological sample, wherein at least one of the oligonucleotide primers is specific for (*i*.*e*., hybridizes to) a polynucleotide encoding the breast tumor protein. The amplified cDNA may optionally be subjected to a fractionation step such as, for example, gel electrophoresis.

A number of template dependent processes are available to amplify the target sequences of interest present in a sample. One of the best known amplification methods is the polymerase chain reaction (PCR^{™}) which is described in detail in U.S. Patent Nos. 4,683,195, 4,683,202 and 4,800,159, each of which is incorporated herein by reference in its entirety. Briefly, in PCR^{™}, two primer sequences are prepared which are complementary to regions on opposite complementary strands of the target sequence. An excess of deoxynucleoside triphosphates is added to a reaction mixture along with a DNA polymerase (*e*.*g*., *Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction product and the process is repeated. Preferably reverse transcription and PCR^{™} amplification procedure may be performed in order to quantify the amount of mRNA amplified. Polymerase chain reaction methodologies are well known in the art.

One preferred methodology for polynucleotide amplification employs RT-PCR, in which PCR is applied in conjunction with reverse transcription. Typically, RNA is extracted from a biological sample, such as blood, serum, lymph node, bone marrow, sputum, urine and tumor biopsy samples, and is reverse transcribed to produce cDNA molecules. PCR amplification using at least one specific primer generates a cDNA molecule, which may be separated and visualized using, for example, gel electrophoresis. Amplification may be performed on biological samples taken from a patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

Any of a variety of commercially available kits may be used to perform the amplification step. One such amplification technique is inverse PCR (*see* Triglia *et al., Nucl. Acids Res. 16*:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom *et al., PCR Methods Applic. 1*:111-19, 1991) and walking PCR (Parker *et al., Nucl. Acids. Res. 19*:3055-60, 1991). Other methods employing amplification may also be employed to obtain a full length cDNA sequence.

Another method for amplification is the ligase chain reaction (referred to as LCR), disclosed in Eur. Pat. Appl. Publ. No. 320,308 (specifically incorporated herein by reference in its entirety). In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR^{™}, bound ligated units dissociate from the target and then serve as "target sequences" for ligation of excess probe pairs. U.S. Patent No. 4,883,750, incorporated herein by reference in its entirety, describes an alternative method of amplification similar to LCR for binding probe pairs to a target sequence.

Qbeta Replicase, described in PCT Intl. Pat. Appl. Publ. No. PCT/US87/00880, incorporated herein by reference in its entirety, may also be used as still another amplification method in the present invention. In this method, a replicative sequence of RNA that has a region complementary to that of a target is added to a sample in the presence of an RNA polymerase. The polymerase will copy the replicative sequence that can then be detected.

An isothermal amplification method, in which restriction endonucleases and ligases are used to achieve the amplification of target molecules that contain nucleotide 5'-[α-thio]triphosphates in one strand of a restriction site (Walker *et al*., 1992, incorporated herein by reference in its entirety), may also be useful in the amplification of nucleic acids in the present invention.

Strand Displacement Amplification (SDA) is another method of carrying out isothermal amplification of nucleic acids which involves multiple rounds of strand displacement and synthesis, *i*.*e*. nick translation. A similar method, called Repair Chain Reaction (RCR) is another method of amplification which may be useful in the present invention and is involves annealing several probes throughout a region targeted for amplification, followed by a repair reaction in which only two of the four bases are present. The other two bases can be added as biotinylated derivatives for easy detection. A similar approach is used in SDA.

Sequences can also be detected using a cyclic probe reaction (CPR). In CPR, a probe having a 3' and 5' sequences of non-target DNA and an internal or "middle" sequence of the target protein specific RNA is hybridized to DNA which is present in a sample. Upon hybridization, the reaction is treated with RNaseH, and the products of the probe are identified as distinctive products by generating a signal that is released after digestion. The original template is annealed to another cycling probe and the reaction is repeated. Thus, CPR involves amplifying a signal generated by hybridization of a probe to a target gene specific expressed nucleic acid.

Still other amplification methods described in Great Britain Pat. Appl. No. 2 202 328, and in PCT Intl. Pat. Appl. Publ. No. PCT/US89/01025, may be used in accordance with the present invention. In the former application, "modified" primers are used in a PCR-like, template and enzyme dependent synthesis. The primers may be modified by labeling with a capture moiety (*e.g*., biotin) and/or a detector moiety (*e.g*., enzyme). In the latter application, an excess of labeled probes is added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS) (Kwoh *et al*., 1989; PCT Intl. Pat. Appl. Publ. No. WO 88/10315), including nucleic acid sequence based amplification (NASBA) and 3SR In NASBA, the nucleic acids can be prepared for amplification by standard phenol/chloroform extraction, heat denaturation of a sample, treatment with lysis buffer and minispin columns for isolation of DNA and RNA or guanidinium chloride extraction of RNA. These amplification techniques involve annealing a primer that has sequences specific to the target sequence. Following polymerization, DNA/RNA hybrids are digested with RNase H while double stranded DNA molecules are heat-denatured again. In either case the single stranded DNA is made fully double stranded by addition of second target-specific primer, followed by polymerization. The double stranded DNA molecules are then multiply transcribed by a polymerase such as T7 or SP6. In an isothermal cyclic reaction, the RNAs are reverse transcribed into DNA, and transcribed once again with a polymerase such as T7 or SP6. The resulting products, whether truncated or complete, indicate target-specific sequences.

Eur. Pat. Appl. Publ. No. 329,822, incorporated herein by reference in its entirety, disclose a nucleic acid amplification process involving cyclically synthesizing single-stranded RNA ("ssRNA"), ssDNA, and double-stranded DNA (dsDNA), which may be used in accordance with the present invention. The ssRNA is a first template for a first primer oligonucleotide, which is elongated by reverse transcriptase (RNA-dependent DNA polymerase). The RNA is then removed from resulting DNA:RNA duplex by the action of ribonuclease H (RNase H, an RNase specific for RNA in a duplex with either DNA or RNA). The resultant ssDNA is a second template for a second primer, which also includes the sequences of an RNA polymerase promoter (exemplified by T7 RNA polymerase) 5' to its homology to its template. This primer is then extended by DNA polymerase (exemplified by the large "Klenow" fragment of *E. coli* DNA polymerase I), resulting as a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

PCT Intl. Pat. Appl. Publ. No. WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic; *i*.*e*. new templates are not produced from the resultant RNA transcripts. Other amplification methods include "RACE" (Frohman, 1990), and "one-sided PCR" (Ohara, 1989) which are well-known to those of skill in the art.

### Compositions and Kits for the Detection of Cancer

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a breast tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

The present invention also provides kits that are suitable for performing the detection methods of the present invention. Exemplary kits comprise oligonucleotide primer pairs each one of which specifically hybridizes to a distinct polynucleotide. Within certain embodiments, kits according to the present invention may also comprise a nucleic acid polymerase and suitable buffer. Exemplary oligonucleotide primers suitable for kits of the present invention are disclosed herein by SEQ ID NOs: 33-71. Exemplary polynucleotides suitable for kits of the present invention are disclosed in SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ ID NO:22, SEQ ID NO:23, SEQ ID NO:24, SEQ ID NO:30, SEQ ID NO:32, and lipophilin B.

Alternatively, a kit may be designed to detect the level of mRNA encoding a breast tumor protein in a biological sample. Such kits generally comprise at least one oligonucleotide probe or primer, as described above, that hybridizes to a polynucleotide encoding a breast tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a breast tumor protein.

In other related aspects, the present invention further provides compositions useful in the methods disclosed herein. Exemplary compositions comprise two or more oligonucleotide primer pairs each one of which specifically hybridizes to a distinct polynucleotide. Exemplary oligonucleotide primers suitable for compositions of the present invention are disclosed herein by SEQ ID NOs: 33-71. Exemplary polynucleotides suitable for compositions of the present invention are disclosed in SEQ ID NO:73, SEQ ID NO:74, SEQ ID NO:75, SEQ ID NO:1, SEQ ID NO:3, SEQ ID NO:5, SEQ ID NO:6, SEQ ID NO:7, SEQ ID NO:11, SEQ ID NO:13, SEQ ID NO:15, SEQ ID NO:17, SEQ ID NO:19, SEQ ID NO:20, SEQ ID NO:21, SEQ ID NO:22, SEQ ID NO:23, SEQ ID NO:24, SEQ ID NO:30, SEQ ID NO:32, and lipophilin B.

The following Examples are offered by way of illustration and not by way of limitation.

### EXAMPLES

### EXAMPLE 1

### DIFFERENTIAL DISPLAY

This example discloses the use of differential display to enrich for polynucleotides that are over-expressed in breast tumor tissues.

Differential display was performed as described in the literature (*see, e.g.,* Liang, P. et al., *Science* 257:967-971 (1993)) with the following modifications: (a) PCR amplification products were visualized on silver stained gels (b) genetically matched pairs of tissues were used to eliminate polymorphic variation (c) two different dilutions of cDNA were used as template to eliminate any dilutional effects (*see,* Mou, E. et al., *Biochem Biophy Res Commun*. 199:564-569 (1994)).

### EXAMPLE 2

### PREPARATION OF CDNA SUBTRACTION LIBRARY

This example discloses the preparation of a breast tumor cDNA subtraction library enriched in breast tumor specific polynucleotides.

cDNA library subtraction was performed as described with some modification. *See*, Hara, T. et al., *Blood* 84: 189-199 (1994). The breast tumor library (tracer) that was made from a pool of three breast tumors was subtracted with normal breast library (driver) to identify breast tumor specific genes. More recent subtractions utilized 6-10 normal tissues as driver to subtract out common genes more efficiently, with an emphasis on essential tissues along with one "immunological" tissue (*e.g*., spleen, lymph node, or PBMC), to assist in the removal of cDNAs derived from lymphocyte infiltration in tumors. The breast tumor specific subtracted cDNA library was generated as follows: driver cDNA library was digested with EcoRI, NotI, and SfuI (SfuI cleaves the vector), filled in with DNA polymerase klenow fragment. After phenol-chloroform extraction and ethanol precipitation, the DNA was labeled with Photoprobe biotin and dissolved in H₂O. Tracer cDNA library was digested with BamHI and XhoI, phenol chloroform extracted, passed through Chroma spin-400 columns, ethanol precipitated, and mixed with driver DNA for hybridization at 68°C for 20 hours [long hybridization (LH)]. The reaction mixture was then subjected to the streptavidin treatment followed by phenol/chloroform extraction for a total of four times. Subtracted DNA was precipitated and subjected to a hybridization at 68°C for 2 hours with driver DNA again [short hybridization (SH)]. After removal of biotinylated double-stranded DNA, subtracted cDNA was ligated into BamHI/XhoI site of Chloramphenicol resistant pBCSK⁺ and transformed into ElectroMax *E. coli* DH10B cells by electroporation to generate subtracted cDNA library. To clone less abundant breast tumor specific genes, cDNA library subtraction was repeated by subtracting the tracer cDNA library with the driver cDNA library plus abundant cDNAs from primary subtractions. This resulted in the depletion of these abundant sequences and the generation of subtraction libraries that contain less abundant sequences.

To analyze the subtracted cDNA library, plasmid DNA was prepared from 100-200 independent clones, which were randomly picked from the subtracted library, and characterized by DNA sequencing. The determined cDNA and expected amino acid sequences for the isolated cDNAs were compared to known sequences using the most recent Genbank and human EST databases.

### EXAMPLE 3

### PCR-SUBTRACTION

This example discloses PCR subtraction to enrich for breast tumor specific polynucleotides.

PCR-subtraction was performed essentially as described in the literature. *See,* Diatchenko, L. et al., *Proc Natl Acad Sci U S A*. 93:6025-6030 (1996) and Yang, G.P. et al., *Nucleic acids Res.* 27:1517-23 (1999). Briefly, this type of subtraction works by ligating two different adapters to different aliquots of a restriction enzyme digested tester (breast tumor) cDNA sample, followed by mixing of the testers separately with excess driver (without adapters). This first hybridization results in normalization of single stranded tester specific cDNA due to the second order kinetics of hybridization. These separate hybridization reactions are then mixed without denaturation, and a second hybridization performed which produces the target molecules; double stranded cDNA fragments containing both of the different adapters. Two rounds of PCR were performed, which results in the exponential amplification of the target population molecules (normalized tester specific cDNAs), while other fragments were either unamplified or only amplified in a linear manner. The subtractions performed included a pool of breast tumors subtracted with a pool of normal breast and a pool of breast tumors subtracted with a pool of normal tissues including PBMC, brain, pancreas, liver, small intestine, stomach, heart and kidney.

Prior to cDNA synthesis RNA was treated with DNase I (Ambion) in the presence of RNasin (Promega Biotech, Madison, WI) to remove DNA contamination. The cDNA for use in real-time PCR tissue panels was prepared using 25µl Oligo dT (Boehringer-Mannheim) primer with superscript II reverse transcriptase (Gibco BRL, Bethesda, MD).

### EXAMPLE 4

### DETECTION OF BREAST CANCER USING BREAST-SPECIFIC ANTIGENS

The isolation and characterization of the breast-specific antigens B511S and B533S is described in U.S. Patent Application 09/346,327, filed July 2, 1999, the disclosure of which is hereby incorporated by reference in its entirety. The determined cDNA sequence for B511S is provided in SEQ ID NO: 30, with the corresponding amino acid sequence being provided in SEQ ID NO: 31. The determined cDNA sequence for B533S is provided in SEQ ID NO: 32. The isolation and characterization of the breast-specific antigen B726P is described in U.S. Patent Applications 09/285,480, filed April 2, 1999, and 09/433,826, filed November 3, 1999.

The determined cDNA sequences for splice variants of B726P are provided in SEQ ID NO: 13, 15, 17 and 19-24, with the corresponding amino acid sequences being provided in SEQ ID NO: 14, 16, 18 and 25-29.

The isolation and characterization of the breast-specific antigen B305D forms A and C has been described in U.S. Patent Application 09/429,755, filed October 28, 1999. Determined cDNA sequences for B305D isoforms A and C are provided in SEQ ID NO: 1, 3 and 5-7, with the corresponding amino acid sequences being provided in SEQ ID NO: 2, 4 and 8-10.

The isolation and characterization of the breast-specific antigen B311D has been described in U.S. Patent Application 09/289,198, filed April 9, 1999.

The determined cDNA sequence for B311D is provided in SEQ ID NO:11, with the corresponding amino acid sequence being provided in SEQ ID NO:12.

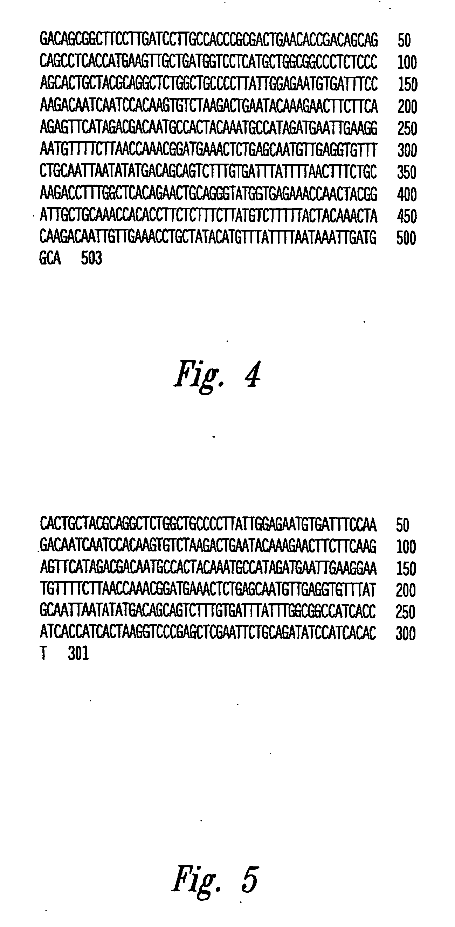

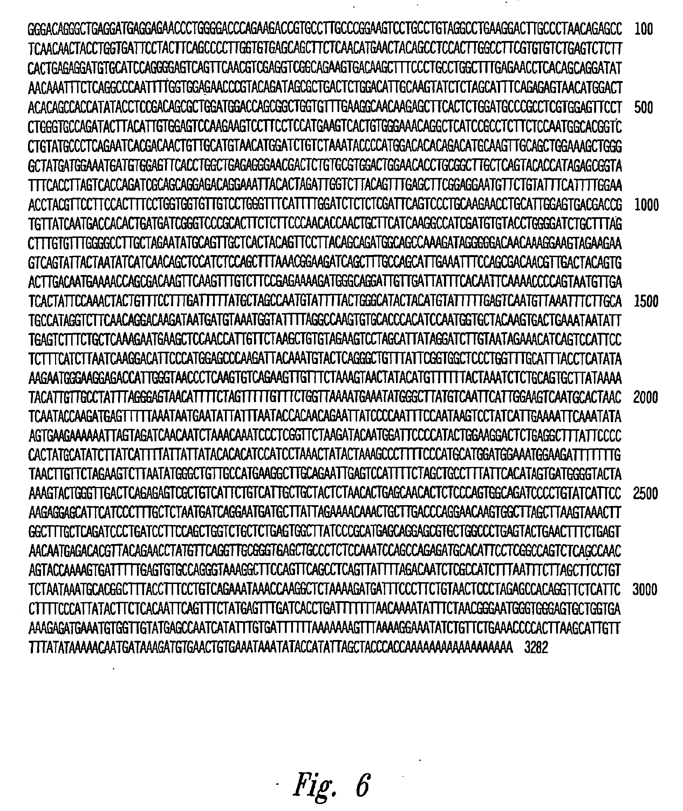

cDNA sequences for mammaglobin are provided in Figs. 4 and 5, with the cDNA sequence for GABAπ being provided in Fig 6 and are disclosed in SEQ ID NOs: 73-75, respectively.

The isolation and characteization of the breast-specific antigen lipophilin B has been described in U.S. Patent Application 09/780,842, filed February 8, 2001 . The determined cDNA sequence for lipophilin B is provided in SEQ ID NO:76, with the corresponding amino acid sequence being provided in SEQ ID NO:77. The nucleotide sequences of several sequence variants of lipophilin B are also described in the 09/780,842 application.

### EXAMPLE 5

### MICROARRAY ANALYSIS

This example discloses the use of microarray analyses to identify polynucleotides that are at least two-fold overexpressed in breast tumor tissue samples as compared to normal breast tissue samples.

mRNA expression of the polynucleotides of interest was performed as follows. cDNA for the different genes was prepared as described above and arrayed on a glass slide (Incyte, Palo Alto, CA). The arrayed cDNA was then hybridized with a 1:1 mixture of Cy3 or Cy5 fluorescent labeled first strand cDNAs obtained from polyA+ RNA from breast tumors, normal breast and normal tissues and other tumors as described in Shalon, D. et al., *Genome Res.* 6:639-45 (1996). Typically Cy3 (Probe 1) was attached to cDNAs from breast tumors and Cy5 (Probe 2) to normal breast tissue or other normal tissues. Both probes were allowed to compete with the immobilized gene specific cDNAs on the chip, washed then scanned for fluorescence intensity of the individual Cy3 and Cy5 fluorescence to determine extent of hybridization. Data were analyzed using GEMTOOLS software (Incyte, Palo Alto, CA) which enabled the overexpression patterns of breast tumors to be compared with normal tissues by the ratios of Cy3/Cy5. The fluorescence intensity was also related to the expression level of the individual genes.

DNA microarray analyses was used primarily as a screening tool to determine tissue/tumor specificity of cDNA's recovered from the differential display, cDNA library and PCR subtractions, prior to more rigorous analysis by quantitative RT-PCR, northern blotting, and immunohistochemistry. Microarray analysis was performed on two microchips. A total of 3603 subtracted cDNA's and 197 differential display templates were evaluated to identify 40 candidates for further analysis by quantitative PCR. From these candidates, several were chosen on the basis of favorable tissue specificity profiles, including B305D, B311D, B726P, B511S and B533S, indicating their overexpression profiles in breast tumors and/or normal breast versus other normal tissues. It was evident that the expression of these genes showed a high degree of specificity for breast tumors and/or breast tissue. In addition, these genes have in many cases complementary expression profiles.

The two known breast-specific genes, mammaglobin and γ-aminobutyrate type A receptor π subunit (GABAπ) were also subjected to microarray analysis. mRNA expression of mammaglobin has been previously described to be upregulated in proliferating breast tissue, including breast tumors. *See,* (Watson et al., *Cancer Res.,* 56: 860-5 (1996); Watson et al., Cancer Res., 59: 3028-3031 (1999); Watson et al., Oncogene. 16:817-24 (1998)).

The GABAπ mRNA levels were over-expressed in breast tumors. Previous studies had demonstrated its overexpression in uterus and to some degree in prostate and lung (Hedblom et al., J Biol. Chem. 272:15346-15350 (1997)) but no previous study had indicated elevated levels in breast tumors.

### EXAMPLE 6

### QUANTITATIVE REAL-TIME PCR ANALYSIS

This example discloses the use of quantitative Real-time PCR to confirm the microarray identification polynucleotide that are at least two-fold overexpressed in breast tumor tissue samples as compared to normal breast tissue samples.

The tumor- and/or tissue-specificity of the polynucleotides identified by the microarray analyses disclosed herein in Example 5, were confirmed by quantitative PCR analyses. Breast metastases, breast tumors, benign breast disorders and normal breast tissue along with other normal tissues and tumors were tested in quantitative (Real time) PCR. This was performed either on the ABI 7700 Prism or on a GeneAmp® 5700 sequence detection system (PE Biosystems, Foster City, CA). The 7700 system uses a forward and a reverse primer in combination with a specific probe designed to anneal to sequence between the forward and reverse primer. This probe was conjugated at the 5'end with a fluorescent reporter dye and a quencher dye at the other 3' end (Taqman^{™}). During PCR the Taq DNA polymerase with it's 5'-3' nuclease activity cleaved the probe which began to fluoresce, allowing the reaction to be monitored by the increase in fluorescence (Real-time). Holland et al., *Proc Natl Acad Sci U S A*. 88:7276-7280 (1991). The 5700 system used SYBR® green, a fluorescent dye, that only binds to double stranded DNA (Schneeberger et al., PCR Methods Appl. 4:234-8 (1995)), and the same forward and reverse primers as the 7700 instrument. No probe was needed. Matching primers and fluorescent probes were designed for each of the genes according to the Primer Express program (PE Biosystems, Foster City, CA).

**Table 1.**

| Primer and Probe Sequences for the Genes of Interest | | | |
|---|---|---|---|
| | Forward Primer | Reverse primer | Probe |
| Mammaglobin | TGCCATAGATG AATTGAAGGAA TG (SEQ ID NO:48) | TGTCATATATTAA TTGCATAAACACC TCA (SEQ ID NO:49) | TCTTAACCAAACG GATGAAACTCTGA GCAATG (SEQ ID NO:50) |
| B305D-C form | AAAGCAGATGG TGGTTGAGGTT (SEQ ID NO:39) | CCTGAGACCAAA TGGCTTCTTC (SEQ ID NO:40) | ATTCCATGCCGGC TGCTTCTTCTG (SEQ ID NO:41) |
| B311D | CCGCTTCTGACA ACACTAGAGAT C (SEQ ID NO:63) | CCTATAAAGATGT TATGTACCAAAA ATGAAGT (SEQ ID NO:64) | CCCCTCCCTCAGG GTATGGCCC (SEQ ID NO:65) |
| B726P | TCTGGTTTTCTC ATTCTTTATTCA TTTATT (SEQ ID NO:42) | TGCCAAGGAGCG GATTATCT (SEQ ID NO:43) | CAACCACGTGACA AACACTGGAATTA CAGG (SEQ ID NO:44) |
| B533S | CCCTTTCTCACC CACACACTGT (SEQ ID NO:66) | TGCATTCTCTCAT ATGTGGAAGCT (SEQ ID NO:67) | CCGGGCCTCAGGC ATATACTATTCTA CTGTCTG (SEQ ID NO:68) |
| GABAπ | AAGCCTCAGAG TCCTTCCAGTAT G (SEQ ID NO:36) | AAATATAAGTGA AGAAAAAAATTA GTAGAT (SEQ ID NO:72) | AATCCATTGTATC TTAGAACCGAGGG ATTTGTTTAGA (SEQ ID NO:38) |
| B511S | GACATTCCAGTT TTACCCAAATG G (SEQ ID NO:69) | TGCAGAAGACTC AAGCTGATTCC (SEQ ID NO:70) | TCTCAGGGACACA CTCTACCATTCGG GA (SEQ ID NO:71) |

The concentrations used in the quantitative PCR for the forward primers for mammaglobin, GABAπ, B305D C form, B311D, B511S, B533S and B726P were 900, 900, 300, 900, 900, 300 and 300nM respectively. For the reverse primers they were 300, 900, 900, 900, 300, 900 and 900nM respectively. Primers and probes so produced were used in the universal thermal cycling program in real-time PCR. They were titrated to determine the optimal concentrations using a checkerboard approach. A pool of cDNA from target tumors was used in this optimization process. The reaction was performed in 25µl volumes. The final probe concentration in all cases was 160nM. dATP, dCTP and dGTP were at 0.2mM and dUTP at 0.4mM. Amplitaq gold and Amperase UNG (PE Biosystems, Foster City, CA) were used at 0.625 units and 0.25 units per reaction. MgCl₂ was at a final concentration of 5mM. Trace amounts of glycerol, gelatin and Tween 20 (Sigma Chem Co, St Louis, MO) were added to stabilize the reaction. Each reaction contained 2µl of diluted template. The cDNA from RT reactions prepared as above was diluted 1:10 for the gene of interest and 1:100 for β-Actin. Primers and probes for β-Actin (PE Biosystems, Foster City, CA) were used in a similar manner to quantitate the presence of β-actin in the samples. In the case of the SYBR® green assay, the reaction mix (25µl) included 2.5µl of SYBR green buffer, 2µl of cDNA template and 2.5µl each of the forward and reverse primers for the gene of interest. This mix also contained 3mM MgCl₂, 0.25units of AmpErase UNG, 0.625 units of Amplitaq gold, 0.08% glycerol, 0.05% gelatin, 0.0001% Tween 20 and 1mM dNTP mix. In both formats, 40 cycles of amplification were performed.

In order to quantitate the amount of specific cDNA (and hence initial mRNA) in the sample, a standard curve was generated for each run using the plasmid containing the gene of interest. Standard curves were generated using the Ct values determined in the real-time PCR which were related to the initial cDNA concentration used in the assay. Standard dilutions ranging from 20-2x10⁶ copies of the gene of interest were used for this purpose. In addition, a standard curve was generated for the housekeeping gene β-actin ranging from 200fg-2000pg to enable normalization to a constant amount of β-Actin. This allowed the evaluation of the over-expression levels seen with each of the genes.

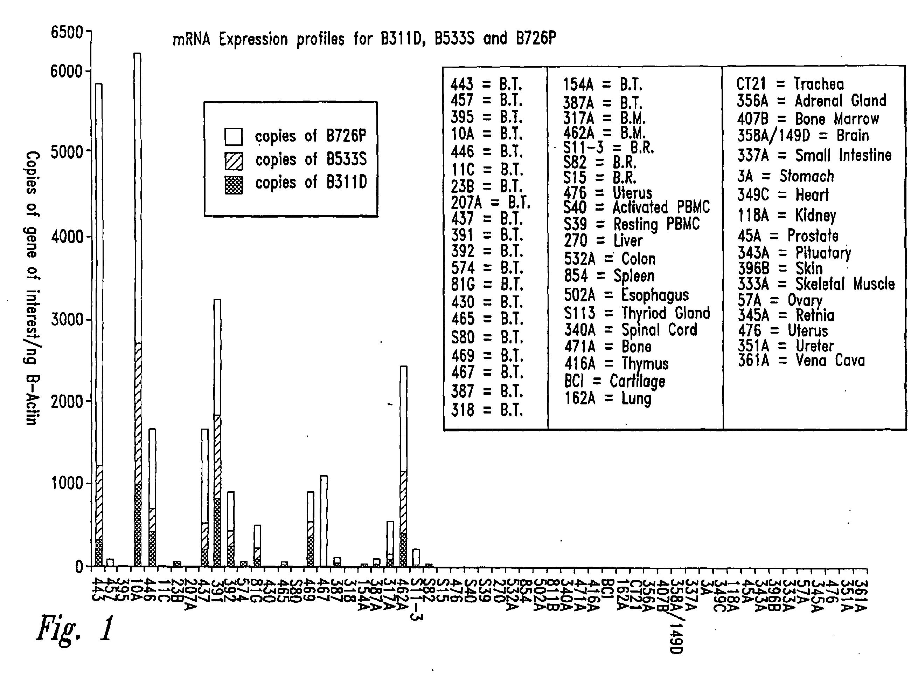

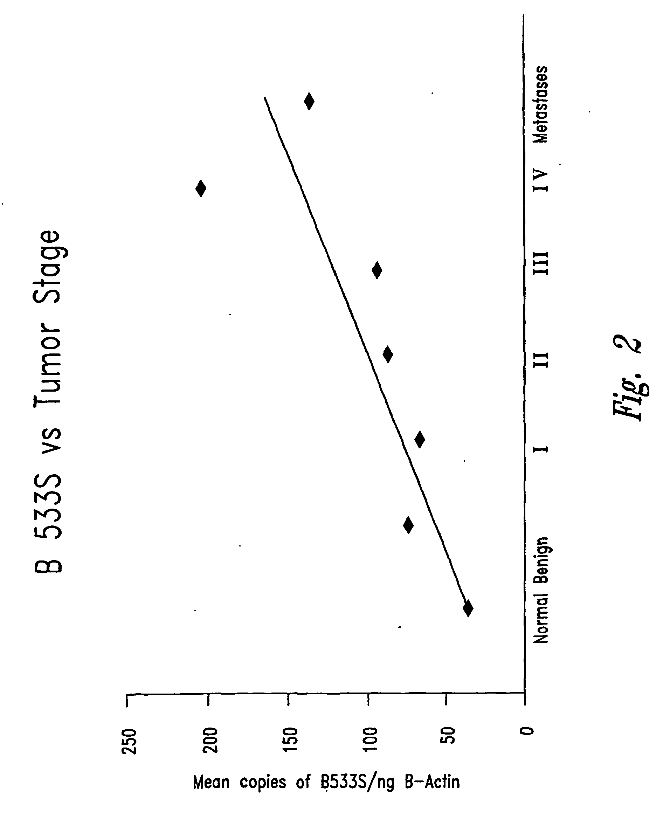

The genes B311D, B533S and B726P were evaluated in quantitative PCR as described above on two different panels consisting of: (a) breast tumor, breast normal and normal tissues; and (b) breast tumor metastases (primarily lymph nodes), using the primers and probes shown above in Table 1. The data for panel (a) is shown in Figure 1 for all three genes. The three genes showed identical breast tissue expression profiles. However, the relative level of gene expression was very different in each case. B311D in general was expressed at lower levels than B533S and both less than B726P, but all three were restricted to breast tissue. The quantitative PCR thus confirmed there was a differential expression between normal breast tissue and breast tumors for all three genes, and that approximately 50% of breast tumors over-expressed these genes. When tested on a panel of distant metastases derived from breast cancers all three genes reacted with 14/21 metastases and presented similar profiles. All three genes also exhibited increasing levels of expression as a function of pathological stage of the tumor, as shown for B533S in Figure 2.

Mammaglobin is a homologue of a rabbit uteroglobin and the rat steroid binding protein subunit C3 and is a low molecular weight protein that is highly glycosylated. In contrast to its homologs, mammaglobin has been reported to be breast specific and over-expression has been described in breast tumor biopsies (23%) and primary and metastatic breast tumors (~75%) with reports of the detection of mammaglobin mRNA expression in 91 % of lymph nodes from metastatic breast cancer patients. However, more rigorous analysis of mammaglobin gene expression by microarray and quantitative PCR as described above (panels (a) and (b) and a panel of other tumors and normal tissues and additional breast tumors), showed expression at significant levels in skin and salivary gland with much lower levels in esophagus and trachea, as shown in Table 2 below.

**Table 2**

| Normalized Distribution of Mammaglobin and B511S mRNA in Various Tissues | | | | | |
|---|---|---|---|---|---|
| Tissue | Mean Copies Mammaglobin /ng β-Actin ± SD | PCR Positive | Mean Copies B511S /ng β-Actin ± SD | PCR Positive | PCR Positive (Mammaglobin/ B511S) |
| Breast Tumors | 1233.88±3612 .74 | 31/42 | 1800.40±3893.24 | 33/42 | 38/42 |
| Breast tumor Metastases | 1912.54±4625 .85 | 14/24 | 3329.50±10820.71 | 14/24 | 17/24 |
| Benign Breast disorders | 121.87±78.63 | 3/3 | 524.66±609.43 | 2/3 | 3/3 |
| Normal breast | 114.19±94.40 | 11/11 | 517.64±376.83 | 8/9 | 11/11 |
| Breast reduction | 231.50±276.6 8 | 2/3 | 482.54±680.28 | ½ | 2/3 |
| Other tumors | 0.13±0.65 | 1/39 | 24.17±36.00 | 5/23 | |
| Salivary gland | 435.65±705.11 | 2/3 | 45766.61±44342.43 | 3/3 | |
| Skin | 415.74±376.14 | 7/9 | 7039.05±7774.24 | 9/9 | |
| Esophagus | 4.45±3.86 | 2/3 | 1.02±0.14 | 0/3 | |
| Bronchia | 0.16 | 0/1 | 84.44±53.31 | 2/2 | |
| Other normal tissues | 0.33±1.07 | 0/85 | 5.49±10.65 | 3/75 | |

The breast-specific gene B511S, while having a different profile of reactivity on breast tumors and normal breast tissue to mammaglobin, reacted with the same subset of normal tissues as mammaglobin. B511S by PSORT analysis is indicated to have an ORF of 90aa and to be a secreted protein as is the case for mammaglobin. B511S has no evidence of a transmembrane domain but may harbor a cleavable signal sequence. Mammaglobin detected 14/24 of distant metastatic breast tumors, 31/42 breast tumors and exhibited ten-fold over-expression in tumors and metastases as compared to normal breast tissue. There was at least 300-fold over-expression in normal breast tissue versus other negative normal tissues and tumors tested, which were essentially negative for mammaglobin expression. B511S detected 33/42 breast tumors and 14/24 distant metastases, while a combination of B511S with mammaglobin would be predicted to detect 38/42 breast tumors and 17/24 metastatic lesions (Table 2 above). The quantitative level of expression of B511S and mammaglobin were also in similar ranges, in concordance with the microarray profiles observed for these two genes. Other genes that were additive with mammaglobin are shown in Table 3.

**Table 3**

| mRNA Complementation of Mammaglobin with Other Genes | | | | | | |
|---|---|---|---|---|---|---|
| | Mammaglobin Positive | Mammaglobin Negative | | | | |
| | | B305D | GABAπ | B726P | B305D + GABAπ | B305D + GABAπ + B726P |
| Breast Metastases | 13/21 | 2/8 | 5/8 | 3/8 | 7/8 | 8/8 |
| Breast tumors | 18/25 | 3/7 | 4/7 | 5/7 | 7/7 | 7/7 |

B305D was shown to be highly over-expressed in breast tumors, prostate tumors, normal prostate tissue and testis compared to normal tissues, including normal breast tissue. Different splice variants of B305D have been identified with form A and C being the most abundant but all tested have similar tissue profiles in quantitative PCR. The A and C forms contain ORF's of 320 and 385 aa, respectively. B305D is predicted by PSORT to be a Type II membrane protein that comprises a series of ankyrin repeats. A known gene shown to be complementary with B305D, in breast tumors, was GABAπ. This gene is a member of the GABA_{A} receptor family and encodes a protein that has 30-40% amino acid homology with other family members, and has been shown by Northern blot analysis to be over-expressed in lung, thymus and prostate at low levels and highly over-expressed in uterus. Its expression in breast tissue has not been previously described. This is in contrast to other GABA_{A} receptors that have appreciable expression in neuronal tissues. Tissue expression profiling of this gene showed it to be over-expressed in breast tumors in an inverse relationship to the B305D gene (Table 3). GABAπ detected 15/25 tumors and 6/21 metastases including 4 tumors and 5 metastases missed by mammaglobin. In contrast, B305D detected 13/25 breast tumors and 8/21 metastases, again including 3 tumors and 2 metastases missed by mammaglobin. A combination of just B305D and the GABAπ would be predicted to identify 22/25 breast tumors and 14/21 metastases. The combination of B305D and GABAπ with mammaglobin in detecting breast metastases is shown in Table 3 above and Figures. 3A and 3B. This combination detected 20/21 of the breast metastases as well as 25/25 breast tumors that were evaluated on the same panels for all three genes. The one breast metastasis that was negative for these three genes was strongly positive for B726P (Figs. 3A and 3B).

To evaluate the presence of circulating tumor cells, an immunocapture (cell capture) method was employed to first enrich for epithelial cells prior to RT-PCR analysis. Immunomagnetic polystyrene beads coated with specific monoclonal antibodies to two glycoproteins on the surface of epithelial cells were used for this purpose. Such an enrichment procedure increased the sensitivity of detection (~100 fold) as compared to direct isolation of poly A⁺ RNA, as shown in Table 4.

**Table 4**

| Extraction of Mammaglobin Positive Cells (MB415) Spiked into Whole Blood and Detection by Real-time PCR | | |
|---|---|---|
| MB415 cells/ml Blood | Epithelial cell extraction (Poly A⁺ RNA) | Direct Extraction (Poly A⁺ RNA) |
| | Copies Mammaglobin/ng β-Actin | |
| 100000 | 54303.2 | 58527.1 |
| 10000 | 45761.9 | 925.9 |
| 1000 | 15421.2 | 61.6 |
| 100 | 368.0 | 5.1 |
| 10 | 282.0 | 1.1 |
| 1 | 110.2 | 0 |
| 0 | 0 | 0 |

Mammaglobin-positive cells (MB415) were spiked into whole blood at various concentrations and then extracted using either epithelial cell enrichment or direct isolation from blood. Using enrichment procedures, mammaglobin mRNA was found to be detectable at much lower levels than when direct isolation was used. Whole blood samples from patients with metatastic breast cancer were subsequently treated with the immunomagnetic beads. Poly A⁺ RNA was then isolated, cDNA prepared and run in quantitative PCR using two gene specific primers (Table 1) and a fluorescent probe (Taqman^{™}). As observed in breast cancer tissues, complementation was also seen in the detection of circulating tumor cells derived from breast cancers. Again, mammaglobin PCR detected circulating tumor cells in a high percentage of blood samples, albeit at low levels, from metastatic breast cancer patients (20/32) when compared to the normal blood samples (Table 5) but several of the other genes tested to date further increased this detection rate. This included B726P, B305D, B311D, B533S and GABAπ. The detection level of mammaglobin in blood samples from metastatic breast cancer patients is higher than described previously (62 vs. 49%), despite testing smaller blood volumes, probably because of the use of epithelial marker-specific enrichment in our study. A combination of all the genes tested indicate that 27/32 samples were positive by one or more of these genes.

**Table 5**

| Gene Complementation in Epithelial Cells Isolated from Blood of Normal Individuals and Metastatic Breast Cancer Patients | | | | | | | |
|---|---|---|---|---|---|---|---|
| Sample ID | Mammaglobin | B305D | B311D | B533S | B726P | GABAπ | Combo |
| 2 | + | - | - | + | - | - | + |
| 3 | + | - | - | + | - | - | + |
| 5 | + | + | + | - | + | - | + |
| 6 | + | - | + | + | + | - | + |
| 8 | - | - | + | - | - | - | + |
| 9 | + | + | + | - | + | - | + |
| 10 | + | - | + | - | + | - | + |
| 11 | - | - | - | - | - | - | - |
| 12 | + | + | + | - | - | - | + |
| 13 | - | - | - | + | - | - | + |
| 15 | - | - | - | - | - | - | - |
| 18 | + | - | - | - | - | - | + |
| 19 | + | - | - | - | - | + | + |
| 21 | + | - | - | - | - | - | + |
| 22 | - | - | - | - | - | - | - |
| 23 | + | - | - | - | - | - | + |
| 24 | + | - | - | - | - | - | + |
| 25 | - | + | - | - | - | - | + |
| 26 | - | - | - | - | - | - | - |
| 29 | + | - | + | + | + | - | + |
| 31 | + | - | - | + | - | - | + |
| 32 | - | - | - | - | - | ± | ± |
| 33 | - | - | - | - | + | - | + |
| 34 | + | - | - | + | - | + | + |
| 35 | + | - | - | - | + | - | + |
| 36 | - | - | - | - | - | + | + |
| 37 | + | - | - | + | - | - | + |
| 38 | - | - | - | - | - | - | - |
| 40 | + | - | - | - | - | - | + |
| 41 | + | - | - | + | - | - | + |
| 42 | + | - | - | - | - | - | + |
| 43 | - | - | - | - | - | + | + |
| Donor 104 | - | - | - | - | - | + | + |
| Donor 348 | - | - | - | - | - | Nd | - |
| Donor 392 | - | - | - | - | - | Nd | - |
| Donor 408 | - | - | - | - | - | Nd | - |
| Donor 244 | - | - | - | - | - | - | - |
| Donor 355 | - | - | - | - | - | - | - |
| Donor 264 | - | - | - | - | - | - | - |
| Donor 232 | - | - | - | - | - | Nd | - |
| Donor 12 | - | - | - | - | - | - | - |
| Donor 415 | - | - | - | - | - | Nd | - |
| Donor 35 | - | - | - | - | - | - | - |
| Sensitivity | 20/32 | 4/32 | 7/32 | 9/32 | 7/32 | 4/32 | 27/32 |

In further studies, mammaglobin, GABAπ, B305D (C form) and B726P specific primers and specific Taqman probes were employed in different combinations to analyze their combined mRNA expression profile in breast metastases (B. met) and breast tumor (B. tumors) samples using real-time PCR. The forward and reverse primers and probes employed for mammaglobin, B305D (C form) and B726P are shown in Table 1. The forward primer and probe employed for GABAπ are shown in Table 1, with the reverse primer being as follows: TTCAAATATAAGTGAAGAAAAAATTAG-TAGATCAA (SEQ ID NO:51). As shown below in Table 6, a combination of mammaglobin, GABAπ, B305D (C form) and B726P was found to detect 22/22 breast tumor samples, with an increase in expression being seen in 5 samples (indicated by ++).

**Table 6**

| Real-time PCR Detection of Tumor Samples using Different Primer Combinations | | | | |
|---|---|---|---|---|
| Tumor sample | Mammaglobin | Mammaglobin + GABA | Mammaglobin + GABA + B305D | Mammaglobin + GABA + B305D + B726P |
| B. Met 316A | | + | + | + |
| B. Met 317A | + | + | + | + |
| B. Met 318A | | + | + | ++ |
| B. Met 595A | + | + | + | + |
| B. Met 611A | + | + | + | + |
| B. Met 612A | + | + | + | + |
| B. Met 614A | | + | + | + |
| B. Met 616A | | + | + | + |
| B. Met 618A | + | + | + | + |
| B. Met 620A | + | + | + | + |
| B. Met 621A | + | + | + | + |
| B. Met 624A | + | + | + | + |
| B. Met 625A | | | + | + |
| B. Met 627A | + | | + | + |
| B. Met 629A | | + | + | + |
| B. Met 631A | + | + | + | + |
| B. Tumor 154A | + | + | + | ++ |
| B. Tumor 155A | + | + | + | ++ |
| B. Tumor 81D | | | + | ++ |
| B. Tumor 209A | | + | + | + |
| B. Tumor 208A | | + | + | ++ |
| B. Tumor 10A | + | + | + | + |

The increase of message signals by the addition of specific primers was further demonstrated in a one plate experiment employing the four tumor samples B. met 316A, B. met 317A, B. tumor 81D and B. tumor 209A.

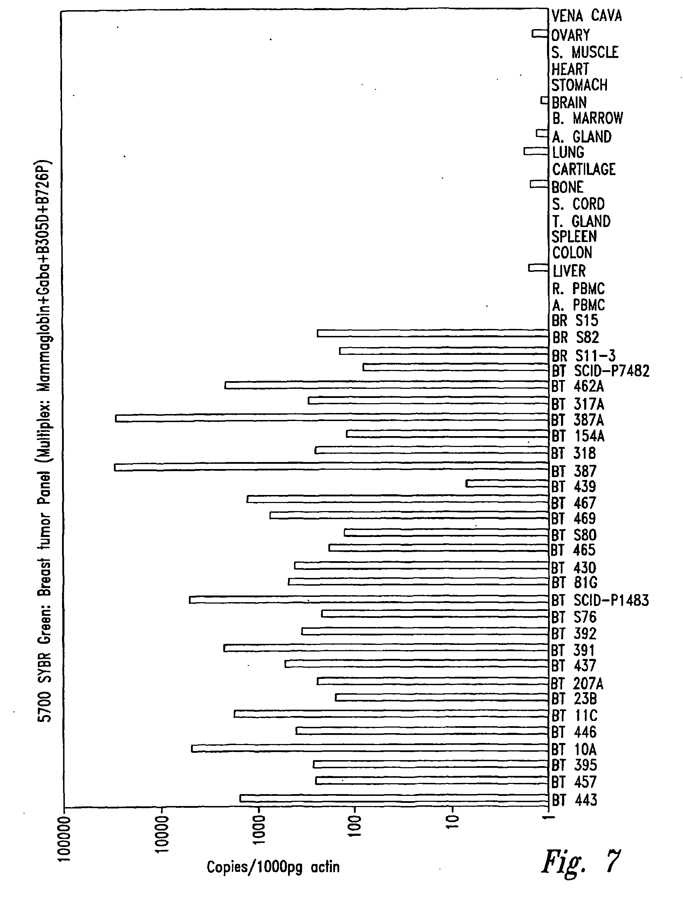

Expression of a combination of mammaglobin, GABAπ, B305D (C form) and B726P in a panel of breast tumor and normal tissue samples was also detected using real-time PCR with a SYBR Green detection system instead of the Taqman probe approach. The results obtained using this system are shown in Figure 7.

### EXAMPLE 7

### QUANTITATIVE PCR IN PERIPHERAL BLOOD OF BREAST CANCER PATIENTS

The known genes evaluated in this study were mammaglobin and γ aminobutyrate type A receptor π subunit (GABAπ). In order to identify novel genes which are over-expressed in breast cancer we have used an improved version of the differential display RT-PCR (DDPCR) technique (Liang et al., Science 257:967-971 (1993); Mou et al., Biochem Biophy Res Commun. 199:564-569 (1994)); cDNA library extraction methods (Hara et al., Blood 84:189-199 (1994)) and PCR subtraction (Diatchenko et al., *Proc Natl Acad Sci U S A*., 93:6025-6030 (1996); Yang et al., Nucleic Acids Res. 27:1517-23 (1999)).

Differential display resulted in the recovery of two cDNA fragments designated as B305D and B311D (Houghton et al., *Cancer Res*. 40:Abstract #217, 32-33, (1999). B511S and B533S are two cDNA fragments isolated using cDNA library subtraction approach (manuscript in preparation) while the B726P cDNA fragment was derived from PCR subtraction (Jiang et al., Proceedings of the Amer Assoc Cancer Res. 40:Abstract #216, 32 (1999); Xu et al., Proceedings of the Amer Assoc Cancer Res. 40:Abstract #2115, 319 (1999); and Molesh et al., Proceedings of Amer Assoc Cancer Res. 41:Abstract #4330, 681 (2000).

Three of the novel genes, B311D, B533S and B726P, showed identical breast tissue expression profile by quantitative PCR analysis. These genes were evaluated in quantitative PCR on two different panels consisting of (a) breast tumor, breast normal and normal tissues and (b) panel of breast tumor metastases (primarily lymph nodes). Primers and probes used are shown in Table 1. The data for panel (a) is shown in Figure 2 for all three genes. Overall, the expression profiles are comparable and are in the same rank order, however, the levels of expression are considerably different. B311D in general was expressed at lower levels than B533S and both less than B726P but all three were restricted to breast tissue. All three sequences were used to search against the Genbank database. Both B311D and B533S sequences contain different repetitive sequences and an ORF has not been identified for either. B726P is a novel gene, with mRNA splicing yielding several different putative ORF's.

The quantitative PCR confirmed there was a differential mRNA expression between normal breast tissue and breast tumors, with approximately 50% of breast tumors overexpressed these genes. When tested on a panel of distant metastases derived from breast cancers all three genes reacted with 14/21 metastases and presented similar profiles (data not shown). Interestingly, when tested on a prostate cancer panel, all three genes identified the same 3/24 prostate tumors but at much lower expression levels than in breast. This group of genes exhibited increasing levels of expression as a function of pathological stage of the tumor as shown for B533S.

More rigorous analysis of mammaglobin gene expression by microarray, and quantitative PCR showed expression at significant levels in skin and salivary gland and much lower levels in esophagus and trachea. B511S had a slightly different profile of reactivity on breast tumors and normal breast tissue when compared to mammaglobin, yet reacted with a similar subset of normal tissues as mammaglobin. Mammaglobin detected 14/24 of distant metastatic breast tumors, 31/42 breast tumors and exhibited ten-fold over-expression in tumors and metastases as compared to normal breast tissue. There was at least 300-fold over-expression of mammaglobin in normal breast tissue versus other negative normal tissues and tumors tested. B511S detected 33/42 breast tumors and 14/24 distant metastases. A combination of B511S with mammaglobin would be predicted to detect 38/42 breast tumors and 17/24 metastatic lesions. The quantitative level of expression of B511S and mammaglobin were also in similar ranges, in concordance with the microarray profiles observed for these two genes.

Certain genes complemented mammglobin's expression profile, *i*.*e*. were shown to express in tumors that mammaglobin did not. B305D was highly over-expressed in breast tumors, prostate tumors, normal prostate tissue and testis compared to normal tissues including normal breast tissue. Different splice variants of B305D were identified with the forms A and C being the most abundant. All forms tested had similar tissue profiles in quantitative PCR. The A and C forms contain ORF's of 320 and 385 aa, respectively. A known gene shown to be complementary with B305D, in breast tumors, was GABAπ. This tissue expression profile is in contrast to other GABA_{A} receptors that typically have appreciable expression in neuronal tissues. An additional observation was that tissue expression profiling of this gene showed it to be over-expressed in breast tumors in an inverse relationship to the B305D gene (Table 3). GABAπ detected 15/25 tumors and 6/21 metastases including 4 tumors and 5 metastases missed by mammaglobin. In contrast, B305D detected 13/25 breast tumors and 8/21 metastases again including 3 tumors and 2 metastases missed by mammaglobin. A combination of just B305D and the GABAπ would be predicted to identify 22/25 breast tumors and 14/21 metastases. This combination detected 20/21 of the breast metastases as well as 25/25 breast tumors that were evaluated on the same panels for all three genes. The one breast metastasis that was negative for these three genes was strongly positive for B726P.

The use of microarray analysis followed by quantitative PCR provided a methodology to accurately determine the expression of breast cancer genes both in breast tissues (tumor and normal) as well as in normal tissues and to assess their diagnostic and therapeutic potential. Five novel genes and two known genes were evaluated using these techniques. Three of these genes B311D, B533S and B726P exhibited concordant mRNA expression and collectively the data is consistent with coordinated expression of these three loci at the level of transcription control. All three genes showed differential expression in breast tumors versus normal breast tissue and the level of overexpression appeared related to the pathological stage of the tumor. In the case of mammaglobin, expression was found in other tissues apart from breast tissue. Expression was seen in skin, salivary gland and to a much lesser degree in trachea.

Expression of GABAπ in breast tumors was also a novel observation. While the expression of several genes complemented that seen with mammaglobin, two genes in particular, B305D and GABAπ added to the diagnostic sensitivity of mammaglobin detection. A combination of these three genes detected 45/46 (97.8%) breast tumors and metastases evaluated. Inclusion of B726P enabled the detection of all 25 of the breast tumors and 21 distant metastases.

### EXAMPLE 8

### ENRICHMENT OF CIRCULATING BREAST CANCER CELLS BY IMMUNOCAPTURE

This example discloses the enhanced sensitivity achieved by use of the immunocapture cell capture methodology for enrichment of circulating breast cancer cells.

To evaluate the presence of circulating tumor cells an immunocapture method was adopted to first enrich for epithelial cells prior to RT-PCR analysis. Epithelial cells were enriched from blood samples with an immunomagnetic bead separation method (Dynal A.S, Oslo, Norway) utilizing magnetic beads coated with monoclonal antibodies specific for glycopolypeptide antigens on the surface of human epithelial cells. (Exemplary suitable cell-surface antigens are described, for example, in Momburg, F. et al., Cancer Res., 41:2883-91 (1997); Naume, B. et al., *Journal of Hemotherapy*. 6:103-113 (1997); Naume, B. et al., Int J Cancer. 78:556-60 (1998); Martin, V.M. et al., Exp Hematol., 26:252-64 (1998); Hildebrandt, M. et al., *Exp Hematol.* 25:57-65 (1997); Eaton, M.C. et al., *Biotechniques* 22:100-5 (1997); Brandt, B. et al., *Clin Exp Metastases* 14:399-408 (1996). Cells isolated this way were lysed and the magnetic beads removed. The lysate was then processed for poly A⁺ mRNA isolation using magnetic beads (Dynabeads) coated with Oligo (dT) ₂₅. After washing the beads in the kit buffer bead/polyA⁺RNA samples were finally suspended in 10mM Tris HCl pH 8 and subjected to reverse transcription. The RNA was then subjected to Real time PCR using gene specific primers and probes with reaction conditions as outlined herein above. β-Actin content was also determined and used for normalization. Samples with gene of interest copies/ng β-actin greater than the mean of the normal samples + 3 standard deviations were considered positive. Real time PCR on blood samples was performed exclusively using the Taqman^{™} procedure but extending to 50 cycles.

Mammaglobin mRNA using enrichment procedures was found to be detectable at much lower levels than when direct isolation was used. Whole blood samples from patients with metatastic breast cancer were subsequently treated with the immunomagnetic beads, polyA⁺ RNA was then isolated, cDNA made and run in quantitative PCR using two gene specific primers to mammaglobin and a fluorescent probe (Taqman^{™}). As observed in breast cancer tissues, complementation was also seen in the detection of circulating tumor cells derived from breast cancers. Again, mammaglobin PCR detected circulating tumor cells in a high percentage of bloods, albeit at low levels, from metastatic breast cancer (20/32) when compared to the normal blood samples. Several of the other genes tested to date could further increase this detection rate; this includes B726P, B305D, B311D, B533S and GABAπ. A combination of all the genes tested indicates that 27/32 samples were positive by one or more of these genes.

### EXAMPLE 9

### MULTIPLEX DETECTION OF BREAST TUMORS

Additional Multiplex Real-time PCR assays were established in order to simultaneously detect the expression of four breast cancer-specific genes: LipophilinB, Gaba (B899P), B305D-C and B726P. In contrast to detection approaches relying on expression analysis of single breast cancer-specific genes, this Multiplex assay was able to detect all breast tumor samples tested.

This Multiplex assay was designed to detect LipophilinB expression instead of Mammaglobin. Due to their similar expression profiles, LipophilinB can replace Mammaglobin in this Multiplex PCR assay for breast cancer detection. The assay was carried out as follows: LipophilinB, B899P (Gaba), B305D, and B726P specific primers, and specific Taqman probes, were used to analyze their combined mRNA expression profile in breast tumors. The primers and probes are shown below:
LipophilinB: Forward Primer (SEQ ID NO: 33): 5' TGCCCCTCCGGAAGCT. Reverse Primer (SEQ ID NO:34): 5' CGTTTCTGAAGGGACATCTGATC. Probe (SEQ ID NO: 35) (FAM-5' - 3'-TAMRA): TTGCAGCCAAGTTAGGAGTGAAGAGATGCA.
GABA (B899P): Forward Primer (SEQ ID NO: 36): 5' AAGCCTCAGAGTCCTTCCAGTATG. Reverse Primer (SEQ ID NO: 37): 5' TTCAAATATAAGTGAAGAAAAAATTAGTAGATCAA. Probe (SEQ ID NO: 38) (FAM-5' - 3'-TAMRA): AATCCATTGTATCTTAGAACCGAGGGATTTGTTTAGA.
B305D (C form): Forward Primer (SEQ ID NO: 39): 5' AAAGCAGATGGTGGTTGAGGTT. Reverse Primer (SEQ ID NO: 40): 5' CCTGAGACCAAATGGCTTCTTC. Probe (SEQ ID NO: 41) (FAM-5'-3'-TAMRA) ATTCCATGCCGGCTGCTTCTTCTG.
B726P: Forward Primer (SEQ ID NO: 42): 5' TCTGGTTTTCTCATTCTTTATTCATTTATT. Reverse Primer (SEQ ID NO: 43): 5' TGCCAAGGAGCGGATTATCT. Probe (SEQ ID NO: 44) (FAM-5' - 3'-TAMRA): CAACCACGTGACAAACACTGGAATTACAGG.
Actin: Forward Primer (SEQ ID NO: 45): 5' ACTGGAACGGTGAAGGTGACA. Reverse Primer (SEQ ID NO 46): 5' CGGCCACATTGTGAACTTTG. Probe (SEQ ID NO: 47): (FAM-5' - 3'-TAMRA): CAGTCGGTTGGAGCGAGCATCCC.

The assay conditions were:

### Taqman protocol (7700 Perkin Elmer):

In 25 µl final volume: 1x Buffer A, 5mM MgCl, 0.2 mM dCTP, 0.2 mM dATP, 0.4 mM dUTP, 0.2 mM dGTP, 0.01 U/µl AmpErase UNG, 0.025 u/µl TaqGold, 8% (v/v) Glycerol, 0.05% (v/v) Gelatin, 0.01% (v/v) Tween20, 4 pmol of each gene specific Taqman probe (LipophilinB + Gaba + B305D + B726P), 100 nM of B726P-F + B726P-R, 300 nM of Gaba-R, and 50 nM of LipophilinB-F + LipophilinB-R + B305D-R + Gaba-R, template cDNA (originating from 0.02 µg polyA + RNA).

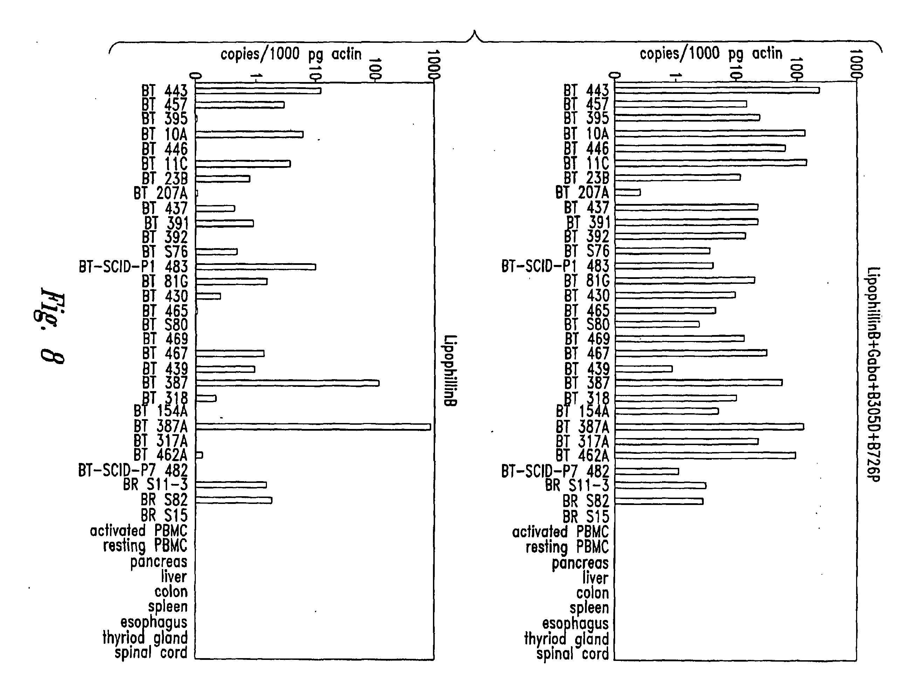

LipophilinB expression was detected in 14 out of 27 breast tumor samples.
However, the Multiplex assay for LipophilinB, B899P, B305D-C and B726P detected an expression signal in 27 out of 27 tumors with the detection level above 10 mRNA copies/1000 pg actin in the majority of samples and above 100 mRNA copies/1000 pg actin in 5 out of the 27 samples tested (Figure 8).

### EXAMPLE 10

### MULTIPLEX DETECTION OPTIMIZATION

The Multiplex Real-time PCR assay described above was used to detect the expression of Mammaglobin (or LipophilinB), Gaba (B899P), B305D-C and B726P simultaneously. According to this Example, assay conditions and primer sequences were optimized to achieve parallel amplification of four PCR products with different lengths. Positive samples of this assay can be further characterized by gel electrophoresis and the expressed gene(s) of interest can be determined according to the detected amplicon size(s).

Mammaglobin (or LipophilinB), Gaba (B899P), B305D and B726P specific primers and specific Taqman probes were used to simultaneously detect their expression. The primers and probes used in this example are shown below.
Mammaglobin: Forward Primer (SEQ ID NO: 48): 5' TGCCATAGATGAATTGAAGGAATG. Reverse Primer (SEQ ID NO: 49): 5' TGTCATATATTAATTGCATAAACACCTCA. Probe (SEQ ID NO: 50): (FAM-5' - 3'-TAMRA): TCTTAACCAAACGGATGAAACTCTGAGCAATG.
GABA (B899P): Forward Primer (SEQ ID NO: 36): 5' AAGCCTCAGAGTCCTTCCAGTATG. Reverse Primer (SEQ ID NO: 51): 5' ATCATTGAAAATTCAAATATAAGTGAAG. Probe (SEQ ID NO: 38) (FAM-5' - 3'-TAMRA) AATCCATTGTATCTTAGAACCGAGGGATTTGTTTAGA.
B305D (C form): Forward Primer (SEQ ID NO: 39): 5' AAAGCAGATGGTGGTTGAGGTT. Reverse Primer (SEQ ID NO: 40): 5' CCTGAGACCAAATGGCTTCTTC. Probe (SEQ ID NO: 41): (FAM-5' - 3'-TAMRA): ATTCCATGCCGGCTGCTTCTTCTG.
B726P: Forward Primer (SEQ ID NO: 52): 5' GTAGTTGTGCATTGAAATAATTATCATTAT. Reverse Primer (SEQ ID NO: 43): 5' TGCCAAGGAGCGGATTATCT. Probe (SEQ ID NO: 44) (FAM-5' - 3'-TAMRA): CAACCACGTGACAAACACTGGAATTACAGG.

Primer locations and assay conditions were optimized to achieve parallel amplification of four PCR products with different sizes. The assay conditions were:

### Taqman protocol (7700 Perkin Elmer):

In 25 µl final volume: 1x Buffer A, 5 mM MgCl, 0.2 mM dCTP, 0.2 mM dATP, 0.4 mM dUTP, 0.2 mM dGTP, 0.01 U/µl AmpErase UNG, 0.0375 U/µl TaqGold, 8% (v/v) Glycerol, 0.05% (v/v) Gelatin, 0.01% (v/v) Tween20, 4 pmol of each gene specific Taqman probe (Mammaglobin + Gaba + B305D + B726P), 300 nM of Gaba-R + Gaba-F, 100 nM of Mammaglobin-F + R; B726P-F + R, and 50 nM of B305D-F + R template cDNA (originating from 0.02 (µg polyA + RNA).

### PCR protocol:

50° for 2': x 1, 95° for 10': X 1, and 95° for 15" / 60 ° for 1' / 68 ° for 1': x 50.

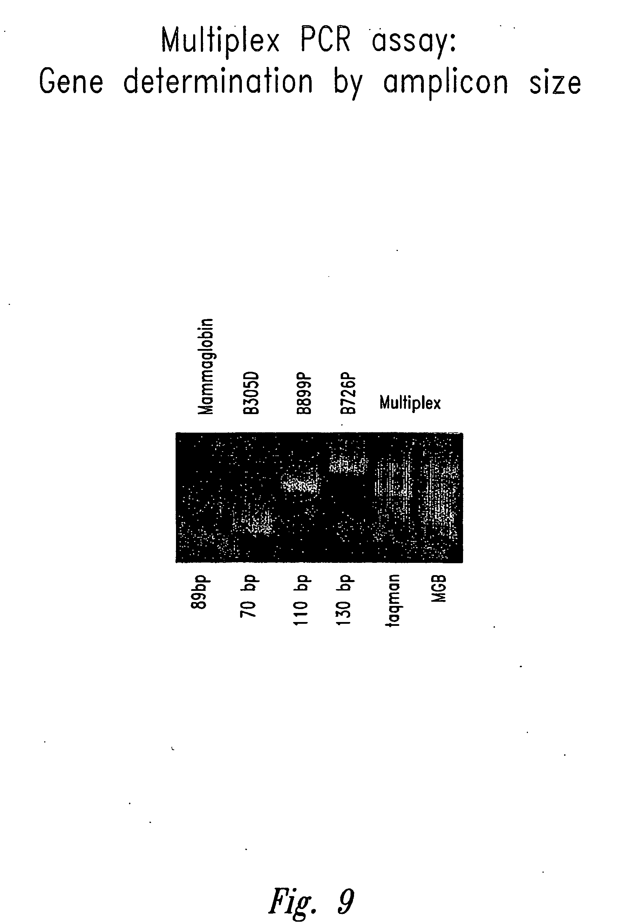

Since each primer set in the multiplex assay results in a band of unique length, expression signals of the four genes of interest can be measured individually by agarose gel analysis (*see,* Figure 9), or the combined expression signal of all four genes can be measured in real-time on an ABI 7700 Prism sequence detection system (PE Biosystems, Foster City, CA). The expression of LipophilinB can also be detected instead of Mammaglobin. Although specific primers have been described herein, different primer sequences, different primer or probe labeling and different detection systems could be used to perform this multiplex assay. For example, a second fluorogenic reporter dye could be incorporated for parallel detection of a reference gene by real-time PCR. Or, for example a SYBR Green detection system could be used instead of the Taqman probe approach.

### EXAMPLE 11

### DESIGN AND USE OF GENOMIC DNA-EXCLUDING, INTRON-EXON BORDER SPANNING PRIMER RAIRS FOR BREAST CANCER MULTIPLEX ASSAY

The Multiplex Real-time PCR assay described herein can detect the expression of Mammaglobin, Gaba (B899P), B305D-C and B726P simultaneously. The combined expression levels of these genes is measured in real-time on an ABI 7700 Prism sequence detection system (PE Biosystems, Foster City, CA). Individually expressed genes can also be identified due to different amplicon sizes via gel electrophoresis. In order to use this assay with samples derived from non-DNase treated RNAs (*e.g*. lymph node cDNA) and to avoid DNase-treatment for small RNA-samples (e.g. from blood specimens, tumor and lymph node aspirates), intron-spanning primer pairs have been designed to exclude the amplification of genomic DNA and therefore to eliminate nonspecific and false positive signals. False positive signal is caused by genomic DNA contamination in cDNA specimens. The optimized Multiplex assay described herein excludes the amplification of genomic DNA and allows specific detection of target gene expression without the necessity of prior DNase treatment of RNA samples. Moreover the genomic match and the location of the Intron-Exon border could be verified with these primer sets.

Mammaglobin, Gaba (B899P), B305D and B726P specific primers and specific Taqman probes were used to simultaneously detect their expression (Table 7). Primer locations were optimized (Intron-Exon border spanning) to exclusively detect cDNA and to exclude genomic DNA from amplification. The identity of the expressed gene(s) was determined by gel electrophoresis.

**Table 7**

| Intron-Exon border Spanning Primer and Probe Sequences for Breast Tumor Multiples Assay | | | |
|---|---|---|---|
| **Gene** | **Forward Primer** | **Reverse Primer** | **Taqman probe (FAM-5' - 3'TAMRA)** |
| **Mammaglobin** | tgccatagatgaattgaagga atg (SEQ ID NO:48) | tgtcatatattaattgcataaacacct ca (SEQ ID NO:49) | tcttaaccaaacggatgaaactctgagca atg (SEQ ID NO:50) |
| **B899P** | aagcctcagagtccttccagta tg (SEQ ID NO:36) | ttcaaatataagtgaagaaaaaatta gtagatcaa (SEQ ID NO:37) | aatccattgtatcttagaaccgagggattt gttt (SEQ ID NO:62) |
| **B305D** | aaagcagatggtggttgaggt t (SEQ ID NO:39) | cctgagaccaaatggcttcttc (SEQ ID NO:40) | attccatgccggctgcttcttctg (SEQ ID NO:41) |
| **B726P** | tctggttttctcattctttattcatt tatt (SEQ ID NO:42) | tgccaaggagcggattatct (SEQ ID NO:43) | caaccacgtgacaaacactggaattaca gg (SEQ ID NO:44) |
| **Actin** | actggaacggtgaaggtgac a (SEQ ID NO:45 | cggccacattgtgaactttg (SEQ ID NO:46) | cagtcggttggagcgagcatccc (SEQ ID NO:47) |
| **B899P-INT** | caattttggtggagaacccg (SEQ ID NO:53) | gctgtcggaggtatatggtg (SEQ ID NO:54) | catttcagagagtaacatggactacaca (SEQ ID NO:55) |
| **B305D-INT** | tctgataaaggccgtacaatg (SEQ ID NO:56) | tcacgacttgctgtttttgctc (SEQ ID NO:57) | atcaaaaaacaagcatggcctcacacca ct (SEQ ID NO:58) |
| **B726P-INT** | gcaagtgccaatgatcagagg (SEQ ID NO:59) | atatagactcaggtatacacact (SEQ ID NO:60) | tcccatcagaatccaaacaagaggaaga tg (SEQ ID NO:61) |

Primer locations and assay conditions were optimized to achieve parallel amplification of the four PCR products. The assay conditions were as follows:

### Taqman protocol (7700 Perkin Elmer)

In 25µl final volume: 1x Buffer A, 5 mM MgCl, 0.2 mM dCTP, 0.2 mM dATP, 0.4 mM dUTP, 0.2 mM dGTP, 0.01 U/AmpErase UNG, 8 % (v/v) Glycerol, 0.05 % (v/v) Gelatin, 0.01 % (v/v) Tween20, 4 pmol of each gene specific Taqman probe (Mammaglobin + B899P-INT + B305D-INT + B726P-INT), 300 nM of B305DINT-F; B899P-INT-F, 100 nM of Mammaglobin-F + R; B726P-INT-F +R, 50 nM of B899P-INT-R; B305D-INT-R, template cDNA (originating from 0.02 µg polyA+ RNA).

### PCR cycling conditions

1 cycle at 50°C for 2 minutes, 1 cycle at 95°C for 10 minutes, 50 cycles of 95°C for 1 minute and 68°C for 1 minute.

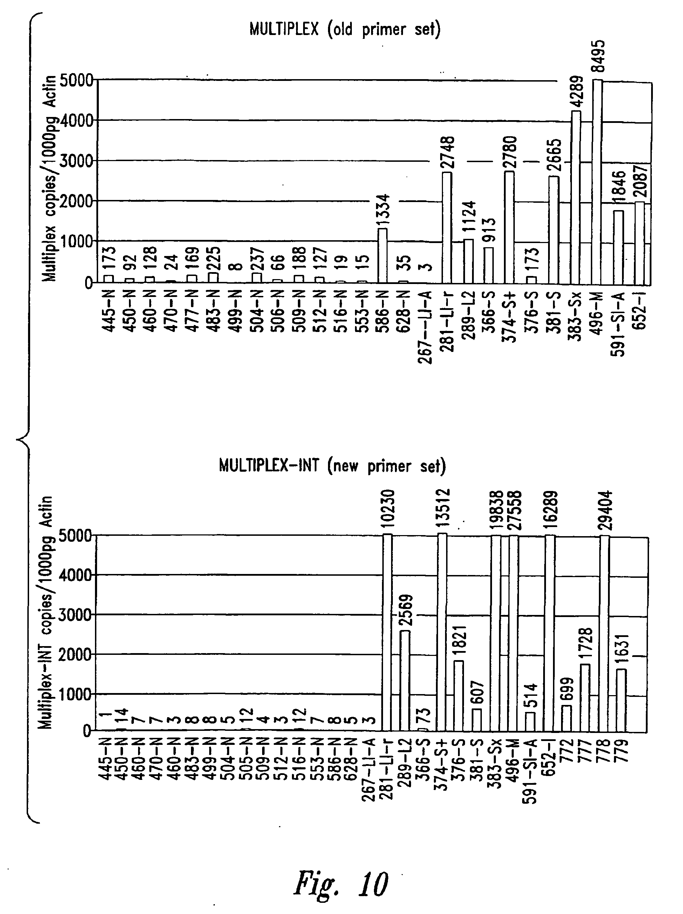

Figure 10 shows a comparison of the multiplex assay using intron-exon border spanning primers (bottom panel) and the multiplex assay using non-optimized primers (top panel), to detect breast cancer cells in a panel of lymph node tissues. This experiment shows that reduction in background resulting from genomic DNA contamination in samples is achieved using the intron-exon spanning primers of the present invention.

### EXAMPLE 12

### MULTIPLEX DETECTION OF METASTASIZED BREAST TUMOR CELLS IN

### SENTINEL LYMPH NODE BIOPSY SAMPLES

Lymph node staging is important for determining appropriate adjuvant hormone and chemotherapy. In contrast to conventional axillary dissection a less invasive approach for staging of minimal residual disease is sentinel lymph node biopsy. Sentinel lymph node biopsy (SLNB) has the potential to improve detection of metastases and to provide prognostic values to lead to therapy with minimal morbidity associated with complete lymph node dissection. SLNB implements mapping of the one or two lymph nodes which primarily drain the tumor and therefore are most likely to harbor metastatic disease (the sentinel nodes). Routine pathological' analysis of lymph nodes result in a high false-negative rate: one-third of women with pathologically negative lymph nodes develop recurrent disease [Bland: The Breast: Saunders 1991]. A more sensitive detection technique for tumor cells would be RT-PCR but its application is limited by lack of a single specific markers. The multimarker assay described above increases the likelihood of cancer detection across the population without producing false-positive results from normal lymph nodes.

As mentioned above, lymphatic afferents from a primary tumor drain into a single node, the sentinel lymph node, before drainage into the regional lymphatic basin occurs. Sentinel lymph nodes are located with dyes and/or radiolabelled colloid injected in the primary lesion site and sentinel lymph node biopsy allows pathological examination for micrometastatic deposits, staging of the axilla and therefore can avoid unnecessary axillary dissection. Nodal micrometastases can be located with staining (haematoxylin or eosin) or immunohistochemical analysis for cytokeratin proteins. Immunocytochemical staining techniques can produce frequent false-negative results by missing small metastatic foci due to inadequate sectioning of the node. Immunohistochemistry can result in false-positive results due to illegitimate expression of cytokeratins (reticulum cells) or in false-negative results when using the antibody Ber-Ep4 which corresponding antigen is not expressed on all tumor cells.

The multiplex assay described herein could provide a more sensitive detection tool for positive sentinel lymph nodes. Moreover the detection of breast cancer cells in bone marrow samples, peripheral blood and small needle aspiration samples is desirable for diagnosis and prognosis in breast cancer patients.

Twenty-two metastatic lymph node samples, in addition to 15 samples also previously analyzed and shown in Figure 3A, were analyzed using the intron-exon border spanning multiplex PCR assay described herein. The results from this analysis are summarized in Table 8. Twenty-seven primary tumors were also analyzed and the results shown in Table 9. Twenty normal lymph node samples tested using this assay were all negative.

**Table 8.**

| Multiples Real-time PCR Analysis of 37 Metastatic Lymph Nodes | | | | | |
|---|---|---|---|---|---|
| breast metastatic lymph node samples | Mammaglobin | B305D | B899P | B726P | Multiplex |
| B.Met 317A | ++ | + | | + | +++ |
| B.Met 318A | | | ++ | | +++ |
| B.Met 595A | + | | | + | +++ |
| B.Met 611A | + | + | +++ | | ++ |
| B.Met 612A | ++ | ++ | | + | ++ |
| B.Met 614A | | ++ | | ++ | +++ |
| B.Met 616A | | | + | | ++ |
| B.Met 618A | +++ | + | | | +++ |
| B.Met 620A | ++ | ++ | | ++ | +++ |
| B.Met 621A | + | +++ | | + | +++ |
| B.Met 624A | | | ++ | | +++ |
| B.Met 625A | | ++ | | ++ | + |
| B.Met 627A | | + | | + | + |
| B.Met 629A | ++ | | | | +++ |
| B.Met 631A | + | | ++ | | + |
| 1255 | +++ | ++ | | ++ | ++ |
| 1257 | +++ | + | + | + | ++ |
| 769 | +++ | | | + | ++ |
| 1258 | ++ | + | + | | + |
| 1259 | | ++ | ++ | | +++ |
| 1250 | +++ | + | | + | +++ |
| 1726 | +++ | + | | + | +++ |
| 786 | +++ | + | + | | +++ |
| 281-LI-r | +++ | | | | +++ |
| 289-L2 | ++ | + | | | ++ |
| 366-S | + | | | | + |
| 374-S+ | +++ | ++ | | | +++ |
| 376-S | ++ | | | + | ++ |
| 381-S | + | + | | | + |
| 383-Sx | +++ | ++ | | | +++ |
| 496-M | +++ | ++ | | | +++ |
| 591-SI-A | + | + | | | + |
| 652-1 | | + | ++ | | +++ |
| 772 | + | | | | + |
| 777 | + | + | | ++ | ++ |
| 778 | +++ | | | | +++ |
| 779 | + | | ++ | | ++ |

**Table 9**

| Multiplex Real-time PCR Analysis of 27 Primary Breast Tumors | | | | | |
|---|---|---|---|---|---|
| breast primary tumor samples | Mammaglobin | B305D | B899P | B726P | Multiplex |
| T443 | + | ++ | | +++ | +++ |
| T457 | | + | + | | ++ |
| T395 | | | ++ | | ++ |
| T10A | + | +++ | | +++ | +++ |
| T446 | | + | | ++ | ++ |
| T11C | + | | +++ | | +++ |
| T23B | + | ++ | | | +++ |
| T207A | | ++ | | | + |
| T437 | + | + | | ++ | +++ |
| T391 | + | ++ | | +++ | +++ |
| T392 | + | + | | | ++ |
| TS76 | + | ++ | | | +++ |
| T483 | ++ | + | | | +++ |
| T81G | + | + | ++ | ++ | +++ |
| T430 | + | | ++ | | ++ |
| T465 | + | + | | + | ++ |
| TS80 | | | + | | + |
| T469 | + | | | + | +++ |
| T467 | + | | | ++ | +++ |
| T439 | | + | | | + |
| T387 | ++ | | + | + | ++ |
| T318 | | | + | | ++ |
| T154A | | | | + | + |
| T387A | +++ | | + | + | +++ |
| T155A | + | | ++ | + | + |
| T209A | | ++ | | | ++ |
| T208A | | + | | + | ++ |

From the foregoing it will be appreciated that, although specific embodiments of the invention have been described herein for purposes of illustration, various modifications may be made without deviating from the scope of the invention. Accordingly, the invention is not limited except as by the appended claims.

## Claims

1. A method for detecting the presence of a breast cancer cell, said method comprising the steps of:
(a) contacting a biological sample obtained from a patient with a first oligonucleotide that hybridizes to a first polynucleotide selected from the group consisting of mammaglobin and lipophilin B;
(b) contacting said biological sample with a second oligonucleotide that hybridizes to a second polynucleotide sequence comprising a GABAπ (B899P) polynucleotide sequence;
(c) detecting in said biological sample an amount of hybridized oligonucleotide of steps (a) and (b); and
(d) comparing the amount of polynucleotide that hybridizes to the oligonucleotides of steps (a) and (b) to a predetermined cut-off value, and therefrom determining the presence or absence of a cancer in the patient.

2. The method of claim 1 wherein said first polynucleotide is selected from the group consisting of polynucleotides depicted in SEQ ID NO:73, SEQ ID NO:74 and SEQ ID NO:76, or the complement thereof; and wherein said second polynucleotide comprises the polynucleotide set forth in SEQ ID NO: 75, or the complement thereof.

3. The method of claim 2 further comprising:
(e) contacting said biological sample with a third oligonucleotide that hybridizes to a third polynucleotide selected from the group consisting of polynucleotides depicted in SEQ ID NO:5, SEQ ID NO:6 and SEQ ID NO:7; or the complement thereof;
(f) contacting said biological sample with a fourth oligonucleotide that hybridizes to a fourth polynucleotide selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24; or the complement thereof and
wherein step (c) comprises detecting in said biological sample an amount of hybridized oligonucleotide of steps (a), (b), -(e), and (f).

4. The method of claim 2 further comprising:
(e) contacting said biological sample with a third oligonucleotide that hybridizes to a third polynucleotide selected from the group consisting of a polynucleotide depicted in SEQ ID NO:1, SEQ ID NO:3, SEQ ID NO:5, SEQ ID NO:6 and SEQ ID NO:7; or the complement thereof;
(f) contacting said biological sample with a fourth oligonucleotide that hybridizes to a fourth polynucleotide selected from the group consisting of a polynucleotide depicted in SEQ ID NO:11, or the complement thereof;
(g) contacting said biological sample with a fifth oligonucleotide that hybridizes to a fifth polynucleotide selected from the group consisting of a polynucleotide depicted in SEQ ID NO:13, 15 and 17; or the complement thereof;
(h) contacting said biological sample with a sixth oligonucleotide that hybridizes to a sixth polynucleotide selected from the group consisting of a polynucleotide depicted in SEQ ID NO:19, SEQ ID NO:20, SEQ ID NO:21, SEQ ID NO:22, SEQ ID NO:23 and SEQ ID NO:24; or the complement thereof;
(i) contacting said biological sample with a seventh oligonucleotide that hybridizes to a seventh polynucleotide depicted in SEQ ID NO:30 or the complement thereof;
(j) contacting said biological sample with an eighth oligonucleotide that hybridizes to an eighth polynucleotide depicted in SEQ ID NO:32 or the complement thereof; and
(k) contacting said biological sample with a ninth oligonucleotide that hybridizes to a polynucleotide depicted in SEQ ID NO:76 or the complement thereof;
wherein step (c) comprises detecting in said biological sample an amount of hybridized oligonucleotide of steps (a), (b), (e), (f), (g), (h), (i), (j), and (k).

5. A method for detecting the presence of a breast cancer cell, said method comprising the steps of:
(a) contacting a biological sample obtained from a patient with a first oligonucleotide pair said first pair comprising a first oligonucleotide and a second oligonucleotide wherein said first oligonucleotide and said second oligonucleotide hybridize to a first polynucleotide and the complement thereof, respectively, wherein said first polynucleotide comprises mammaglobin or lipophilin B;
(b) contacting said biological sample with a second oligonucleotide pair said second pair comprising a third oligonucleotide and a fourth oligonucleotide wherein said third and said fourth oligonucleotide hybridize to a second polynucleotide and the complement thereof, respectively, and wherein said second polynucleotide comprises GABAπ (B899P);
(c) amplifying said first polynucleotide and said second polynucleotide; and
(d) detecting said amplified first polynucleotide and said amplified second polynucleotide;
wherein the presence of said amplified first polynucleotide or said amplified second polynucleotide indicates the presence of a breast cancer cell in said patient.

6. The method of claim 5 wherein said first polynucleotide is selected from the group consisting of polynucleotides depicted in SEQ ID NO:73. SEQ ID NO:74 and SEQ ID NO:76; and wherein said second polynucleotide comprises the polynucleotide set forth in SEQ ID NO: 75.

7. The method of claim 6 further comprising contacting said biological sample with a third oligonucleotide pair said third pair comprising a forward primer and a reverse primer that hybridize to a third polynucleotide and the complement thereof, respectively, wherein said third polynucleotide is selected from the group consisting of polynucleotides depicted in SEQ ID NO:5, SEQ ID NO:6 and SEQ ID NO:7; wherein step (d) further comprises amplifying said third polynucleotide; and wherein step (e) further comprises detecting said third polynucleotide.

8. The method of claim 7 further comprising contacting said biological sample with a fourth oligonucleotide pair said fourth pair comprising a forward primer and a reverse primer that hybridize to a fourth polynucleotide and the complement thereof, respectively, wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24; wherein step (d) further comprises amplifying said fourth polynucleotide; and wherein step (e) further comprises detecting said fourth polynucleotide.

9. The method of any one of claims 1-8 wherein said biological sample is selected from the group consisting of blood, serum, lymph node, bone marrow, sputum, urine and tumor biopsy sample.

10. The method of claim 9 wherein said biological sample is selected from the group consisting of blood, a lymph node and bone marrow.

11. The method of claim 9 wherein said lymph node is a sentinel lymph node.

12. The method of any one of claims 5-8 wherein said oligonucleotides are selected from the group consisting of oligonucleotides depicted in SEQ ID NOs:33-38, 48-51, and 53-62.

13. The method of any one of claims 5-8 wherein the step (e) comprises a step selected from the group consisting of detecting a radiolabel and detecting a fluorophore.

14. The method of any one of claims 1-8 wherein said step of detection comprises a step of fractionation.

15. The method of any one of claims 5-8 wherein said oligonucleotides are intron spanning oligonucleotides.

16. The method of claim 15 wherein said intron spanning oligonucleotides are selected from the group consisting of oligonucleotides depicted in SEQ ID NOs:36-44 and 48-62.

17. The method of any one of claims 5-8 wherein detection of said amplified first, second, third, or fourth polynucleotide comprises contacting said amplified first, second, third or fourth polynucleotide with a labeled oligonucleotide probe that hybridizes, under moderately stringent conditions, to said first, second, third, or fourth polynucleotide.

18. The method of claim 17 wherein said labeled oligonucleotide probe comprises a detectable moiety selected from the group consisting of a radiolabel and a fluorophore.

19. The method of any one of claims 1-8 further comprising a step of enriching said cancer cell from said biological sample prior to hybridizing said oligonucleotide primer(s).

20. The method of claim 1-8 wherein said step of enriching said cancer cell from said biological sample is achieved by a methodology selected from the group consisting of cell capture and cell depletion.

21. The method of claim 20 wherein cell capture is achieved by immunocapture, said immunocapture comprising the steps of:
(a) adsorbing an antibody to the surface of said cancer cells; and
(b) separating said antibody adsorbed cancer cells from the remainder of said biological sample.

22. The method of claim 21 wherein said antibody is directed to an antigen selected from the group consisting of CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE and TCRαβ.

23. The method of claim 21 wherein said antibody is directed to a breast tumor antigen.

24. The method of any one of claims 21-23 wherein said antibody is a monoclonal antibody.

25. The method of claim 21 wherein said antibody is conjugated to magnetic beads.

26. The method of claim 21 wherein said antibody is formulated in a tetrameric antibody complex.

27. The method of claim 21 wherein cell depletion is achieved by a method comprising the steps of:
(a) cross-linking red cells and white cells, and
(b) fractionating said cross-linked red and white cells from the remainder of said biological sample.

28. The method of any one of claims 5-8 wherein said step of amplifying is achieved by a polynucleotide amplification methodology selected from the group consisting of reverse transcription polymerase chain reaction (RT-PCR), inverse PCR, RACE, ligase chain reaction (LCR), Qbeta Replicase, isothermal amplification, strand displacement amplification (SDA), rolling chain reaction (RCR), cyclic probe reaction (CPR), transcription-based amplification systems (TAS), nucleic acid sequence based amplification (NASBA) and 3SR.

29. A composition for detecting a breast cancer cell in a biological sample of a patient, said composition comprising:
(a) a first oligonucleotide; and
(b) a second oligonucleotide;
wherein said first oligonucleotide and said second oligonucleotide are at least 15 nucleotides in length and specifically hybridize to a first polynucleotide or the complement thereof and to a second polynucleotide or the complement thereof, respectively; wherein said first polynucleotide is selected from the group consisting of SEQ ID NO:73, SEQ ID NO:74 and SEQ ID NO:76; and wherein said second polynucleotide comprises the polynucleotide set forth in SEQ ID NO: 75.

30. The composition of claim 29 further comprising a third and a fourth oligonucleotide wherein said third and said fourth oligonucleotide hybridize to a third polynucleotide or the complement thereof and a fourth polynucleotide or the complement thereof, respectively; wherein said third polynucleotide is selected from the group consisting of polynucleotides depicted in SEQ ID NO:5, SEQ ID NO:6 and SEQ ID NO:7; and wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24.

31. The composition of claim 30 or claim 31 wherein said oligonucleotides are selected from the group consisting of oligonucleotides as disclosed in SEQ ID NOs: 33-44, 48-62, and 72.

32. A composition for detecting a breast cancer cell in a biological sample of a patient, said composition comprising:
(a) a first oligonucleotide pair; and
(b) a second oligonucleotide pair;
wherein said first oligonucleotide pair and said second oligonucleotide pair are at least 10 nucleotides in length and specifically hybridize to a first polynucleotide or the complement thereof and to a second polynucleotide or the complement thereof, respectively; wherein said first polynucleotide is selected from the group consisting of SEQ ID NO:73, SEQ ID NO:74 and SEQ ID NO:76; and wherein said second polynucleotide comprises the polynucleotide set forth in SEQ ID NO: 75.

33. The composition of claim 32 further comprising a third oligonucleotide pair and a fourth oligonucleotide pair wherein said third oligonucleotide pair and said fourth oligonucleotide pair hybridize to a third polynucleotide or the complement thereof and to a fourth polynucleotide or the complement thereof, respectively; wherein said third polynucleotide is selected from the group consisting of polynucleotides depicted in SEQ ID NO:5, SEQ ID NO:6 and SEQ ID NO:7; and wherein said fourth polynucleotide is selected from the group consisting of polynucleotides depicted in SEQ ID NO:13, SEQ ID NO:15, SEQ ID NO:17, SEQ ID NO:19, SEQ ID NO:20, SEQ ID NO:21, SEQ ID NO:22, SEQ ID NO:23 and SEQ ID NO:24.

34. The composition of claim 32 or claim 33 wherein said oligonucleotides are selected from the group consisting of oligonucleotides as disclosed in SEQ ID NOs: 33-44, 48-62, and 72.

35. A kit for the detection of breast cancer, including the composition of any one of claims 29 to 34.

## Patentansprüche

1. Verfahren zum Nachweis der Anwesenheit von Brustkrebszellen umfassend die Schritte, dass
(a) eine biologische Probe, die von einem Patienten erhalten wurde, mit einem ersten Oligonukleotid in Verbindung gebracht wird, das mit einem ersten Polynukleotid hybridisiert, ausgewählt aus der Gruppe bestehend aus Mammaglobin und Lipophilin B;
(b) dass die biologische Probe mit einem zweiten Oligonukleotid, das mit einer zweiten Polynukleotidsequenz hybridisiert, enthaltend eine GABAπ (B899P)-Polynukleotidsequenz, in Verbindung gebracht wird;
(c) die Menge an hybridisierten Oligonukleotiden der Schritte (a) und (b) in der biologischen Probe nachgewiesen wird; und
(d) die Menge an Polynukleotiden, die mit den Oligonukleotiden der Schritte (a) und (b) hybridisiert mit einem vorbestimmten Ausschlusswert verglichen wird, und daraus die An- oder Abwesenheit von Krebs bei einem Patienten bestimmt wird.

2. Verfahren nach Anspruch 1, wobei das erste Polynukleotid aus der Gruppe bestehend aus Polynukleotiden wie in SEQ ID NO:73, SEQ ID NO:74 und SEQ ID NO:76 gezeigt, oder deren Komplement ausgewählt ist; und wobei das zweite Polynukleotid dasjenige Polynukleotid enthält, das in SEQ ID NO:75 dargestellt ist, oder ein Komplement davon.

3. Verfahren nach Anspruch 2, ferner umfassend, dass
(e) die biologische Probe mit einem dritten Oligonukleotid, das mit einem dritten Polynukleotid hybridisiert, ausgewählt aus der Gruppe bestehend aus Polynukleotiden wie in SEQ ID NO:5, SEQ ID NO:6 und SEQ ID NO:7 gezeigt, oder einem Komplement davon in Verbindung gebracht wird;
(f) die biologische Probe mit einem vierten Oligonukleotid, das mit einem vierten Polynukleotid hybridisiert, ausgewählt aus der Gruppe bestehend aus Polynukleotiden wie in SEQ ID NO:13, SEQ ID NO:15, SEQ ID NO:17, SEQ ID NO:19, SEQ ID NO:20, SEQ ID NO:21, SEQ ID NO:22, SEQ ID NO:23 und SEQ ID NO:24 gezeigt, oder einem Komplement davon in Verbindung gebracht wird und
wobei
Schritt (c) den Nachweis der Menge an hybridisiertem Oligonukleotid der Schritte (a), (b), (e) und (f) in der biologischen Probe umfasst.

4. Verfahren nach Anspruch 2 ferner umfassend, dass
(e) die biologische Probe mit einem dritten Oligonukleotid, das mit einem dritten Polynukleotid hybridisiert, ausgewählt aus der Gruppe bestehend aus Polynukleotiden wie in SEQ ID NO:1, SEQ ID NO:3, SEQ ID NO:5, SEQ ID NO:6 und SEQ ID NO:7 gezeigt, oder einem Komplement davon in Verbindung gebracht wird;
(f) die biologische Probe mit einem vierten Oligonukleotid, das mit einem vierten Polynukleotid hybridisiert, ausgewählt aus der Gruppe bestehend aus Polynukleotiden wie in SEQ ID NO:11 gezeigt, oder einem Komplement davon in Verbindung gebracht wird;
(g) die biologische Probe mit einem fünften Oligonukleotid, das mit einem fünften Polynukleotid hybridisiert, ausgewählt aus der Gruppe bestehend aus Polynukleotiden wie in SEQ ID NO:13, 15 und 17 gezeigt, oder einem Komplement davon in Verbindung gebracht wird;
(h) die biologische Probe mit einem sechsten Oligonukleotid, das mit einem sechsten Polynukleotid hybridisiert, ausgewählt aus der Gruppe bestehend aus Polynukleotiden wie in SEQ ID NO:19, SEQ ID NO:20, SEQ ID NO:21, SEQ ID NO:22, SEQ ID NO: 23 und SEQ ID NO: 24 gezeigt, oder einem Komplement davon in Verbindung gebracht wird;
(i) die biologische Probe mit einem siebten Oligonukleotid, das mit einem siebten Polynukleotid hybridisiert wie in SEQ ID NO:30 gezeigt, oder einem Komplement davon in Verbindung gebracht wird;
(j) die biologische Probe mit einem achten Oligonukleotid, das mit einem achten Polynukleotid hybridisiert wie in SEQ ID NO:32 gezeigt, oder einem Komplement davon in Verbindung gebracht wird; und
(k) die biologische Probe mit einem neunten Oligonukleotid, das mit einem neunten Polynukleotid hybridisiert wie in SEQ ID NO:76 gezeigt, oder einem Komplement davon in Verbindung gebracht wird;
wobei Schritt (c) den Nachweis der Menge an hybridisiertem Oligonukleotid der Schritte (a), (b), (e), (f), (g), (h), (i), (j) und (k) in der biologischen Probe umfasst.

5. Verfahren zum Nachweis der Anwesenheit von Brustkrebszellen umfassend die Schritte, dass
(a) eine biologische Probe, die von einem Patienten erhalten wurde mit einem ersten Oligonukleotidpaar in Kontakt gebracht wird, wobei das erste Paar ein erstes und ein zweites Oligonukleotid enthält, wobei das erste Oligonukleotid und das zweite Oligonukleotid mit einem ersten Polynukleotid bzw. dem Komplement davon hybridisieren, wobei das erste Polynukleotid Mammaglobulin oder Lipophilin B enthält.
(b) die biologische Probe mit einem zweiten Oligonukleotidpaar in Kontakt gebracht wird, wobei das zweite Paar ein drittes Oligonukleotid und ein viertes Oligonukleotid enthält, wobei das dritte und vierte Oligonukleotid mit einem zweiten Polynukleotid bzw. dem Komplement davon hybridisieren, wobei das zweite Polynukleotid GABAπ (B899P) enthält;
(c) das erste Polynukleotid und das zweite Polynukleotid amplifiziert werden; und
(d) das amplifizierte erste Polynukleotid und das amplifizierte zweite Polynukleotid nachgewiesen werden; wobei
die Anwesenheit des amplifizierten ersten Polynukleotids oder des amplifizierten zweiten Polynukleotids auf die Anwesenheit von Brustkrebszellen im Patienten hindeutet.

6. Verfahren nach Anspruch 5, wobei das erste Polynukleotid aus der Gruppe, bestehend aus Polynukleotiden wie in SEQ ID NO:73, SEQ ID NO:74 und SEQ ID NO:76 gezeigt ausgewählt ist und wobei das zweite Polynukleotid das Polynucleotid, wie in SEQ ID NO:75 gezeigt, enthält.

7. Verfahren nach Anspruch 6 ferner umfassend, dass die biologische Probe mit einem dritten Oligonukleotidpaar in Verbindung gebracht wird, wobei das dritte Paar einen Vorwärts-Primer und einen Rückwärts-Primer enthält, die mit einem dritten Polynukleotid bzw. dem Komplement davon hybridisieren, wobei das dritte Polynukleotid aus einer Gruppe bestehend aus Polynukleotiden wie in SEQ ID NO:5, SEQ ID NO:6 und SEQ ID NO:7 gezeigt ausgewählt ist; wobei Schritt (d) ferner die Amplikation des dritten Polynukleotids umfasst; und wobei Schritt (e) ferner den Nachweis des dritten Polynukleotids umfasst.

8. Verfahren nach Anspruch 7 umfassend, dass die biologische Probe mit einem vierten Oligonukleotidpaar in Kontakt gebracht wird, wobei das vierte Paar einen Vorwärts-Primer und einen Rückwärts-Primer enthält, die mit einem vierten Polynukleotid bzw. dem Komplement davon hybridisieren, wobei das vierte Polynukleotid aus einer Gruppe bestehend aus Polynukleotiden wie in SEQ ID NO:13, SEQ ID NO:15, SEQ ID NO:17, SEQ ID NO:19, SEQ ID NO:20, SEQ ID NO:21, SEQ ID NO:22, SEQ ID NO:23 und SEQ ID NO:24 gezeigt ausgewählt ist; wobei Schritt (d) ferner die Amplikation des vierten Polynukleotids umfasst; und wobei Schritt (e) ferner den Nachweis des vierten Polynukleotids umfasst.

9. Verfahren nach einem der Ansprüche 1 bis 8, wobei die biologische Probe aus einer Gruppe bestehend aus Blut, Serum, Lymphknoten, Knochenmark, Sputum, Urin und Tumorbiopsieproben ausgewählt ist.

10. Verfahren nach Anspruch 9, wobei die biologische Probe aus einer Gruppe bestehend aus Blut, Lymphknoten und Knochenmark ausgewählt ist.

11. Verfahren nach Anspruch 9, wobei der Lymphknoten ein Indikatorlymphknoten ist.

12. Verfahren nach einem der Ansprüche 5 bis 8, wobei die Oligonukleotide aus einer Gruppe, bestehend aus Oligonukleotiden wie in SEQ ID NOs: 33-38, 48-51 und 53-62 gezeigt, ausgewählt sind.

13. Verfahren nach einem der Ansprüche 5 bis 8, wobei der Schritt (e) einen Schritt, ausgewählt aus der Gruppe bestehend aus dem Nachweis einer radioaktiven Markierung und Nachweis eines Fluorophors, umfasst.

14. Verfahren nach einem der Ansprüche 1 bis 8, wobei der Schritt zum Nachweis einen Schritt zur Fraktionierung umfasst.

15. Verfahren nach einem der Ansprüche 5 bis 8, wobei die Oligonukleotide Intron überspannende Oligonukleotide sind.

16. Verfahren nach Anspruch 15, wobei die intronüberspannenden Oligonukleotide aus einer Gruppe bestehend aus Oligonukleotiden wie in SEQ ID Nos: 36-44 und 48-62 gezeigt ausgewählt sind.

17. Verfahren nach einem der Ansprüche 5 bis 8, wobei der Nachweis des amplifizierten ersten, zweiten, dritten und vierten Polynukleotids umfasst, dass das amplifizierte erste, zweite, dritte und vierte Polynukleotid mit einer markierten Oligonukleotidsonde in Kontakt gebracht wird, die unter mäßig stringenten Bedingungen mit einem ersten, zweiten, dritten und vierten Polynukleotid hybridisiert.

18. Verfahren nach Anspruch 17, wobei die markierte Oligonukleotidsonde einen nachweisbaren Anteil, ausgewählt aus der Gruppe bestehend aus radioaktiver Markierung und Fluorophor, enthält.

19. Verfahren nach einem der Ansprüche 1 bis 8, ferner umfassend einen Schritt zur Anreicherung von Krebszellen aus der biologischen Probe vor Hybridisierung des/der Oligonukleotidprimer(s).

20. Verfahren nach Anspruch 1 bis 8, wobei der Schritt zu Anreicherung von Krebszellen aus der biologischen Probe durch Methoden, ausgewählt aus der Gruppe bestehend aus Zelleinfang und Zellabreicherung, erzielt wird.

21. Verfahren nach Anspruch 20, wobei der Zelleinfang durch Immunocapture erzielt wird und die Immunocapture die Schritte umfasst, dass
(a) ein Antikörper an der Oberfläche der Krebszellen absorbiert wird; und
(b) der auf den Krebszellen absorbierte Antikörper von dem Rest der biologischen Probe separiert wird.

22. Verfahren nach Anspruch 21, wobei der Antikörper gegen ein Antigen gerichtet ist, das aus der Gruppe bestehend aus CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE und TCRαβ ausgewählt ist.

23. Verfahren nach Anspruch 21, wobei der Antikörper gegen ein Brustkrebsantigen gerichtet ist.

24. Verfahren nach einem der Ansprüche 21 bis 23, wobei der Antikörper ein monoklonaler Antikörper ist.

25. Verfahren nach Anspruch 21, wobei der Antikörper mit einer magnetischen Kugel konjugiert ist.

26. Verfahren nach Anspruch 21, wobei der Antikörper als tetramerer Antikörperkomplex formuliert ist.

27. Verfahren nach Anspruch 21, wobei die Zellabreicherung durch eine Methode erzielt werden kann, umfassend die Schritte, dass
(a) rote und weiße Zellen quervernetzen, und
(b) die quervernetzten roten und weißen Zellen von dem Rest der biologischen Probe fraktioniert werden.

28. Verfahren nach einem der Ansprüche 5 bis 8, wobei der Schritt zur Amplikation durch eine Polynukleotid-Amplikationsmethode erzielt wird, die aus der Gruppe bestehend aus Reverse-Transkriptase-Polymerase-Kettenreaktion (RT-PCR), inverser PCR, RACE, Ligase-Kettenreaktion (LCR), Qbeta-Replicase, isothermer Amplifikation, Strand Displacement Amplification (SDA), rollender Kettenreaktion (RCR), der Reaktion cyclischer Sonden (CPR), transkriptionsbasierter Amplifikationsysteme (TAS), nucleinsäuresequenzbasierter Amplifikation (NASBA) und 3SR ausgewählt ist.

29. Zusammensetzung zum Nachweis von Brustkrebszellen in einer biologischen Probe eines Patienten enthaltend:
(a) ein erstes Oligonukleotid; und
(b) ein zweites Oligonukleotid; wobei,
das erste Oligonukleotid und das zweite Oligonukleotid mindestens 15 Nukleotide lang sind und spezifisch mit einem ersten Polynukleotid oder dem Komplement davon bzw. mit einem zweiten Polynukleotid oder dem Komplement davon hybridisieren;
wobei das erste Polynukleotid aus der Gruppe bestehend aus SEQ ID NO:73, SEQ ID NO:74 und SEQ ID NO:76 ausgewählt ist; und wobei das zweite Polynukleotid das Polynukleotid wie in SEQ ID NO:75 gezeigt enthält.

30. Zusammensetzung nach Anspruch 29 ferner umfassend ein drittes und ein viertes Oligonukleotid, wobei das dritte und vierte Oligonukleotid mit einem dritten Polynukleotid oder dem Komplement davon bzw. einem vierten Polynukleotid oder dem Komplement davon hybridisieren; wobei das dritte Polynukleotid aus einer Gruppe bestehend aus Polynukleotiden wie in SEQ ID NO:5, SEQ ID NO:6 und SEQ ID NO:7 gezeigt ausgewählt ist; und wobei das vierte Polynukleotid aus einer Gruppe bestehend aus Polynukleotiden wie in SEQ ID NO:13, SEQ ID NO:15, SEQ ID NO:17, SEQ ID NO:19, SEQ ID NO:20, SEQ ID NO:21, SEQ ID NO:22, SEQ ID NO:23 und SEQ ID NO:24 gezeigt ausgewählt ist.

31. Zusammensetzung nach Anspruch 30 oder 31, wobei die Oligonukleotide aus einer Gruppe bestehend aus Oligonukleotiden wie in SEQ ID NOs: 33-44, 48-62 und 72 offenbart ausgewählt sind.

32. Zusammensetzung zum Nachweis von Brustkrebszellen in einer biologischen Probe eines Patienten enthaltend:
(a) ein erstes Oligonukleotidpaar; und
(b) ein zweites Oligonukleotidpaar; wobei,
das erste Oligonukleotidpaar und das zweite Oligonukleotidpaar mindestens 10 Nukleotide lang sind und spezifisch mit einem ersten Polynukleotid oder dem Komplement davon bzw. mit einem zweiten Polynukleotid oder dem Komplement davon hybridisieren; wobei das erste Polynukleotid aus der Gruppe bestehend aus SEQ ID NO:73, SEQ ID NO:74 und SEQ ID NO:76 ausgewählt ist; und wobei das zweite Polynukleotid das Polynukleotid wie in SEQ ID NO:75 gezeigt, enthält.

33. Zusammensetzung nach Anspruch 32 ferner umfassend ein drittes Oligonukleotidpaar und ein viertes Oligonukleotidpaar, wobei das dritte Oligonukleotidpaar und das vierte Oligonukleotidpaar mit einem dritten Polynukleotid oder dem Komplement davon bzw. einem vierten Polynukleotid oder dem Komplement davon hybridisieren; wobei das dritte Polynukleotid aus einer Gruppe bestehend aus Polynukleotiden wie in SEQ ID NO:5, SEQ ID NO:6 und SEQ ID NO:7 gezeigt ausgewählt ist; und wobei das vierte Polynukleotid aus einer Gruppe bestehend aus Polynukleotiden wie in SEQ ID NO:13, SEQ ID NO:15, SEQ ID NO:17, SEQ ID NO:19, SEQ ID NO:20, SEQ ID NO:21, SEQ ID NO:22, SEQ ID NO:23 und SEQ ID NO:24 gezeigt ausgewählt ist.

34. Zusammensetzung nach Anspruch 32 oder 33, wobei die Oligonukleotide aus einer Gruppe bestehend aus Oligonukleotiden wie in SEQ ID NOs: 33-44, 48-62 und 72 offenbart ausgewählt sind.

35. Kit zum Nachweis von Brustkrebs, enthaltend die Zusammensetzung nach einem der Ansprüche 29 bis 34.

## Revendications

1. Procédé destiné à détecter la présence d'une cellule du cancer du sein, ledit procédé comprenant les étapes de :
(a) mise en contact d'un échantillon biologique obtenu d'un patient avec un premier oligonucléotide qui s'hybride avec un premier polynucléotide choisi dans le groupe consistant en la mammaglobine et la lipophiline B ;
(b) mise en contact dudit échantillon biologique avec un deuxième oligonucléotide qui s'hybride avec une deuxième séquence polynucléotidique comprenant une séquence polynucléotidique GABAn (B899P) ;
(c) détection dans ledit échantillon biologique d'une quantité d'oligonucléotide hybridé des étapes (a) et (b) ; et
(d) comparaison de la quantité de polynucléotide qui s'hybride avec les oligonucléotides des étapes (a) et (b) avec une valeur seuil prédéterminée, et, à partir de là, détermination de la présence ou de l'absence d'un cancer chez le patient.

2. Procédé selon la revendication 1 dans lequel ledit premier polynucléotide est choisi dans le groupe consistant en les polynucléotides décrits dans les séquences ayant pour identificateurs SEQ ID N° 73, SEQ ID N° 74 et SEQ ID N° 76, ou le complémentaire de ceux-ci ; et dans lequel ledit deuxième polynucléotide comprend le polynucléotide décrit dans la séquence ayant pour identificateur SEQ ID N° 75, ou le complémentaire de celui-ci.

3. Procédé selon la revendication 2 comprenant en outre :
(e) la mise en contact dudit échantillon biologique avec un troisième oligonucléotide qui s'hybride avec un troisième polynucléotide choisi dans le groupe consistant en les polynucléotides décrits dans les séquences ayant pour identificateurs SEQ ID N° 5, SEQ ID N° 6 et SEQ ID N° 7 ; ou le complémentaire de ceux-ci ;
(f) la mise en contact dudit échantillon biologique avec un quatrième oligonucléotide qui s'hybride avec un quatrième polynucléotide choisi dans le groupe consistant en les polynucléotides décrits dans les séquences ayant pour identificateurs SEQ ID N° 13, SEQ ID N° 15, SEQ ID N° 17, SEQ ID N° 19, SEQ ID N° 20, SEQ ID N° 21, SEQ ID N° 22, SEQ ID N° 23 et SEQ ID N° 24 ; ou le complémentaire de ceux-ci et
dans lequel l'étape (c) comprend la détection dans ledit échantillon biologique d'une quantité de l'oligonucléotide hybridé des étapes (a), (b), (e), et (f).

4. Procédé selon la revendication 2 comprenant en outre :
(e) la mise en contact dudit échantillon biologique avec un troisième oligonucléotide qui s'hybride avec un troisième polynucléotide choisi dans le groupe consistant en un polynucléotide décrit dans les séquences ayant pour identificateurs SEQ ID N° 1, SEQ ID N° 3, SEQ ID N° 5, SEQ ID N° 6 et SEQ ID N° 7 ; ou le complémentaire de celui-ci ;
(f) la mise en contact dudit échantillon biologique avec un quatrième oligonucléotide qui s'hybride avec un quatrième polynucléotide choisi dans le groupe consistant en un polynucléotide décrit dans la séquence ayant pour identificateur SEQ ID N° 11, ou le complémentaire de cclui-ci ;
(g) la mise en contact dudit échantillon biologique avec un cinquième oligonucléotide qui s'hybride avec un cinquième polynucléotide choisi dans le groupe consistant en un polynucléotide décrit dans les séquences ayant pour identificateurs SEQ ID N° 13, 15 et 17 ; ou le complémentaire de celui-ci ;
(h) la mise en contact dudit échantillon biologique avec un sixième oligonucléotide qui s'hybride avec un sixième polynucléotide choisi dans le groupe consistant en un polynucléotide décrit dans les séquences ayant pour identificateurs SEQ ID N° 19, SEQ ID N° 20, SEQ ID N° 21, SEQ ID N° 22, SEQ ID N° 23 et SEQ ID N° 24 ; ou le complémentaire de celui-ci ;
(i) la mise en contact dudit échantillon biologique avec un septième oligonucléotide qui s'hybride avec un septième polynucléotide décrit dans la séquence ayant pour identificateur SEQ ID N° 30 ou le complémentaire de celui-ci ;
(j) la mise en contact dudit échantillon biologique avec un huitième oligonucléotide qui s'hybride avec un huitième polynucléotide décrit dans la séquence ayant pour identificateur SEQ ID N° 32 ou le complémentaire de celui-ci ; et
(k) la mise en contact dudit échantillon biologique avec un neuvième oligonucléotide qui s'hybride avec un polynucléotide décrit dans la séquence ayant pour identificateur SEQ ID N° 76 ou le complémentaire de celui-ci ;
dans lequel l'étape (c) comprend la détection dans ledit échantillon biologique d'une quantité d'oligonucléotide hybridé des étapes (a), (b), (e), (f), (g), (h), (i), (j), et (k).

5. Procédé destiné à détecter la présence d'une cellule du cancer du sein, ledit procédé comprenant les étapes suivantes :
(a) la mise en contact d'un échantillon biologique obtenu d'un patient avec une première paire d'oligonucléotides, ladite première paire comprenant un premier oligonucléotide et un deuxième oligonucléotide dans lesquels le premier oligonucléotide et ledit deuxième oligonucléotide s'hybrident avec un premier polynucléotide et le complémentaire de celui-ci, respectivement, dans lesquels ledit premier polynucléotide comprend de la mammaglobine ou de la lipophiline B ;
(b) la mise en contact dudit échantillon biologique avec une deuxième paire d'oligonucléotides, ladite deuxième paire comprenant un troisième oligonucléotide et un quatrième oligonucléotide dans lesquels lesdits troisième et quatrième oligonucléotides s'hybrident avec un deuxième polynucléotide et le complémentaire de celui-ci, respectivement, et dans lesquels ledit deuxième polynucléotide comprend une GABAπ (B899P);
(c) l'amplification dudit premier polynucléotide et dudit deuxième polynucléotide; et
(d) la détection dudit premier polynucléotide amplifié et dudit deuxième polynucléotide amplifié ;
dans lequel la présence dudit premier polynucléotide amplifié ou dudit deuxième polynucléotide amplifié indique la présence d'une cellule du cancer du sein chez ledit patient.

6. Procédé selon la revendication 5 dans lequel ledit premier polynucléotide est choisi dans le groupe consistant en les polynucléotides décrits dans les séquences ayant pour identificateurs SEQ ID N° 73, SEQ ID N° 74 et SEQ ID N° 76 ; et dans lequel ledit deuxième polynucléotide comprend le polynucléotide décrit dans la séquence ayant pour identificateur SEQ ID N° 75.

7. Procédé selon la revendication 6 comprenant en outre la mise en contact dudit échantillon biologique avec une troisième paire d'oligonucléotides, ladite troisième paire comprenant une amorce directe et une amorce inverse qui s'hybrident avec un troisième polynucléotide et le complémentaire de celui-ci, respectivement, dans lequel ledit troisième polynucléotide est choisi dans le groupe consistant en les polynucléotides décrits dans les séquences ayant pour identificateurs SEQ ID N° 5, SEQ ID N° 6 et SEQ ID N° 7; dans lequel l'étape (d) comprend en outre l'amplification dudit troisième polynucléotide ; et dans lequel l'étape (e) comprend en outre la détection dudit troisième polynucléotide.

8. Procédé selon la revendication 7 comprenant en outre la mise en contact dudit échantillon biologique avec une quatrième paire d'oligonucléotides, ladite quatrième paire comprenant une amorce directe et une amorce inverse qui s'hybrident avec un quatrième polynucléotide et le complémentaire de celui-ci, respectivement, dans lequel ledit quatrième polynucléotide est choisi dans le groupe consistant en les polynucléotides décrits dans les séquences ayant pour identificateurs SEQ ID N° 13, SEQ ID N° 15, SEQ ID N° 17, SEQ ID N° 19, SEQ ID N° 20, SEQ ID N° 21, SEQ ID N° 22, SEQ ID N° 23 et SEQ ID N° 24 ; dans lequel l'étape (d) comprend en outre l'amplification dudit quatrième polynucléotide; et dans lequel l'étape (e) comprend en outre la détection dudit quatrième polynucléotide.

9. Procédé selon l'une quelconque des revendications 1 à 8 dans lequel ledit échantillon biologique est choisi parmi le sang, le sérum, un ganglion lymphatique, la moelle osseuse, un crachat, l'urine et un échantillon de biopsie de tumeur.

10. Procédé selon la revendication 9 dans lequel ledit échantillon biologique est choisi parmi le sang, un ganglion lymphatique et la moelle osscuse.

11. Procédé selon la revendication 9 dans lequel ledit ganglion lymphatique est un ganglion lymphatique sentinelle.

12. Procédé selon l'une quelconque des revendications 5 à 8 dans lequel lesdits oligonucléotides sont choisis dans le groupe consistant en oligonucléotides décrits dans les séquences ayant pour identificateurs SEQ ID N° 33 à 38, 48 à 51, et 53 à 62.

13. Procédé selon l'une quelconque des revendications 5 à 8 dans lequel l'étape (e) comprend une étape choisie parmi la détection d'un marqueur radioactif et la détection d'un fluorophore.

14. Procédé selon l'une quelconque des revendications 1 à 8 dans lequel ladite étape de détection comprend une étape de fractionnement.

15. Procédé selon l'une quelconque des revendications 5 à 8 dans lequel lesdits oligonucléotides sont des oligonucléotides à recouvrement d'intron(s).

16. Procédé selon la revendication 15 dans lequel lesdits oligonucléotides à recouvrement d'intron(s) sont choisis dans le groupe consistant en les oligonucléotides décrits dans les séquences ayant pour identificateurs SEQ ID N° 36 à 44 et 48 à 62.

17. Procédé selon l'une quelconque des revendications 5 à 8 dans lequel la détection dudit premier, deuxième, troisième ou quatrième polynucléotide amplifié comprend la mise en contact dudit premier, deuxième, troisième ou quatrième polynucléotide amplifié avec une sonde oligonucléotidique marquée qui s'hybride, dans des conditions modérément stringentes, audit premier, deuxième, troisième ou quatrième polynucléotide.

18. Procédé selon la revendication 17 dans lequel ladite sonde oligonucléotidique marquée comprend un fragment détectable choisi dans le groupe consistant en un marqueur radioactif et un fluorophore.

19. Procédé selon l'une quelconque des revendications 1 à 8 comprenant en outre une étape d'enrichissement de ladite cellule cancéreuse à partir dudit échantillon biologique avant d'hybrider ladite ou lesdites amorce(s) oligonucléotidique(s).

20. Procédé selon revendications 1 à 8 dans lequel ladite étape d'enrichissement de ladite cellule cancéreuse à partir dudit échantillon biologique est réalisé par une méthodologie choisie dans le groupe consistant en la capture de cellules et la déplétion de cellules.

21. Procédé selon la revendication 20 dans lequel la capture de cellules est réalisée par immunocapture, ladite immunocapture comprenant les étapes suivantes :
(a) adsorption d'un anticorps à la surface desdites cellules cancéreuses ; et
(b) séparation desdites cellules cancéreuses adsorbées par anticorps du restant dudit échantillon biologique.

22. Procédé selon la revendication 21 dans lequel ledit anticorps est adressé à un antigène choisi dans le groupe consistant en CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE et TCRαβ.

23. Procédé selon la revendication 21 dans lequel ledit anticorps est adressé à l'antigène du cancer du sein.

24. Procédé selon l'une quelconque des revendications 21 à 23 dans lequel ledit anticorps est un anticorps monoclonal.

25. Procédé selon la revendication 21 dans lequel ledit anticorps est conjugué avec des billes magnétiques.

26. Procédé selon la revendication 21 dans lequel ledit anticorps est formulé dans un complexe d'anticorps tétramériques.

27. Procédé selon la revendication 21 dans lequel la déplétion des cellules est réalisée au moyen d'un procédé comprenant les étapes suivantes :
(a) réticulation des globules rouges et des leucocytes, et
(b) fractionnement desdits globules rouges et leucocytes réticulés à partir du restant dudit échantillon biologique.

28. Procédé selon l'une quelconque des revendications 5 à 8 dans lequel ladite étape d'amplification est réalisée par une méthodologie d'amplification polynucléotidique choisie dans le groupe consistant en la réaction en chaîne de la polymérase transcriptase inverse (RT-PCR), la PCR inverse, la RACE, la réaction en chaîne par ligase (LCR), la Qbêta Réplicase, l'amplification isotherme, l'amplification par déplacement de brin (SDA), la réaction en chaîne par rouleau (RCR), la réaction par sonde cyclique (CPR), les systèmes d'amplification fondée sur la transcription (TAS), l'amplification fondée sur la séquence d'acide nucléique (NASBA) et le 3SR.

29. Composition destinée à détecter une cellule de cancer du sein dans un échantillon biologique d'un patient, ladite composition comprenant :
(a) un premier oligonucléotide ; et
(b) un deuxième oligonucléotide ;
dans laquelle ledit premier oligonucléotide et ledit deuxième oligonucléotide ont une longueur d'au moins 15 nucléotides et s'hybrident spécifiquement avec un premier polynucléotide ou le complémentaire de celui-ci et avec un deuxième polynucléotide ou le complémentaire de celui-ci, respectivement ; dans laquelle ledit premier polynucléotide est choisi dans le groupe consistant en les séquences ayant pour identificateurs SEQ ID N° 73, SEQ ID N° 74 et SEQ ID N° 76; et dans laquelle le dit deuxième polynucléotide comprend le polynucléotide décrit dans la séquence ayant pour identificateur SEQ ID N° 75.

30. Composition selon la revendication 29 comprenant en outre un troisième et un quatrième oligonucléotides dans laquelle lesdits troisième et quatrième oligonucléotides s'hybrident avec un troisième polynucléotide ou le complémentaire de celui-ci et un quatrième polynucléotide ou le complémentaire de celui-ci, respectivement ; dans laquelle ledit troisième polynucléotide est choisi dans le groupe consistant en les polynucléotides décrits dans les séquences ayant pour identificateurs SEQ ID N° 5, SEQ ID N° 6 et SEQ ID N° :7 ; et dans laquelle ledit quatrième polynucléotide est choisi dans le groupe consistant en les polynucléotides décrits dans les séquences ayant pour identificateurs SEQ ID N° 13, SEQ ID N° 15, SEQ ID N° 17, SEQ ID N° 19, SEQ ID N° 20, SEQ ID N° 21, SEQ ID N° 22, SEQ ID N° 23 et SEQ ID N° 24.

31. Composition selon la revendication 30 ou selon la revendication 31 dans laquelle lesdits oligonucléotides sont choisis dans le groupe consistant en les oligonucléotides décrits dans les séquences ayant pour identificateurs SEQ ID N° 33 à 44, 48 à 62, et 72.

32. Composition destinée à détecter la cellule du cancer du sein dans un échantillon biologique d'un patient, ladite composition comprenant :
(a) une première paire d'oligonucléotides ; et
(b) une deuxième paire d'oligonucléotides ;
dans laquelle ladite première paire d'oligonucléotides et ladite deuxième paire d'oligonucléotides ont une longueur d'au moins 10 nucléotides et s'hybrident spécifiquement avec un premier polynucléotide ou le complémentaire de celui-ci et avec un deuxième polynucléotide ou le complémentaire de celui-ci, respectivement ;
dans laquelle ledit premier polynucléotide est choisi dans le groupe consistant en les séquences ayant pour identificateurs SEQ ID N° 73, SEQ ID N° 74 et SEQ ID N° 76 ; et dans laquelle ledit deuxième polynucléotide comprend le polynucléotide décrit dans la séquence ayant pour identificateur SEQ ID N° 75.

33. Composition selon la revendication 32 comprenant en outre une troisième paire d'oligonucléotides et une quatrième paire d'oligonucléotides dans laquelle ladite troisième paire d'oligonucléotides et ladite quatrième paire d'oligonucléotides s'hybrident avec un troisième polynucléotide ou le complémentaire de celui-ci et avec un quatrième polynucléotide ou le complémentaire de celui-ci, respectivement ; dans laquelle le troisième polynucléotide est choisi dans le groupe consistant en les polynucléotides décrits dans les séquences ayant pour identificateurs SEQ ID N° 5, SEQ ID N° 6 et SEQ ID N° 7 ; et dans laquelle le quatrième polynucléotide est choisi dans le groupe consistant en les polynucléotides décrits dans les séquences ayant pour identificateurs SEQ ID N° 13, SEQ ID N° 15, SEQ ID N° 17, SEQ ID N° 19, SEQ ID N° 20, SEQ ID N° 21, SEQ ID N° 22, SEQ ID N° 23 et SEQ ID N° 24.

34. Composition selon la revendication 32 ou selon la revendication 33 dans laquelle lesdits oligonucléotides sont choisis dans le groupe consistant en les oligonucléotides décrits dans les séquences ayant pour identificateurs SEQ ID N° 33 à 44, 48 à 62, et 72.

35. Kit destiné à la détection du cancer du sein, comprenant la composition de l'une quelconque des revendications 29 à 34.


### SEQUENCE LISTING

<110> Corixa Corporation
   Houghton, Raymond L.
   Dillon, Davin C.
   Molesh, David A.
   Xu, Jiangchun
   Zehentner, Barbara
   Persing, David H.
<120> METHODS, COMPOSITIONS AND KITS FOR THE DETECTION AND MONITORING OF BREAST CANCER
<130> 210121.513PC
<140> PCT
   <141> 2001-04-02
<160> 77
<170> FastSEQ for Windows Version 3.0
<210> 1
   <211> 1851
   <212> DNA
   <213> Homo sapien
<400> 1
   tcatcaccat tgccagcagc ggcaccgtta gtcaggtttt ctgggaatcc cacatgagta     60
   cttccgtgtt cttcattctt cttcaatagc cataaatctt ctagctctgg ctggctgttt    120
   tcacttcctt taagcctttg tgactcttcc tctgatgtca gctttaagtc ttgttctgga    180
   ttgctgtttt cagaagagat ttttaacatc tgtttttctt tgtagtcaga aagtaactgg    240
   caaattacat gatgatgact agaaacagca tactctctgg ccgtctttcc agatcttgag    300
   aagatacatc aacattttgc tcaagtagag ggctgactat acttgctgat ccacaacata    360
   cagcaagtat gagagcagtt cttccatatc tatccagcgc atttaaattc gcttttttct   420
   tgattaaaaa tttcaccact tgctgttttt gctcatgtat accaagtagc agtggtgtga    480
   ggccatgctt gttttttgat tcgatatcag caccgtataa gagcagtgct ttggccatta    540
   atttatcttc attgtagaca gcatagtgta gagtggtatt tccatactca tctggaatat    600
   ttggatcagt gccatgttcc agcaacatta acgcacattc atcttcctgg cattgtacgg    660
   cctttgtcag agctgtcctc tttttgttgt caaggacatt aagttgacat cgtctgtcca    720
   gcacgagttt tactacttct gaattcccat tggcagaggc cagatgtaga gcagtcctct    780
   tttgcttgtc cctcttgttc acatccgtgt ccctgagcat gacgatgaga tcctttctgg    840
   ggactttacc ccaccaggca gctctgtgga gcttgtccag atcttctcca tggacgtggt    900
   acctgggatc catgaaggcg ctgtcatcgt agtctcccca agcgaccacg ttgctcttgc    960
   cgctcccctg cagcagggga agcagtggca gcaccacttg cacctcttgc tcccaagcgt   1020
   cttcacagag gagtcgttgt ggtctccaga agtgcccacg ttgctcttgc cgctccccct  1080
   gtccatccag ggaggaagaa atgcaggaaa tgaaagatgc atgcacgatg gtatactcct   1140
   cagccatcaa acttctggac agcaggtcac ttccagcaag gtggagaaag ctgtccaccc   1200
   acagaggatg agatccagaa accacaatat ccattcacaa acaaacactt ttcagccaga   1260
   cacaggtact gaaatcatgt catctgcggc aacatggtgg aacctaccca atcacacatc   1320
   aagagatgaa gacactgcag tatatctgca caacgtaata ctcttcatcc ataacaaaat   1380
   aatataattt tcctctggag ccatatggat gaactatgaa ggaagaactc cccgaagaag   1440
   ccagtcgcag agaagccaca ctgaagctct gtcctcagcc atcagcgcca cggacaggar   1500
   tgtgtttctt ccccagtgat gcagcctcaa gttatcccga agctgccgca gcacacggtg   1560
   gctcctgaga aacaccccag ctcttccggt ctaacacagg caagtcaata aatgtgataa   1620
   tcacataaac agaattaaaa gcaaagtcac ataagcatct caacagacac agaaaaggca   1680
   tttgacaaaa tccagcatcc ttgtatttat tgttgcagtt ctcagaggaa atgcttctaa   1740
   cttttcccca tttagtatta tgttggctgt gggcttgtca taggtggttt ttattacttt   1800
   aaggtatgtc ccttctatgc ctgttttgct gagggtttta attctcgtgc c            1851
<210> 2
   <211> 329
   <212> PRT
   <213> Homo sapien
<400> 2
   Met Asp Ile Val Val Ser Gly Ser His Pro Leu Trp Val Asp Ser Phe
    1               5                  10                  15
   Leu His Leu Ala Gly Ser Asp Leu Leu Ser Arg Ser Leu Met Ala Glu
               20                  25                  30
   Glu Tyr Thr Ile Val His Ala Ser Phe Ile Ser Cys Ile Ser Ser Ser
           35                  40                  45
   Leu Asp Gly Gln Gly Glu Arg Gln Glu Gln Arg Gly His Phe Trp Arg
       50                  55                  60
   Pro Gln Arg Leu Leu Cys Glu Asp Ala Trp Glu Gln Glu Val Gln Val
   65                  70                  75                  80
   Val Leu Pro Leu Leu Pro Leu Leu Gln Gly Ser Gly Lys Ser Asn Val
                   85                  90                  95
   Val Ala Trp Gly Asp Tyr Asp Asp Ser Ala Phe Met Asp Pro Arg Tyr
               100                 105                 110
   His Val His Gly Glu Asp Leu Asp Lys Leu His Arg Ala Ala Trp Trp
           115                 120                 125
   Gly Lys Val Pro Arg Lys Asp Leu Ile Val Met Leu Arg Asp Thr Asp
       130                 135                 140
   Val Asn Lys Arg Asp Lys Gln Lys Arg Thr Ala Leu His Leu Ala Ser
   145                 150                 155                 160
   Ala Asn Gly Asn Ser Glu Val Val Lys Leu Val Leu Asp Arg Arg Cys
                   165                 170                 175
   Gln Leu Asn Val Leu Asp Asn Lys Lys Arg Thr Ala Leu Thr Lys Ala
               180                 185                 190
   Val Gln Cys Gln Glu Asp Glu Cys Ala Leu Met Leu Leu Glu His Gly
           195                 200                 205
   Thr Asp Pro Asn Ile Pro Asp Glu Tyr Gly Asn Thr Thr Leu His Tyr
       210                 215                 220
   Ala Val Tyr Asn Glu Asp Lys Leu Met Ala Lys Ala Leu Leu Leu Tyr
   225                 230                 235                 240
   Gly Ala Asp Ile Glu Ser Lys Asn Lys His Gly Leu Thr Pro Leu Leu
                   245                 250                 255
   Leu Gly Ile His Glu Gln Lys Gln Gln Val Val Lys Phe Leu Ile Lys
               260                 265                 270
   Lys Lys Ala Asn Leu Asn Ala Leu Asp Arg Tyr Gly Arg Thr Ala Leu
           275                 280                 285
   Ile Leu Ala Val Cys Cys Gly Ser Ala Ser Ile Val Ser Pro Leu Leu
       290                 295                 300
   Glu Gln Asn Val Asp Val Ser Ser Gln Asp Leu Glu Arg Arg Pro Glu
   305                 310                 315                 320
   Ser Met Leu Phe Leu Val Ile Ile Met
                   325
<210> 3
   <211> 1852
   <212> DNA
   <213> Homo sapiens
<400> 3
   ggcacgagaa ttaaaaccct cagcaaaaca ggcatagaag ggacatacct taaagtaata 60
   aaaaccacct atgacaagcc cacagccaac ataatactaa atggggaaaa gttagaagca 120
   tttcctctga gaactgcaac aataaataca aggatgctgg attttgtcaa atgccttttc 180
   tgtgtctgtt gagatgctta tgtgactttg cttttaattc tgtttatgtg attatcacat 240
   ttattgactt gcctgtgtta gaccggaaga gctggggtgt ttctcaggag ccaccgtgtg 300
   ctgcggcagc ttcgggataa cttgaggctg catcactggg gaagaaacac aytcctgtcc 360
   gtggcgctga tggctgagga cagagcttca gtgtggcttc tctgcgactg gcttcttcgg 420
   ggagttcttc cttcatagtt catccatatg gctccagagg aaaattatat tattttgtta 480
   tggatgaaga gtattacgtt gtgcagatat actgcagtgt cttcatctct tgatgtgtga 540
   ttgggtaggt tccaccatgt tgccgcagat gacatgattt cagtacctgt gtctggctga 600
   aaagtgtttg tttgtgaatg gatattgtgg tttctggatc tcatcctctg tgggtggaca 660
   gctttctcca ccttgctgga agtgacctgc tgtccagaag tttgatggct gaggagtata 720
   ccatcgtgca tgcatctttc atttcctgca tttcttcctc cctggatgga cagggggagc 780
   ggcaagagca acgtgggcac ttctggagac cacaacgact cctctgtgaa gacgcttggg 840
   agcaagaggt gcaagtggtg ctgccactgc ttcccctgct gcagggggag cggcaagagc 900
   aacgtggtcg cttggggaga ctacgatgac agcgccttca tggatcccag gtaccacgtc 960
   catggagaag atctggacaa gctccacaga gctgcctggt ggggtaaagt ccccagaaag 1020
   gatctcatcg tcatgctcag ggacacggat gtgaacaaga gggacaagca aaagaggact 1080
   gctctacatc tggcctctgc caatgggaat tcagaagtag taaaactcgt gctggacaga 1140
   cgatgtcaac ttaatgtcct tgacaacaaa aagaggacag ctctgacaaa ggccgtacaa 1200
   tgccaggaag atgaatgtgc gttaatgttg ctggaacatg gcactgatcc aaatattcca 1260
   gatgagtatg gaaataccac tctacactat gctgtctaca atgaagataa attaatggcc 1320
   aaagcactgc tcttatacgg tgctgatatc gaatcaaaaa acaagcatgg cctcacacca 1380
   ctgctacttg gtatacatga gcaaaaacag caagtggtga aatttttaat caagaaaaaa 1440
   gcgaatttaa atgcgctgga tagatatgga agaactgctc tcatacttgc tgtatgttgt 1500
   ggatcagcaa gtatagtcag ccctctactt gagcaaaatg ttgatgtatc ttctcaagat 1560
   ctggaaagac ggccagagag tatgctgttt ctagtcatca tcatgtaatt tgccagttac 1620
   tttctgacta caaagaaaaa cagatgttaa aaatctcttc tgaaaacagc aatccagaac 1680
   aagacttaaa gctgacatca gaggaagagt cacaaaggct taaaggaagt gaaaacagcc 1740
   agccagagct agaagattta tggctattga agaagaatga agaacacgga agtactcatg 1800
   tgggattccc agaaaacctg actaacggtg ccgctgctgg caatggtgat ga         1852
<210> 4
   <211> 292
   <212> PRT
   <213> Homo sapiens
<400> 4
   Met His Leu Ser Phe Pro Ala Phe Leu Pro Pro Trp Met Asp Arg Gly
                   5                   10                  15
   Ser Gly Lys Ser Asn Val Gly Thr Ser Gly Asp His Asn Asp Ser Ser
               20                  25                  30
   Val Lys Thr Leu Gly Ser Lys Arg Cys Lys Trp Cys Cys His Cys Phe
           35                  40                  45
   Pro Cys Cys Arg Gly Ser Gly Lys Ser Asn Val Val Ala Trp Gly Asp
       50                  55                  60
   Tyr Asp Asp Ser Ala Phe Met Asp Pro Arg Tyr His Val His Gly Glu
   65                  70                  75                  80
   Asp Leu Asp Lys Leu His Arg Ala Ala Trp Trp Gly Lys Val Pro Arg
                   85                  90                  95
   Lys Asp Leu Ile Val Met Leu Arg Asp Thr Asp Val Asn Lys Arg Asp
   100 105 110
   Lys Gln Lys Arg Thr Ala Leu His Leu Ala Ser Ala Asn Gly Asn Ser
   115 120 125
   Glu Val Val Lys Leu Val Leu Asp Arg Arg Cys Gln Leu Asn Val Leu
   130 135 140
   Asp Asn Lys Lys Arg Thr Ala Leu Thr Lys Ala Val Gln Cys Gln Glu
   145 150 155 160
   Asp Glu Cys Ala Leu Met Leu Leu Glu His Gly Thr Asp Pro Asn Ile
   165 170 175
   Pro Asp Glu Tyr Gly Asn Thr Thr Leu His Tyr Ala Val Tyr Asn Glu
   180 185 190
   Asp Lys Leu Met Ala Lys Ala Leu Leu Leu Tyr Gly Ala Asp Ile Glu
   195 200 205
   Ser Lys Asn Lys His Gly Leu Thr Pro Leu Leu Leu Gly Ile His Glu
   210 215 220
   Gln Lys Gln Gln Val Val Lys Phe Leu Ile Lys Lys Lys Ala Asn Leu
   225 230 235 240
   Asn Ala Leu Asp Arg Tyr Gly Arg Thr Ala Leu Ile Leu Ala Val Cys
   245 250 255
   Cys Gly Ser Ala Ser Ile Val Ser Pro Leu Leu Glu Gln Asn Val Asp
   260 265 270
   Val Ser Ser Gln Asp Leu Glu Arg Arg Pro Glu Ser Met Leu Phe Leu
   275 280 285
   Val Ile Ile Met
   290
<210> 5
   <211> 1155
   <212> DNA
   <213> Homo sapien
<400> 5
   atggtggttg aggttgattc catgccggct gcctcttctg tgaagaagcc atttggtctc      60
   aggagcaaga tgggcaagtg gtgctgccgt tgcttcccct gctgcaggga gagcggcaag     120
   agcaacgtgg gcacttctgg agaccacgac gactctgcta tgaagacact caggagcaag     180
   atgggcaagt ggtgccgcca ctgcttcccc tgctgcaggg ggagtggcaa gagcaacgtg     240
   ggcgcttctg gagaccacga cgactctgct atgaagacac tcaggaacaa gatgggcaag     300
   tggtgctgcc actgcttccc ctgctgcagg gggagcggca agagcaaggt gggcgcttgg     360
   ggagactacg atgacagtgc cttcatggag cccaggtacc acgtccgtgg agaagatctg     420
   gacaagctcc acagagctgc ctggtggggt aaagtcccca gaaaggatct catcgtcatg     480
   ctcagggaca ctgacgtgaa caagaaggac aagcaaaaga ggactgctct acatctggcc     540
   tctgccaatg ggaattcaga agtagtaaaa ctcctgctgg acagacgatg tcaacttaat     600
   gtccttgaca acaaaaagag gacagctctg ataaaggccg tacaatgcca ggaagatgaa     660
   tgtgcgttaa tgttgctgga acatggcact gatccaaata ttccagatga gtatggaaat     720
   accactctgc actacgctat ctataatgaa gataaattaa tggccaaagc actgctctta     780
   tatggtgctg atatcgaatc aaaaaacaag catggcctca caccactgtt acttggtgta      840
   catgagcaaa aacagcaagt cgtgaaattt ttaatcaaga aaaaagcgaa tttaaatgca      900
   ctggatagat atggaaggac tgctctcata cttgctgtat gttgtggatc agcaagtata      960
   gtcagccttc tacttgagca aaatattgat gtatcttctc aagatctatc tggacagacg     1020
   gccagagagt atgctgtttc tagtcatcat catgtaattt gccagttact ttctgactac     1080
   aaagaaaaac agatgctaaa aatctcttct gaaaacagca atccagaaaa tgtctcaaga     1140
   accagaaata aataa                                                      1155
<210> 6
   <211> 2000
   <212> DNA
   <213> Homo sapien
<400> 6
   atggtggttg aggttgattc catgccggct gcctcttctg tgaagaagcc atttggtctc       60
   aggagcaaga tgggcaagtg gtgctgccgt tgcttcccct gctgcaggga gagcggcaag      120
   agcaacgtgg gcacttctgg agaccacgac gactctgcta tgaagacact caggagcaag      180
   atgggcaagt ggtgccgcca ctgcttcccc tgctgcaggg ggagtggcaa gagcaacgtg      240
   ggcgcttctg gagaccacga cgactctgct atgaagacac tcaggaacaa gatgggcaag      300
   tggtgctgcc actgcttccc ctgctgcagg gggagcggca agagcaaggt gggcgcttgg      360
   ggagactacg atgacagtgc cttcatggag cccaggtacc acgtccgtgg agaagatctg      420
   gacaagctcc acagagctgc ctggtggggt aaagtcccca gaaaggatct catcgtcatg      480
   ctcagggaca ctgacgtgaa caagaaggac aagcaaaaga ggactgctct acatctggcc      540
   tctgccaatg ggaattcaga agtagtaaaa ctcctgctgg acagacgatg tcaacttaat      600
   gtccttgaca acaaaaagag gacagctctg ataaaggccg tacaatgcca ggaagatgaa      660
   tgtgcgttaa tgttgctgga acatggcact gatccaaata ttccagatga gtatggaaat      720
   accactctgc actacgctat ctataatgaa gataaattaa tggccaaagc actgctctta      780
   tatggtgctg atatcgaatc aaaaaacaag catggcctca caccactgtt acttggtgta      840
   catgagcaaa aacagcaagt cgtgaaattt ttaatcaaga aaaaagcgaa tttaaatgca      900
   ctggatagat atggaaggac tgctctcata cttgctgtat gttgtggatc agcaagtata      960
   gtcagccttc tacttgagca aaatattgat gtatcttctc aagatctatc tggacagacg     1020
   gccagagagt atgctgtttc tagtcatcat catgtaattt gccagttact ttctgactac     1080
   aaagaaaaac agatgctaaa aatctcttct gaaaacagca atccagaaca agacttaaag     1140
   ctgacatcag aggaagagtc acaaaggttc aaaggcagtg aaaatagcca gccagagaaa     1200
   atgtctcaag aaccagaaat aaataaggat ggtgatagag aggttgaaga agaaatgaag     1260
   aagcatgaaa gtaataatgt gggattacta gaaaacctga ctaatggtgt cactgctggc     1320
   aatggtgata atggattaat tcctcaaagg aagagcagaa cacctgaaaa tcagcaattt     1380
   cctgacaacg aaagtgaaga gtatcacaga atttgcgaat tagtttctga ctacaaagaa     1440
   aaacagatgc caaaatactc ttctgaaaac agcaacccag aacaagactt aaagctgaca     1500
   tcagaggaag agtcacaaag gcttgagggc agtgaaaatg gccagccaga gctagaaaat     1560
   tttatggcta tcgaagaaat gaagaagcac ggaagtactc atgtcggatt cccagaaaac     1620
   ctgactaatg gtgccactgc tggcaatggt gatgatggat taattcctcc aaggaagagc     1680
   agaacacctg aaagccagca atttcctgac actgagaatg aagagtatca cagtgacgaa     1740
   caaaatgata ctcagaagca attttgtgaa gaacagaaca ctggaatatt acacgatgag     1800
   attctgattc atgaagaaaa gcagatagaa gtggttgaaa aaatgaattc tgagctttct     1860
   cttagttgta agaaagaaaa agacatcttg catgaaaata gtacgttgcg ggaagaaatt     1920
   gccatgctaa gactggagct agacacaatg aaacatcaga gccagctaaa aaaaaaaaaa     1980
   aaaaaaaaaa aaaaaaaaaa                                                 2000
<210> 7
   <211> 2040
   <212> DNA
   <213> Homo sapien
<400> 7
   atggtggttg aggttgattc catgccggct gcctcttctg tgaagaagcc atttggtctc      60
   aggagcaaga tgggcaagtg gtgctgccgt tgcttcccct gctgcaggga gagcggcaag     120
   agcaacgtgg gcacttctgg agaccacgac gactctgcta tgaagacact caggagcaag     180
   atgggcaagt ggtgccgcca ctgcttcccc tgctgcaggg ggagtggcaa gagcaacgtg     240
   ggcgcttctg gagaccacga cgactctgct atgaagacac tcaggaacaa gatgggcaag     300
   tggtgctgcc actgcttccc ctgctgcagg gggagcggca agagcaaggt gggcgcttgg     360
   ggagactacg atgacagtgc cttcatggag cccaggtacc acgtccgtgg agaagatctg     420
   gacaagctcc acagagctgc ctggtggggt aaagtcccca gaaaggatct catcgtcatg     480
   ctcagggaca ctgacgtgaa caagaaggac aagcaaaaga ggactgctct acatctggcc     540
   tctgccaatg ggaattcaga agtagtaaaa ctcctgctgg acagacgatg tcaacttaat     600
   gtccttgaca acaaaaagag gacagctctg ataaaggccg tacaatgcca ggaagatgaa     660
   tgtgcgttaa tgttgctgga acatggcact gatccaaata ttccagatga gtatggaaat     720
   accactctgc actacgctat ctataatgaa gataaattaa tggccaaagc actgctctta     780
   tatggtgctg atatcgaatc aaaaaacaag catggcctca caccactgtt acttggtgta     840
   catgagcaaa aacagcaagt cgtgaaattt ttaatcaaga aaaaagcgaa tttaaatgca     900
   ctggatagat atggaaggac tgctctcata cttgctgtat gttgtggatc agcaagtata     960
   gtcagccttc tacttgagca aaatattgat gtatcttctc aagatctatc tggacagacg    1020
   gccagagagt atgctgtttc tagtcatcat catgtaattt gccagttact ttctgactac    1080
   aaagaaaaac agatgctaaa aatctcttct gaaaacagca atccagaaca agacttaaag    1140
   ctgacatcag aggaagagtc acaaaggttc aaaggcagtg aaaatagcca gccagagaaa    1200
   atgtctcaag aaccagaaat aaataaggat ggtgatagag aggttgaaga agaaatgaag    1260
   aagcatgaaa gtaataatgt gggattacta gaaaacctga ctaatggtgt cactgctggc    1320
   aatggtgata atggattaat tcctcaaagg aagagcagaa cacctgaaaa tcagcaattt    1380
   cctgacaacg aaagtgaaga gtatcacaga atttgcgaat tagtttctga ctacaaagaa    1440
   aaacagatgc caaaatactc ttctgaaaac agcaacccag aacaagactt aaagctgaca    1500
   tcagaggaag agtcacaaag gcttgagggc agtgaaaatg gccagccaga gaaaagatct    1560
   caagaaccag aaataaataa ggatggtgat agagagctag aaaattttat ggctatcgaa    1620
   gaaatgaaga agcacggaag tactcatgtc ggattcccag aaaacctgac taatggtgcc    1680
   actgctggca atggtgatga tggattaatt cctccaagga agagcagaac acctgaaagc    1740
   cagcaatttc ctgacactga gaatgaagag tatcacagtg acgaacaaaa tgatactcag    1800
   aagcaatttt gtgaagaaca gaacactgga atattacacg atgagattct gattcatgaa    1860
   gaaaagcaga tagaagtggt tgaaaaaatg aattctgagc tttctcttag ttgtaagaaa    1920
   gaaaaagaca tcttgcatga aaatagtacg ttgcgggaag aaattgccat gctaagactg    1980
   gagctagaca caatgaaaca tcagagccag ctaaaaaaaa aaaaaaaaaa aaaaaaaaaa    2040
<210> 8
   <211> 384
   <212> PRT
   <213> Homo sapien
<400> 8
   Met Val Val Glu Val Asp Ser Met Pro Ala Ala Ser Ser Val Lys Lys
   1               5                   10                  15
   Pro Phe Gly Leu Arg Ser Lys Met Gly Lys Trp Cys Cys Arg Cys Phe
               20                  25                  30
   Pro Cys Cys Arg Glu Ser Gly Lys Ser Asn Val Gly Thr Ser Gly Asp
           35                  40                  45
   His Asp Asp Ser Ala Met Lys Thr Leu Arg Ser Lys Met Gly Lys Trp
       50                  55                  60
   Cys Arg His Cys Phe Pro Cys Cys Arg Gly Ser Gly Lys Ser Asn Val
   65                  70                  75                  80
   Gly Ala Ser Gly Asp His Asp Asp Ser Ala Met Lys Thr Leu Arg Asn
                   85                  90                  95
   Lys Met Gly Lys Trp Cys Cys His Cys Phe Pro Cys Cys Arg Gly Ser
               100                 105                 110
   Gly Lys Ser Lys Val Gly Ala Trp Gly Asp Tyr Asp Asp Ser Ala Phe
           115                 120                 125
   Met Glu Pro Arg Tyr His Val Arg Gly Glu Asp Leu Asp Lys Leu His
       130                 135                 140
   Arg Ala Ala Trp Trp Gly Lys Val Pro Arg Lys Asp Leu Ile Val Met
   145                 150                 155                 160
   Leu Arg Asp Thr Asp Val Asn Lys Lys Asp Lys Gln Lys Arg Thr Ala
                   165                 170                 175
   Leu His Leu Ala Ser Ala Asn Gly Asn Ser Glu Val Val Lys Leu Leu
               180                 185                 190
   Leu Asp Arg Arg Cys Gln Leu Asn Val Leu Asp Asn Lys Lys Arg Thr
           195                 200                 205
   Ala Leu Ile Lys Ala Val Gln Cys Gln Glu Asp Glu Cys Ala Leu Met
       210                 215                 220
   Leu Leu Glu His Gly Thr Asp Pro Asn Ile Pro Asp Glu Tyr Gly Asn
   225                 230                 235                 240
   Thr Thr Leu His Tyr Ala Ile Tyr Asn Glu Asp Lys Leu Met Ala Lys
                   245                 250                 255
   Ala Leu Leu Leu Tyr Gly Ala Asp Ile Glu Ser Lys Asn Lys His Gly
               260                 265                 270
   Leu Thr Pro Leu Leu Leu Gly Val His Glu Gln Lys Gln Gln Val Val
           275                 280                 285
   Lys Phe Leu Ile Lys Lys Lys Ala Asn Leu Asn Ala Leu Asp Arg Tyr
       290                 295                 300
   Gly Arg Thr Ala Leu Ile Leu Ala Val Cys Cys Gly Ser Ala Ser Ile
   305                 310                 315                 320
   Val Ser Leu Leu Leu Glu Gln Asn Ile Asp Val Ser Ser Gln Asp Leu
                   325                 330                 335
   Ser Gly Gln Thr Ala Arg Glu Tyr Ala Val Ser Ser His His His Val
               340                 345                 350
   Ile Cys Gln Leu Leu Ser Asp Tyr Lys Glu Lys Gln Met Leu Lys Ile
           355                 360                 365
   Ser Ser Glu Asn Ser Asn Pro Glu Asn Val Ser Arg Thr Arg Asn Lys
       370                 375                 380
<210> 9
   <211> 656
   <212> PRT
   <213> Homo sapien
<400> 9
   Met Val Val Glu Val Asp Ser Met Pro Ala Ala Ser Ser Val Lys Lys
    1               5                  10                  15
   Pro Phe Gly Leu Arg Ser Lys Met Gly Lys Trp Cys Cys Arg Cys Phe
               20                  25                  30
   Pro Cys Cys Arg Glu Ser Gly Lys Ser Asn Val Gly Thr Ser Gly Asp
           35                  40                  45
   His Asp Asp Ser Ala Met Lys Thr Leu Arg Ser Lys Met Gly Lys Trp
       50                  55                  60
   Cys Arg His Cys Phe Pro Cys Cys Arg Gly Ser Gly Lys Ser Asn Val
   65                  70                  75                  80
   Gly Ala Ser Gly Asp His Asp Asp Ser Ala Met Lys Thr Leu Arg Asn
                   85                  90                  95
   Lys Met Gly Lys Trp Cys Cys His Cys Phe Pro Cys Cys Arg Gly Ser
               100                 105                 110
   Gly Lys Ser Lys Val Gly Ala Trp Gly Asp Tyr Asp Asp Ser Ala Phe
           115                 120                 125
   Met Glu Pro Arg Tyr His Val Arg Gly Glu Asp Leu Asp Lys Leu His
       130                 135                 140
   Arg Ala Ala Trp Trp Gly Lys Val Pro Arg Lys Asp Leu Ile Val Met
   145                 150                 155                 160
   Leu Arg Asp Thr Asp Val Asn Lys Lys Asp Lys Gln Lys Arg Thr Ala
                   165                 170                 175
   Leu His Leu Ala Ser Ala Asn Gly Asn Ser Glu Val Val Lys Leu Leu
                   180                      185                      190
   Leu Asp Arg Arg Cys Gln Leu Asn Val Leu Asp Asn Lys Lys Arg Thr
               195                      200                      205
   Ala Leu Ile Lys Ala Val Gln Cys Gln Glu Asp Glu Cys Ala Leu Met
       210                      215                      220
   Leu Leu Glu His Gly Thr Asp Pro Asn Ile Pro Asp Glu Tyr Gly Asn
   225                      230                      235                      240
   Thr Thr Leu His Tyr Ala Ile Tyr Asn Glu Asp Lys Leu Met Ala Lys
                       245                      250                      255
   Ala Leu Leu Leu Tyr Gly Ala Asp Ile Glu Ser Lys Asn Lys His Gly
                   260                      265                      270
   Leu Thr Pro Leu Leu Leu Gly Val His Glu Gln Lys Gln Gln Val Val
               275                      280                      285
   Lys Phe Leu Ile Lys Lys Lys Ala Asn Leu Asn Ala Leu Asp Arg Tyr
       290                      295                      300
   Gly Arg Thr Ala Leu Ile Leu Ala Val Cys Cys Gly Ser Ala Ser Ile
   305                      310                      315                      320
   Val Ser Leu Leu Leu Glu Gln Asn Ile Asp Val Ser Ser Gln Asp Leu
                       325                      330                      335
   Ser Gly Gln Thr Ala Arg Glu Tyr Ala Val Ser Ser His His His Val
                   340                      345                      350
   Ile Cys Gln Leu Leu Ser Asp Tyr Lys Glu Lys Gln Met Leu Lys Ile
               355                      360                      365
   Ser Ser Glu Asn Ser Asn Pro Glu Gln Asp Leu Lys Leu Thr Ser Glu
       370                      375                      380
   Glu Glu Ser Gln Arg Phe Lys Gly Ser Glu Asn Ser Gln Pro Glu Lys
   385                      390                      395                      400
   Met Ser Gln Glu Pro Glu Ile Asn Lys Asp Gly Asp Arg Glu Val Glu
                       405                      410                      415
   Glu Glu Met Lys Lys His Glu Ser Asn Asn Val Gly Leu Leu Glu Asn
                   420                      425                      430
   Leu Thr Asn Gly Val Thr Ala Gly Asn Gly Asp Asn Gly Leu Ile Pro
               435                      440                      445
   Gln Arg Lys Ser Arg Thr Pro Glu Asn Gln Gln Phe Pro Asp Asn Glu
       450                      455                      460
   Ser Glu Glu Tyr His Arg Ile Cys Glu Leu Val Ser Asp Tyr Lys Glu
   465                      470                      475                      480
   Lys Gln Met Pro Lys Tyr Ser Ser Glu Asn Ser Asn Pro Glu Gln Asp
                       485                      490                      495
   Leu Lys Leu Thr Ser Glu Glu Glu Ser Gln Arg Leu Glu Gly Ser Glu
                   500                      505                      510
   Asn Gly Gln Pro Glu Leu Glu Asn Phe Met Ala Ile Glu Glu Met Lys
               515                      520                      525
   Lys His Gly Ser Thr His Val Gly Phe Pro Glu Asn Leu Thr Asn Gly
       530                      535                      540
   Ala Thr Ala Gly Asn Gly Asp Asp Gly Leu Ile Pro Pro Arg Lys Ser
   545                      550                      555                      560
   Arg Thr Pro Glu Ser Gln Gln Phe Pro Asp Thr Glu Asn Glu Glu Tyr
                       565                      570                      575
   His Ser Asp Glu Gln Asn Asp Thr Gln Lys Gln Phe Cys Glu Glu Gln
                   580                      585                      590
   Asn Thr Gly Ile Leu His Asp Glu Ile Leu Ile His Glu Glu Lys Gln
               595                      600                      605
   Ile Glu Val Val Glu Lys Met Asn Ser Glu Leu Ser Leu Ser Cys Lys
       610                      615                      620
   Lys Glu Lys Asp Ile Leu His Glu Asn Ser Thr Leu Arg Glu Glu Ile
   625                      630                      635                      640
   Ala Met Leu Arg Leu Glu Leu Asp Thr Met Lys His Gln Ser Gln Leu
   645 650 655
<210> 10
   <211> 671
   <212> PRT
   <213> Homo sapien
<400> 10
   Met Val Val Glu Val Asp Ser Met Pro Ala Ala Ser Ser Val Lys Lys
    1               5                  10                  15
   Pro Phe Gly Leu Arg Ser Lys Met Gly Lys Trp Cys Cys Arg Cys Phe
               20                  25                  30
   Pro Cys Cys Arg Glu Ser Gly Lys Ser Asn Val Gly Thr Ser Gly Asp
           35                  40                  45
   His Asp Asp Ser Ala Met Lys Thr Leu Arg Ser Lys Met Gly Lys Trp
       50                  55                  60
   Cys Arg His Cys Phe Pro Cys Cys Arg Gly Ser Gly Lys Ser Asn Val
   65                  70                  75                  80
   Gly Ala Ser Gly Asp His Asp Asp Ser Ala Met Lys Thr Leu Arg Asn
                   85                  90                  95
   Lys Met Gly Lys Trp Cys Cys His Cys Phe Pro Cys Cys Arg Gly Ser
               100                 105                 110
   Gly Lys Ser Lys Val Gly Ala Trp Gly Asp Tyr Asp Asp Ser Ala Phe
           115                 120                 125
   Met Glu Pro Arg Tyr His Val Arg Gly Glu Asp Leu Asp Lys Leu His
       130                 135                 140
   Arg Ala Ala Trp Trp Gly Lys Val Pro Arg Lys Asp Leu Ile Val Met
   145                 150                 155                 160
   Leu Arg Asp Thr Asp Val Asn Lys Lys Asp Lys Gln Lys Arg Thr Ala
                   165                 170                 175
   Leu His Leu Ala Ser Ala Asn Gly Asn Ser Glu Val Val Lys Leu Leu
               180                 185                 190
   Leu Asp Arg Arg Cys Gln Leu Asn Val Leu Asp Asn Lys Lys Arg Thr
           195                 200                 205
   Ala Leu Ile Lys Ala Val Gln Cys Gln Glu Asp Glu Cys Ala Leu Met
       210                 215                 220
   Leu Leu Glu His Gly Thr Asp Pro Asn Ile Pro Asp Glu Tyr Gly Asn
   225                 230                 235                 240
   Thr Thr Leu His Tyr Ala Ile Tyr Asn Glu Asp Lys Leu Met Ala Lys
                   245                 250                 255
   Ala Leu Leu Leu Tyr Gly Ala Asp Ile Glu Ser Lys Asn Lys His Gly
               260                 265                 270
   Leu Thr Pro Leu Leu Leu Gly Val His Glu Gln Lys Gln Gln Val Val
           275                 280                 285
   Lys Phe Leu Ile Lys Lys Lys Ala Asn Leu Asn Ala Leu Asp Arg Tyr
       290                 295                 300
   Gly Arg Thr Ala Leu Ile Leu Ala Val Cys Cys Gly Ser Ala Ser Ile
   305                 310                 315                 320
   Val Ser Leu Leu Leu Glu Gln Asn Ile Asp Val Ser Ser Gln Asp Leu
                   325                 330                 335
   Ser Gly Gln Thr Ala Arg Glu Tyr Ala Val Ser Ser His His His Val
               340                 345                 350
   Ile Cys Gln Leu Leu Ser Asp Tyr Lys Glu Lys Gln Met Leu Lys Ile
           355                 360                 365
   Ser Ser Glu Asn Ser Asn Pro Glu Gln Asp Leu Lys Leu Thr Ser Glu
       370                 375                 380
   Glu Glu Ser Gln Arg Phe Lys Gly Ser Glu Asn Ser Gln Pro Glu Lys
   385                 390                 395                 400
   Met Ser Gln Glu Pro Glu Ile Asn Lys Asp Gly Asp Arg Glu Val Glu
                   405                 410                 415
   Glu Glu Met Lys Lys His Glu Ser Asn Asn Val Gly Leu Leu Glu Asn
               420                 425                 430
   Leu Thr Asn Gly Val Thr Ala Gly Asn Gly Asp Asn Gly Leu Ile Pro
           435                 440                 445
   Gln Arg Lys Ser Arg Thr Pro Glu Asn Gln Gln Phe Pro Asp Asn Glu
       450                 455                 460
   Ser Glu Glu Tyr His Arg Ile Cys Glu Leu Val Ser Asp Tyr Lys Glu
   465                 470                 475                 480
   Lys Gln Met Pro Lys Tyr Ser Ser Glu Asn Ser Asn Pro Glu Gln Asp
                   485                 490                 495
   Leu Lys Leu Thr Ser Glu Glu Glu Ser Gln Arg Leu Glu Gly Ser Glu
               500                 505                 510
   Asn Gly Gln Pro Glu Lys Arg Ser Gln Glu Pro Glu Ile Asn Lys Asp
           515                 520                 525
   Gly Asp Arg Glu Leu Glu Asn Phe Met Ala Ile Glu Glu Met Lys Lys
       530                 535                 540
   His Gly Ser Thr His Val Gly Phe Pro Glu Asn Leu Thr Asn Gly Ala
   545                 550                 555                 560
   Thr Ala Gly Asn Gly Asp Asp Gly Leu Ile Pro Pro Arg Lys Ser Arg
                   565                 570                 575
   Thr Pro Glu Ser Gln Gln Phe Pro Asp Thr Glu Asn Glu Glu Tyr His
               580                 585                 590
   Ser Asp Glu Gln Asn Asp Thr Gln Lys Gln Phe Cys Glu Glu Gln Asn
           595                 600                 605
   Thr Gly Ile Leu His Asp Glu Ile Leu Ile His Glu Glu Lys Gln Ile
       610                 615                 620
   Glu Val Val Glu Lys Met Asn Ser Glu Leu Ser Leu Ser Cys Lys Lys
   625                 630                 635                 640
   Glu Lys Asp Ile Leu His Glu Asn Ser Thr Leu Arg Glu Glu Ile Ala
                   645                 650                 655
   Met Leu Arg Leu Glu Leu Asp Thr Met Lys His Gln Ser Gln Leu
               660                 665                 670
<210> 11
   <211> 800
   <212> DNA
   <213> Homo sapien
<400> 11
   atkagcttcc gcttctgaca acactagaga tccctcccct ccctcagggt atggccctcc     60
   acttcatttt tggtacataa catctttata ggacaggggt aaaatcccaa tactaacagg    120
   agaatgctta ggactctaac aggtttttga gaatgtgttg gtaagggcca ctcaatccaa    180
   tttttcttgg tcctccttgt ggtctaggag gacaggcaag ggtgcagatt ttcaagaatg    240
   catcagtaag ggccactaaa tccgaccttc ctcgttcctc cttgtggtct gggaggaaaa    300
   ctagtgtttc tgttgctgtg tcagtgagca caactattcc gatcagcagg gtccagggac    360
   cactgcaggt tcttgggcag ggggagaaac aaaacaaacc aaaaccatgg gcrgttttgt     420
   ctttcagatg ggaaacactc aggcatcaac aggctcacct ttgaaatgca tcctaagcca    480
   atgggacaaa tttgacccac aaaccctgga aaaagaggtg gctcattttt tttgcactat    540
   ggcttggccc caacattctc tctctgatgg ggaaaaatgg ccacctgagg gaagtacaga    600
   ttacaatact atcctgcagc ttgacctttt ctgtaagagg gaaggcaaat ggagtgaaat    660
   accttatgtc caagctttct tttcattgaa ggagaataca ctatgcaaag cttgaaattt    720
   acatcccaca ggaggacctc tcagcttacc cccatatcct agcctcccta tagctcccct    780
   tcctattagt gataagcctc                                                800
<210> 12
   <211> 102
   <212> PRT
   <213> Homo sapien
<220>
   <221> VARIANT
   <222> (1)...(102)
   <223> Xaa = Any Amino Acid
<400> 12
   Met Gly Xaa Phe Val Phe Gln Met Gly Asn Thr Gln Ala Ser Thr Gly
    1               5                  10                  15
   Ser Pro Leu Lys Cys Ile Leu Ser Gln Trp Asp Lys Phe Asp Pro Gln
               20                  25                  30
   Thr Leu Glu Lys Glu Val Ala His Phe Phe Cys Thr Met Ala Trp Pro
           35                  40                  45
   Gln His Ser Leu Ser Asp Gly Glu Lys Trp Pro Pro Glu Gly Ser Thr
       50                  55                  60
   Asp Tyr Asn Thr Ile Leu Gln Leu Asp Leu Phe Cys Lys Arg Glu Gly
   65                  70                  75                  80
   Lys Trp Ser Glu Ile Pro Tyr Val Gln Ala Phe Phe Ser Leu Lys Glu
                   85                  90                  95
   Asn Thr Leu Cys Lys Ala
               100
<210> 13
   <211> 1206
   <212> DNA
   <213> Homo sapien
<400> 13
   ggcacgagga agttttgtgt actgaaaaag aaactgtcag aagcaaaaga aataaaatca      60
   cagttagaga accaaaaagt taaatgggaa caagagctct gcagtgtgag gtttctcaca     120
   ctcatgaaaa tgaaaattat ctcttacatg aaaattgcat gttgaaaaag gaaattgcca     180
   tgctaaaact ggaaatagcc acactgaaac accaatacca ggaaaaggaa aataaatact     240
   ttgaggacat taagatttta aaagaaaaga atgctgaact tcagatgacc ctaaaactga     300
   aagaggaatc attaactaaa agggcatctc aatatagtgg gcagcttaaa gttctgatag     360
   ctgagaacac aatgctcact tctaaattga aggaaaaaca agacaaagaa atactagagg     420
   cagaaattga atcacaccat cctagactgg cttctgctgt acaagaccat gatcaaattg     480
   tgacatcaag aaaaagtcaa gaacctgctt tccacattgc aggagatgct tgtttgcaaa     540
   gaaaaatgaa tgttgatgtg agtagtacga tatataacaa tgaggtgctc catcaaccac     600
   tttctgaagc tcaaaggaaa tccaaaagcc taaaaattaa tctcaattat gccggagatg     660
   ctctaagaga aaatacattg gtttcagaac atgcacaaag agaccaacgt gaaacacagt     720
   gtcaaatgaa ggaagctgaa cacatgtatc aaaacgaaca agataatgtg aacaaacaca     780
   ctgaacagca ggagtctcta gatcagaaat tatttcaact acaaagcaaa aatatgtggc     840
   ttcaacagca attagttcat gcacataaga aagctgacaa caaaagcaag ataacaattg     900
   atattcattt tcttgagagg aaaatgcaac atcatctcct aaaagagaaa aatgaggaga     960
   tatttaatta caataaccat ttaaaaaacc gtatatatca atatgaaaaa gagaaagcag    1020
   aaacagaagt tatataatag tataacactg ccaaggagcg gattatctca tcttcatcct    1080
   gtaattccag tgtttgtcac gtggttgttg aataaatgaa taaagaatga gaaaaccaga    1140
   agctctgata cataatcata atgataatta tttcaatgca caactacggg tggtgctgct    1200
   cgtgcc                                                               1206
<210> 14
   <211> 317
   <212> PRT
   <213> Homo sapien
<400> 14
   Met Gly Thr Arg Ala Leu Gln Cys Glu Val Ser His Thr His Glu Asn
   1               5                   10                  15
   Glu Asn Tyr Leu Leu His Glu Asn Cys Met Leu Lys Lys Glu Ile Ala
               20                  25                  30
   Met Leu Lys Leu Glu Ile Ala Thr Leu Lys His Gln Tyr Gln Glu Lys
           35                  40                  45
   Glu Asn Lys Tyr Phe Glu Asp Ile Lys Ile Leu Lys Glu Lys Asn Ala
       50                  55                  60
   Glu Leu Gln Met Thr Leu Lys Leu Lys Glu Glu Ser Leu Thr Lys Arg
   65                  70                  75                  80
   Ala Ser Gln Tyr Ser Gly Gln Leu Lys Val Leu Ile Ala Glu Asn Thr
                   85                  90                  95
   Met Leu Thr Ser Lys Leu Lys Glu Lys Gln Asp Lys Glu Ile Leu Glu
               100                 105                 110
   Ala Glu Ile Glu Ser His His Pro Arg Leu Ala Ser Ala Val Gln Asp
           115                 120                 125
   His Asp Gln Ile Val Thr Ser Arg Lys Ser Gln Glu Pro Ala Phe His
       130                 135                 140
   Ile Ala Gly Asp Ala Cys Leu Gln Arg Lys Met Asn Val Asp Val Ser
   145                 150                 155                 160
   Ser Thr Ile Tyr Asn Asn Glu Val Leu His Gln Pro Leu Ser Glu Ala
                   165                 170                 175
   Gln Arg Lys Ser Lys Ser Leu Lys Ile Asn Leu Asn Tyr Ala Gly Asp
               180                 185                 190
   Ala Leu Arg Glu Asn Thr Leu Val Ser Glu His Ala Gln Arg Asp Gln
           195                 200                 205
   Arg Glu Thr Gln Cys Gln Met Lys Glu Ala Glu His Met Tyr Gln Asn
       210                 215                 220
   Glu Gln Asp Asn Val Asn Lys His Thr Glu Gln Gln Glu Ser Leu Asp
   225                 230                 235                 240
   Gln Lys Leu Phe Gln Leu Gln Ser Lys Asn Met Trp Leu Gln Gln Gln
                   245                 250                 255
   Leu Val His Ala His Lys Lys Ala Asp Asn Lys Ser Lys Ile Thr Ile
               260                 265                 270
   Asp Ile His Phe Leu Glu Arg Lys Met Gln His His Leu Leu Lys Glu
           275                 280                 285
   Lys Asn Glu Glu Ile Phe Asn Tyr Asn Asn His Leu Lys Asn Arg Ile
       290                 295                 300
   Tyr Gln Tyr Glu Lys Glu Lys Ala Glu Thr Glu Val Ile
   305                 310                 315
<210> 15
   <211> 1665
   <212> DNA
   <213> Homo sapien
<400> 15
   gcaaactttc aagcagagcc tcccgagaag ccatctgcct tcgagcctgc cattgaaatg      60
   caaaagtctg ttccaaataa agccttggaa ttgaagaatg aacaaacatt gagagcagat     120
   cagatgttcc cttcagaatc aaaacaaaag aaggttgaag aaaattcttg ggattctgag     180
   agtctccgtg agactgtttc acagaaggat gtgtgtgtac ccaaggctac acatcaaaaa     240
   gaaatggata aaataagtgg aaaattagaa gattcaacta gcctatcaaa aatcttggat     300
   acagttcatt cttgtgaaag agcaagggaa cttcaaaaag atcactgtga acaacgtaca     360
   ggaaaaatgg aacaaatgaa aaagaagttt tgtgtactga aaaagaaact gtcagaagca     420
   aaagaaataa aatcacagtt agagaaccaa aaagttaaat gggaacaaga gctctgcagt     480
   gtgaggtttc tcacactcat gaaaatgaaa attatctctt acatgaaaat tgcatgttga     540
   aaaaggaaat tgccatgcta aaactggaaa tagccacact gaaacaccaa taccaggaaa     600
   aggaaaataa atactttgag gacattaaga ttttaaaaga aaagaatgct gaacttcaga     660
   tgaccctaaa actgaaagag gaatcattaa ctaaaagggc atctcaatat agtgggcagc    720
   ttaaagttct gatagctgag aacacaatgc tcacttctaa attgaaggaa aaacaagaca    780
   aagaaatact agaggcagaa attgaatcac accatcctag actggcttct gctgtacaag    840
   accatgatca aattgtgaca tcaagaaaaa gtcaagaacc tgctttccac attgcaggag    900
   atgcttgttt gcaaagaaaa atgaatgttg atgtgagtag tacgatatat aacaatgagg    960
   tgctccatca accactttct gaagctcaaa ggaaatccaa aagcctaaaa attaatctca   1020
   attatgccgg agatgctcta agagaaaata cattggtttc agaacatgca caaagagacc   1080
   aacgtgaaac acagtgtcaa atgaaggaag ctgaacacat gtatcaaaac gaacaagata   1140
   atgtgaacaa acacactgaa cagcaggagt ctctagatca gaaattattt caactacaaa   1200
   gcaaaaatat gtggcttcaa cagcaattag ttcatgcaca taagaaagct gacaacaaaa   1260
   gcaagataac aattgatatt cattttcttg agaggaaaat gcaacatcat ctcctaaaag   1320
   agaaaaatga ggagatattt aattacaata accatttaaa aaaccgtata tatcaatatg   1380
   aaaaagagaa agcagaaaca gaaaactcat gagagacaag cagtaagaaa cttcttttgg   1440
   agaaacaaca gaccagatct ttactcacaa ctcatgctag gaggccagtc ctagcattac   1500
   cttatgttga aaatcttacc aatagtctgt gtcaacagaa tacttatttt agaagaaaaa   1560
   ttcatgattt cttcctgaag cctgggcgac agagcgagac tctgtctcaa aaaaaaaaaa   1620
   aaaaaaagaa agaaagaaat gcctgtgctt acttcgcttc ccagg                   1665
<210> 16
   <211> 179
   <212> PRT
   <213> Homo sapien
<400> 16
   Ala Asn Phe Gln Ala Glu Pro Pro Glu Lys Pro Ser Ala Phe Glu Pro
    1               5                  10                  15
   Ala Ile Glu Met Gln Lys Ser Val Pro Asn Lys Ala Leu Glu Leu Lys
               20                  25                  30
   Asn Glu Gln Thr Leu Arg Ala Asp Gln Met Phe Pro Ser Glu Ser Lys
           35                  40                  45
   Gln Lys Lys Val Glu Glu Asn Ser Trp Asp Ser Glu Ser Leu Arg Glu
       50                  55                  60
   Thr Val Ser Gln Lys Asp Val Cys Val Pro Lys Ala Thr His Gln Lys
   65                  70                  75                  80
   Glu Met Asp Lys Ile Ser Gly Lys Leu Glu Asp Ser Thr Ser Leu Ser
                   85                  90                  95
   Lys Ile Leu Asp Thr Val His Ser Cys Glu Arg Ala Arg Glu Leu Gln
               100                 105                 110
   Lys Asp His Cys Glu Gln Arg Thr Gly Lys Met Glu Gln Met Lys Lys
           115                 120                 125
   Lys Phe Cys Val Leu Lys Lys Lys Leu Ser Glu Ala Lys Glu Ile Lys
       130                 135                 140
   Ser Gln Leu Glu Asn Gln Lys Val Lys Trp Glu Gln Glu Leu Cys Ser
   145                 150                 155                 160
   Val Arg Phe Leu Thr Leu Met Lys Met Lys Ile Ile Ser Tyr Met Lys
                   165                 170                 175
   Ile Ala Cys
<210> 17
   <211> 1681
   <212> DNA
   <213> Homo sapien
<400> 17
   gatacagtca ttcttgtgaa agagcaaggg aacttcaaaa agatcactgt gaacaacgta 60
   caggaaaaat ggaacaaatg aaaaagaagt tttgtgtact gaaaaagaaa ctgtcagaag 120
   caaaagaaat aaaatcacag ttagagaacc aaaaagttaa atgggaacaa gagctctgca 180
   gtgtgagatt gactttaaac caagaagaag agaagagaag aaatgccgat atattaaatg 240
   aaaaaattag ggaagaatta ggaagaatcg aagagcagca taggaaagag ttagaagtga 300
   aacaacaact tgaacaggct ctcagaatac aagatataga attgaagagt gtagaaagta 360
   atttgaatca ggtttctcac actcatgaaa atgaaaatta tctcttacat gaaaattgca 420
   tgttgaaaaa ggaaattgcc atgctaaaac tggaaatagc cacactgaaa caccaatacc 480
   aggaaaagga aaataaatac tttgaggaca ttaagatttt aaaagaaaag aatgctgaac 540
   ttcagatgac cctaaaactg aaagaggaat cattaactaa aagggcatct caatatagtg 600
   ggcagcttaa agttctgata gctgagaaca caatgctcac ttctaaattg aaggaaaaac 660
   aagacaaaga aatactagag gcagaaattg aatcacacca tcctagactg gcttctgctg 720
   tacaagacca tgatcaaatt gtgacatcaa gaaaaagtca agaacctgct ttccacattg 780
   caggagatgc ttgtttgcaa agaaaaatga atgttgatgt gagtagtacg atatataaca 840
   atgaggtgct ccatcaacca ctttctgaag ctcaaaggaa atccaaaagc ctaaaaatta 900
   atctcaatta tgccggagat gctctaagag aaaatacatt ggtttcagaa catgcacaaa 960
   gagaccaacg tgaaacacag tgtcaaatga aggaagctga acacatgtat caaaacgaac 1020
   aagataatgt gaacaaacac actgaacagc aggagtctct agatcagaaa ttatttcaac 1080
   tacaaagcaa aaatatgtgg cttcaacagc aattagttca tgcacataag aaagctgaca 1140
   acaaaagcaa gataacaatt gatattcatt ttcttgagag gaaaatgcaa catcatctcc 1200
   taaaagagaa aaatgaggag atatttaatt acaataacca tttaaaaaac cgtatatatc 1260
   aatatgaaaa agagaaagca gaaacagaaa actcatgaga gacaagcagt aagaaacttc 1320
   ttttggagaa acaacagacc agatctttac tcacaactca tgctaggagg ccagtcctag 1380
   cattacctta tgttgaaaaa tcttaccaat agtctgtgtc aacagaatac ttattttaga 1440
   agaaaaattc atgatttctt cctgaagcct acagacataa aataacagtg tgaagaatta 1500
   cttgttcacg aattgcataa aagctgccca ggatttccat ctaccctgga tgatgccgga 1560
   gacatcattc aatccaacca gaatctcgct ctgtcactca ggctggagtg cagtgggcgc 1620
   aatctcggct cactgcaact ctgcctccca ggttcacgcc attctctggc acagcctccc 1680
   g 1681
<210> 18
   <211> 432
   <212> PRT
   <213> Homo sapien
<400> 18
   Asp Thr Val His Ser Cys Glu Arg Ala Arg Glu Leu Gln Lys Asp His
   1               5                   10                  15
   Cys Glu Gln Arg Thr Gly Lys Met Glu Gln Met Lys Lys Lys Phe Cys
               20                  25                  30
   Val Leu Lys Lys Lys Leu Ser Glu Ala Lys Glu Ile Lys Ser Gln Leu
           35                  40                  45
   Glu Asn Gln Lys Val Lys Trp Glu Gln Glu Leu Cys Ser Val Arg Leu
       50                  55                  60
   Thr Leu Asn Gln Glu Glu Glu Lys Arg Arg Asn Ala Asp Ile Leu Asn
   65                  70                  75                  80
   Glu Lys Ile Arg Glu Glu Leu Gly Arg Ile Glu Glu Gln His Arg Lys
                   85                  90                  95
   Glu Leu Glu Val Lys Gln Gln Leu Glu Gln Ala Leu Arg Ile Gln Asp
               100                 105                 110
   Ile Glu Leu Lys Ser Val Glu Ser Asn Leu Asn Gln Val Ser His Thr
           115                 120                 125
   His Glu Asn Glu Asn Tyr Leu Leu His Glu Asn Cys Met Leu Lys Lys
       130                 135                 140
   Glu Ile Ala Met Leu Lys Leu Glu Ile Ala Thr Leu Lys His Gln Tyr
   145                 150                 155                 160
   Gln Glu Lys Glu Asn Lys Tyr Phe Glu Asp Ile Lys Ile Leu Lys Glu
                   165                 170                 175
   Lys Asn Ala Glu Leu Gln Met Thr Leu Lys Leu Lys Glu Glu Ser Leu
               180                 185                 190
   Thr Lys Arg Ala Ser Gln Tyr Ser Gly Gln Leu Lys Val Leu Ile Ala
           195                 200                 205
   Glu Asn Thr Met Leu Thr Ser Lys Leu Lys Glu Lys Gln Asp Lys Glu
       210                 215                 220
   Ile Leu Glu Ala Glu Ile Glu Ser His His Pro Arg Leu Ala Ser Ala
   225                 230                 235                 240
   Val Gln Asp His Asp Gln Ile Val Thr Ser Arg Lys Ser Gln Glu Pro
                   245                 250                 255
   Ala Phe His Ile Ala Gly Asp Ala Cys Leu Gln Arg Lys Met Asn Val
               260                 265                 270
   Asp Val Ser Ser Thr Ile Tyr Asn Asn Glu Val Leu His Gln Pro Leu
           275                 280                 285
   Ser Glu Ala Gln Arg Lys Ser Lys Ser Leu Lys Ile Asn Leu Asn Tyr
       290                 295                 300
   Ala Gly Asp Ala Leu Arg Glu Asn Thr Leu Val Ser Glu His Ala Gln
   305                 310                 315                 320
   Arg Asp Gln Arg Glu Thr Gln Cys Gln Met Lys Glu Ala Glu His Met
                   325                 330                 335
   Tyr Gln Asn Glu Gln Asp Asn Val Asn Lys His Thr Glu Gln Gln Glu
               340                 345                 350
   Ser Leu Asp Gln Lys Leu Phe Gln Leu Gln Ser Lys Asn Met Trp Leu
           355                 360                 365
   Gln Gln Gln Leu Val His Ala His Lys Lys Ala Asp Asn Lys Ser Lys
       370                 375                 380
   Ile Thr Ile Asp Ile His Phe Leu Glu Arg Lys Met Gln His His Leu
   385                 390                 395                 400
   Leu Lys Glu Lys Asn Glu Glu Ile Phe Asn Tyr Asn Asn His Leu Lys
                   405                 410                 415
   Asn Arg Ile Tyr Gln Tyr Glu Lys Glu Lys Ala Glu Thr Glu Asn Ser
               420                 425                 430
<210> 19
   <211> 3681
   <212> DNA
   <213> Homo sapiens
<400> 19
   tccgagctga ttacagacac caaggaagat gctgtaaaga gtcagcagcc acagccctgg 60
   ctagctggcc ctgtgggcat ttattagtaa agttttaatg acaaaagctt tgagtcaaca 120
   cacccgtggg taattaacct ggtcatcccc accctggaga gccatcctgc ccatgggtga 180
   tcaaagaagg aacatctgca ggaacacctg atgaggctgc acccttggcg gaaagaacac 240
   ctgacacagc tgaaagcttg gtggaaaaaa cacctgatga ggctgcaccc ttggtggaaa 300
   gaacacctga cacggctgaa agcttggtgg aaaaaacacc tgatgaggct gcatccttgg 360
   tggagggaac atctgacaaa attcaatgtt tggagaaagc gacatctgga aagttcgaac 420
   agtcagcaga agaaacacct agggaaatta cgagtcctgc aaaagaaaca tctgagaaat 480
   ttacgtggcc agcaaaagga agacctagga agatcgcatg ggagaaaaaa gaagacacac 540
   ctagggaaat tatgagtccc gcaaaagaaa catctgagaa atttacgtgg gcagcaaaag 600
   gaagacctag gaagatcgca tgggagaaaa aagaaacacc tgtaaagact ggatgcgtgg 660
   caagagtaac atctaataaa actaaagttt tggaaaaagg aagatctaag atgattgcat 720
   gtcctacaaa agaatcatct acaaaagcaa gtgccaatga tcagaggttc ccatcagaat 780
   ccaaacaaga ggaagatgaa gaatattctt gtgattctcg gagtctcttt gagagttctg 840
   caaagattca agtgtgtata cctgagtcta tatatcaaaa agtaatggag ataaatagag 900
   aagtagaaga gcctcctaag aagccatctg ccttcaagcc tgccattgaa atgcaaaact 960
   ctgttccaaa taaagccttt gaattgaaga atgaacaaac attgagagca gatccgatgt 1020
   tcccaccaga atccaaacaa aaggactatg aagaaaattc ttgggattct gagagtctct 1080
   gtgagactgt ttcacagaag gatgtgtgtt tacccaaggc tacacatcaa aaagaaatag 1140
   ataaaataaa tggaaaatta gaagagtctc ctaataaaga tggtcttctg aaggctacct 1200
   gcggaatgaa agtttctatt ccaactaaag ccttagaatt gaaggacatg caaactttca 1260
   aagcagagcc tccggggaag ccatctgcct tcgagcctgc cactgaaatg caaaagtctg 1320
   tcccaaataa agccttggaa ttgaaaaatg aacaaacatt gagagcagat gagatactcc 1380
   catcagaatc caaacaaaag gactatgaag aaagttcttg ggattctgag agtctctgtg 1440
   agactgtttc acagaaggat gtgtgtttac ccaaggctrc rcatcaaaaa gaaatagata 1500
   aaataaatgg aaaattagaa gggtctcctg ttaaagatgg tcttctgaag gctaactgcg 1560
   gaatgaaagt ttctattcca actaaagcct tagaattgat ggacatgcaa actttcaaag 1620
   cagagcctcc cgagaagcca tctgccttcg agcctgccat tgaaatgcaa aagtctgttc 1680
   caaataaagc cttggaattg aagaatgaac aaacattgag agcagatgag atactcccat 1740
   cagaatccaa acaaaaggac tatgaagaaa gttcttggga ttctgagagt ctctgtgaga 1800
   ctgtttcaca gaaggatgtg tgtttaccca aggctrcrca tcaaaaagaa atagataaaa 1860
   taaatggaaa attagaagag tctcctgata atgatggttt tctgaaggct ccctgcagaa 1920
   tgaaagtttc tattccaact aaagccttag aattgatgga catgcaaact ttcaaagcag 1980
   agcctcccga gaagccatct gccttcgagc ctgccattga aatgcaaaag tctgttccaa 2040
   ataaagcctt ggaattgaag aatgaacaaa cattgagagc agatcagatg ttcccttcag 2100
   aatcaaaaca aaagaasgtt gaagaaaatt cttgggattc tgagagtctc cgtgagactg 2160
   tttcacagaa ggatgtgtgt gtacccaagg ctacacatca aaaagaaatg gataaaataa 2220
   gtggaaaatt agaagattca actagcctat caaaaatctt ggatacagtt cattcttgtg 2280
   aaagagcaag ggaacttcaa aaagatcact gtgaacaacg tacaggaaaa atggaacaaa 2340
   tgaaaaagaa gttttgtgta ctgaaaaaga aactgtcaga agcaaaagaa ataaaatcac 2400
   agttagagaa ccaaaaagtt aaatgggaac aagagctctg cagtgtgagg tttctcacac 2460
   tcatgaaaat gaaaattatc tcttacatga aaattgcatg ttgaaaaagg aaattgccat 2520
   gctaaaactg gaaatagcca cactgaaaca ccaataccag gaaaaggaaa ataaatactt 2580
   tgaggacatt aagattttaa aagaaaagaa tgctgaactt cagatgaccc taaaactgaa 2640
   agaggaatca ttaactaaaa gggcatctca atatagtggg cagcttaaag ttctgatagc 2700
   tgagaacaca atgctcactt ctaaattgaa ggaaaaacaa gacaaagaaa tactagaggc 2760
   agaaattgaa tcacaccatc ctagactggc ttctgctgta caagaccatg atcaaattgt 2820
   gacatcaaga aaaagtcaag aacctgcttt ccacattgca ggagatgctt gtttgcaaag 2880
   aaaaatgaat gttgatgtga gtagtacgat atataacaat gaggtgctcc atcaaccact 2940
   ttctgaagct caaaggaaat ccaaaagcct aaaaattaat ctcaattatg cmggagatgc 3000
   tctaagagaa aatacattgg tttcagaaca tgcacaaaga gaccaacgtg aaacacagtg 3060
   tcaaatgaag gaagctgaac acatgtatca aaacgaacaa gataatgtga acaaacacac 3120
   tgaacagcag gagtctctag atcagaaatt atttcaacta caaagcaaaa atatgtggct 3180
   tcaacagcaa ttagttcatg cacataagaa agctgacaac aaaagcaaga taacaattga 3240
   tattcatttt cttgagagga aaatgcaaca tcatctccta aaagagaaaa atgaggagat 3300
   atttaattac aataaccatt taaaaaaccg tatatatcaa tatgaaaaag agaaagcaga 3360
   aacagaaaac tcatgagaga caagcagtaa gaaacttctt ttggagaaac aacagaccag 3420
   atctttactc acaactcatg ctaggaggcc agtcctagca tcaccttatg ttgaaaatct 3480
   taccaatagt ctgtgtcaac agaatactta ttttagaaga aaaattcatg atttcttcct 3540
   gaagcctaca gacataaaat aacagtgtga agaattactt gttcacgaat tgcataaagc 3600
   tgcacaggat tcccatctac cctgatgatg cagcagacat cattcaatcc aaccagaatc 3660
   tcgctctgtc actcaggctg g                                          3681
<210> 20
   <211> 1424
   <212> DNA
   <213> Homo sapiens
<400> 20
   tccgagctga ttacagacac caaggaagat gctgtaaaga gtcagcagcc acagccctgg 60
   ctagctggcc ctgtgggcat ttattagtaa agttttaatg acaaaagctt tgagtcaaca 120
   cacccgtggg taattaacct ggtcatcccc accctggaga gccatcctgc ccatgggtga 180
   tcaaagaagg aacatctgca ggaacacctg atgaggctgc acccttggcg gaaagaacac 240
   ctgacacagc tgaaagcttg gtggaaaaaa cacctgatga ggctgcaccc ttggtggaaa 300
   gaacacctga cacggctgaa agcttggtgg aaaaaacacc tgatgaggct gcatccttgg 360
   tggagggaac atctgacaaa attcaatgtt tggagaaagc gacatctgga aagttcgaac 420
   agtcagcaga agaaacacct agggaaatta cgagtcctgc aaaagaaaca tctgagaaat 480
   ttacgtggcc agcaaaagga agacctagga agatcgcatg ggagaaaaaa gaagacacac 540
   ctagggaaat tatgagtccc gcaaaagaaa catctgagaa atttacgtgg gcagcaaaag 600
   gaagacctag gaagatcgca tgggagaaaa aagaaacacc tgtaaagact ggatgcgtgg 660
   caagagtaac atctaataaa actaaagttt tggaaaaagg aagatctaag atgattgcat 720
   gtcctacaaa agaatcatct acaaaagcaa gtgccaatga tcagaggttc ccatcagaat 780
   ccaaacaaga ggaagatgaa gaatattctt gtgattctcg gagtctcttt gagagttctg 840
   caaagattca agtgtgtata cctgagtcta tatatcaaaa agtaatggag ataaatagag 900
   aagtagaaga gcctcctaag aagccatctg ccttcaagcc tgccattgaa atgcaaaact 960
   ctgttccaaa taaagccttt gaattgaaga atgaacaaac attgagagca gatccgatgt 1020
   tcccaccaga atccaaacaa aaggactatg aagaaaattc ttgggattct gagagtctct 1080
   gtgagactgt ttcacagaag gatgtgtgtt tacccaaggc tacacatcaa aaagaaatag 1140
   ataaaataaa tggaaaatta gaaggtaaga accgtttttt atttaaaaat cagttgaccg 1200
   aatatttctc taaactgatg aggagggata tcctctagta gctgaagaaa attacctcct 1260
   aaatgcaaac catggaaaaa aagagaagtg caatggtcgt aagttgtatg tctcatcagg 1320
   tgttggcaac agactatatt gagagtgctg aaaaggagct gaattattag tttgaattca 1380
   agatattgca agacctgaga gaaaaaaaaa aaaaaaaaaa aaaa                  1424
<210> 21
   <211> 674
   <212> DNA
   <213> Homo sapiens
<400> 21
   attccgagct gattacagac accaaggaag atgctgtaaa gagtcagcag ccacagccct 60
   ggctagctgg ccctgtgggc atttattagt aaagttttaa tgacaaaagc tttgagtcaa 120
   cacacccgtg ggtaattaac ctggtcatcc ccaccctgga gagccatcct gcccatgggt 180
   gatcaaagaa ggaacatctg caggaacacc tgatgaggct gcacccttgg cggaaagaac 240
   acctgacaca gctgaaagct tggtggaaaa aacacctgat gaggctgcac ccttggtgga 300
   aagaacacct gacacggctg aaagcttggt ggaaaaaaca cctgatgagg ctgcatcctt 360
   ggtggaggga acatctgaca aaattcaatg tttggagaaa gcgacatctg gaaagttcga 420
   acagtcagca gaagaaacac ctagggaaat tacgagtcct gcaaaagaaa catctgagaa 480
   atttacgtgg ccagcaaaag gaagacctag gaagatcgca tgggagaaaa aagatgactc 540
   agttaaggca aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaa                                                   674
<210> 22
   <211> 1729
   <212> DNA
   <213> Homo sapiens
<220>
   <221> unsure
   <222> (11)
   <223> n=A,T,C or G
   <221> unsure
   <222> (1128)
   <223> n=A,T,C or G
<400> 22
   gaaagttcga ncagtcagca gaagaaacac ctagggaaat tacgagtcct gcaaaagaaa 60
   catctgagaa atttacgtgg ccagcaaaag gaagacctag gaagatcgca tgggagaaaa 120
   aagaagacac acctagggaa attatgagtc ccgcaaaaga aacatctgag aaatttacgt 180
   gggcagcaaa aggaagacct aggaagatcg catgggagaa aaaagaaaca cctgtaaaga 240
   ctggatgcgt ggcaagagta acatctaata aaactaaagt tttggaaaaa ggaagatcta 300
   agatgattgc atgtcctaca aaagaatcat ctacaaaagc aagtgccaat gatcagaggt 360
   tcccatcaga atccaaacaa gaggaagatg aagaatattc ttgtgattct cggagtctct 420
   ttgagagttc tgcaaagatt caagtgtgta tacctgagtc tatatatcaa aaagtaatgg 480
   agataaatag agaagtagaa gagcctccta agaagccatc tgccttcaag cctgccattg 540
   aaatgcaaaa ctctgttcca aataaagcct ttgaattgaa gaatgaacaa acattgagag 600
   cagatccgat gttcccacca gaatccaaac aaaaggacta tgaagaaaat tcttgggatt 660
   ctgagagtct ctgtgagact gtttcacaga aggatgtgtg tttacccaag gctacacatc 720
   aaaaagaaat agataaaata aatggaaaat tagaagagtc tcctaataaa gatggtcttc 780
   tgaaggctac ctgcggaatg aaagtttcta ttccaactaa agccttagaa ttgaaggaca 840
   tgcaaacttt caaagcagag cctccgggga agccatctgc cttcgagcct gccactgaaa 900
   tgcaaaagtc tgtcccaaat aaagccttgg aattgaaaaa tgaacaaaca ttgagagcag 960
   atgagatact cccatcagaa tccaaacaaa aggactatga agaaaattct tgggatactg 1020
   agagtctctg tgagactgtt tcacagaagg atgtgtgttt acccaaggct gcgcatcaaa 1080
   aagaaataga taaaataaat ggaaaattag aagggtctcc tggtaaanat ggtcttctga 1140
   aggctaactg cggaatgaaa gtttctattc caactaaagc cttagaattg atggacatgc 1200
   aaactttcaa agcagagcct cccgagaagc catctgcctt cgagcctgcc attgaaatgc 1260
   aaaagtctgt tccaaataaa gccttggaat tgaagaatga acaaacattg agagcagatg 1320
   agatactccc atcagaatcc aaacaaaagg actatgaaga aagttcttgg gattctgaga 1380
   gtctctgtga gactgtttca cagaaggatg tgtgtttacc caaggctgcg catcaaaaag 1440
   aaatagataa aataaatgga aaattagaag gtaagaaccg ttttttattt aaaaatcatt 1500
   tgaccaaata tttctctaaa ttgatgagga aggatatcct ctagtagctg aagaaaatta 1560
   cctcctaaat gcaaaccatg gaaaaaaaga gaagtgcaat ggtcataagc tatgtgtctc 1620
   atcaggcatt ggcaacagac tatattgtga gtgctgaaga ggagctgaat tactagttta 1680
   aattcaagat attccaagac gtgaggaaaa tgagaaaaaa aaaaaaaaa           1729
<210> 23
   <211> 1337
   <212> DNA
   <213> Homo sapiens
<400> 23
   aaaaagaaat agataaaata aatggaaaat tagaagggtc tcctgttaaa gatggtcttc 60
   tgaaggctaa ctgcggaatg aaagtttcta ttccaactaa agccttagaa ttgatggaca 120
   tgcaaacttt caaagcagag cctcccgaga agccatctgc cttcgagcct gccattgaaa 180
   tgcaaaagtc tgttccaaat aaagccttgg aattgaagaa tgaacaaaca ttgagagcag 240
   atgagatact cccatcagaa tccaaacaaa aggactatga agaaagttct tgggattctg 300
   agagtctctg tgagactgtt tcacagaagg atgtgtgttt acccaaggct gcgcatcaaa 360
   aagaaataga taaaataaat ggaaaattag aagagtctcc tgataatgat ggttttctga 420
   aggctccctg cagaatgaaa gtttctattc caactaaagc cttagaattg atggacatgc 480
   aaactttcaa agcagagcct cccgagaagc catctgcctt cgagcctgcc attgaaatgc 540
   aaaagtctgt tccaaataaa gccttggaat tgaagaatga acaaacattg agagcagatc 600
   agatgttccc ttcagaatca aaacaaaaga aggttgaaga aaattcttgg gattctgaga 660
   gtctccgtga gactgtttca cagaaggatg tgtgtgtacc caaggctaca catcaaaaag 720
   aaatggataa aataagtgga aaattagaag attcaactag cctatcaaaa atcttggata 780
   cagttcattc ttgtgaaaga gcaagggaac ttcaaaaaga tcactgtgaa caacgtacag 840
   gaaaaatgga acaaatgaaa aagaagtttt gtgtactgaa aaagaaactg tcagaagcaa 900
   aagaaataaa atcacagtta gagaaccaaa aagttaaatg ggaacaagag ctctgcagtg 960
   tgagattgac tttaaaccaa gaagaagaga agagaagaaa tgccgatata ttaaatgaaa 1020
   aaattaggga agaattagga agaatcgaag agcagcatag gaaagagtta gaagtgaaac 1080
   aacaacttga acaggctctc agaatacaag atatagaatt gaagagtgta gaaagtaatt 1140
   tgaatcaggt ttctcacact catgaaaatg aaaattatct cttacatgaa aattgcatgt 1200
   tgaaaaagga aattgccatg ctaaaactgg aaatagccac actgaaacac caataccagg 1260
   aaaaggaaaa taaatacttt gaggacatta agattttaaa agaaaagaat gctgaacttc 1320
   agatgacccc tcgtgcc                                                1337
<210> 24
   <211> 2307
   <212> DNA
   <213> Homo sapiens
<400> 24
   attgagagca gatgagatac tcccatcaga atccaaacaa aaggactatg aagaaagttc 60
   ttgggattct gagagtctct gtgagactgt ttcacagaag gatgtgtgtt tacccaaggc 120
   tacacatcaa aaagaaatag ataaaataaa tggaaaatta gaagggtctc ctgttaaaga 180
   tggtcttctg aaggctaact gcggaatgaa agtttctatt ccaactaaag ccttagaatt 240
   gatggacatg caaactttca aagcagagcc tcccgagaag ccatctgcct tcgagcctgc 300
   cattgaaatg caaaagtctg ttccaaataa agccttggaa ttgaagaatg aacaaacatt 360
   gagagcagat gagatactcc catcagaatc caaacaaaag gactatgaag aaagttcttg 420
   ggattctgag agtctctgtg agactgtttc acagaaggat gtgtgtttac ccaaggctac 480
   acatcaaaaa gaaatagata aaataaatgg aaaattagaa gagtctcctg ataatgatgg 540
   ttttctgaag tctccctgca gaatgaaagt ttctattcca actaaagcct tagaattgat 600
   ggacatgcaa actttcaaag cagagcctcc cgagaagcca tctgccttcg agcctgccat 660
   tgaaatgcaa aagtctgttc caaataaagc cttggaattg aagaatgaac aaacattgag 720
   agcagatcag atgttccctt cagaatcaaa acaaaagaac gttgaagaaa attcttggga 780
   ttctgagagt ctccgtgaga ctgtttcaca gaaggatgtg tgtgtaccca aggctacaca 840
   tcaaaaagaa atggataaaa taagtggaaa attagaagat tcaactagcc tatcaaaaat 900
   cttggataca gttcattctt gtgaaagagc aagggaactt caaaaagatc actgtgaaca 960
   acgtacagga aaaatggaac aaatgaaaaa gaagttttgt gtactgaaaa agaaactgtc 1020
   agaagcaaaa gaaataaaat cacagttaga gaaccaaaaa gttaaatggg aacaagagct 1080
   ctgcagtgtg aggtttctca cactcatgaa aatgaaaatt atctcttaca tgaaaattgc 1140
   atgttgaaaa aggaaattgc catgctaaaa ctggaaatag ccacactgaa acaccaatac 1200
   caggaaaagg aaaataaata ctttgaggac attaagattt taaaagaaaa gaatgctgaa 1260
   cttcagatga ccctaaaact gaaagaggaa tcattaacta aaagggcatc tcaatatagt 1320
   gggcagctta aagttctgat agctgagaac acaatgctca cttctaaatt gaaggaaaaa 1380
   caagacaaag aaatactaga ggcagaaatt gaatcacacc atcctagact ggcttctgct 1440
   gtacaagacc atgatcaaat tgtgacatca agaaaaagtc aagaacctgc tttccacatt 1500
   gcaggagatg cttgtttgca aagaaaaatg aatgttgatg tgagtagtac gatatataac 1560
   aatgaggtgc tccatcaacc actttctgaa gctcaaagga aatccaaaag cctaaaaatt 1620
   aatctcaatt atgcaggaga tgctctaaga gaaaatacat tggtttcaga acatgcacaa 1680
   agagaccaac gtgaaacaca gtgtcaaatg aaggaagctg aacacatgta tcaaaacgaa 1740
   caagataatg tgaacaaaca cactgaacag caggagtctc tagatcagaa attatttcaa 1800
   ctacaaagca aaaatatgtg gcttcaacag caattagttc atgcacataa gaaagctgac 1860
   aacaaaagca agataacaat tgatattcat tttcttgaga ggaaaatgca acatcatctc 1920
   ctaaaagaga aaaatgagga gatatttaat tacaataacc atttaaaaaa ccgtatatat 1980
   caatatgaaa aagagaaagc agaaacagaa aactcatgag agacaagcag taagaaactt 2040
   cttttggaga aacaacagac cagatcttta ctcacaactc atgctaggag gccagtccta 2100
   gcatcacctt atgttgaaaa tcttaccaat agtctgtgtc aacagaatac ttattttaga 2160
   agaaaaattc atgatttctt cctgaagcct acagacataa aataacagtg tgaagaatta 2220
   cttgttcacg aattgcataa agctgcacag gattcccatc taccctgatg atgcagcaga 2280
   catcattcaa tccaaccaga atctcgc 2307
<210> 25
   <211> 650
   <212> PRT
   <213> Homo sapiens
<220>
   <221> unsure
   <222> (310)
   <223> Xaa = Any Amino Acid
   <221> unsure
   <222> (429)
   <223> Xaa = Any Amino Acid
   <221> unsure
   <222> (522)
   <223> Xaa = Any Amino Acid
<400> 25
   Met Ser Pro Ala Lys Glu Thr Ser Glu Lys Phe Thr Trp Ala Ala Lys
   5 10 15
   Gly Arg Pro Arg Lys Ile Ala Trp Glu Lys Lys Glu Thr Pro Val Lys
               20                  25                  30
   Thr Gly Cys Val Ala Arg Val Thr Ser Asn Lys Thr Lys Val Leu Glu
           35                  40                  45
   Lys Gly Arg Ser Lys Met Ile Ala Cys Pro Thr Lys Glu Ser Ser Thr
       50                  55                  60
   Lys Ala Ser Ala Asn Asp Gln Arg Phe Pro Ser Glu Ser Lys Gln Glu
   65                  70                  75                  80
   Glu Asp Glu Glu Tyr Ser Cys Asp Ser Arg Ser Leu Phe Glu Ser Ser
                   85                  90                  95
   Ala Lys Ile Gln Val Cys Ile Pro Glu Ser Ile Tyr Gln Lys Val Met
               100                 105                 110
   Glu Ile Asn Arg Glu Val Glu Glu Pro Pro Lys Lys Pro Ser Ala Phe
           115                 120                 125
   Lys Pro Ala Ile Glu Met Gln Asn Ser Val Pro Asn Lys Ala Phe Glu
       130                 135                 140
   Leu Lys Asn Glu Gln Thr Leu Arg Ala Asp Pro Met Phe Pro Pro Glu
   145                 150                 155                 160
   Ser Lys Gln Lys Asp Tyr Glu Glu Asn Ser Trp Asp Ser Glu Ser Leu
                   165                 170                 175
   Cys Glu Thr Val Ser Gln Lys Asp Val Cys Leu Pro Lys Ala Thr His
               180                 185                 190
   Gln Lys Glu Ile Asp Lys Ile Asn Gly Lys Leu Glu Glu Ser Pro Asn
           195                 200                 205
   Lys Asp Gly Leu Leu Lys Ala Thr Cys Gly Met Lys Val Ser Ile Pro
       210                 215                 220
   Thr Lys Ala Leu Glu Leu Lys Asp Met Gln Thr Phe Lys Ala Glu Pro
   225                 230                 235                 240
   Pro Gly Lys Pro Ser Ala Phe Glu Pro Ala Thr Glu Met Gln Lys Ser
                   245                 250                 255
   Val Pro Asn Lys Ala Leu Glu Leu Lys Asn Glu Gln Thr Leu Arg Ala
               260                 265                 270
   Asp Glu Ile Leu Pro Ser Glu Ser Lys Gln Lys Asp Tyr Glu Glu Ser
           275                 280                 285
   Ser Trp Asp Ser Glu Ser Leu Cys Glu Thr Val Ser Gln Lys Asp Val
       290                 295                 300
   Cys Leu Pro Lys Ala Xaa His Gln Lys Glu Ile Asp Lys Ile Asn Gly
   305                 310                 315                 320
   Lys Leu Glu Gly Ser Pro Val Lys Asp Gly Leu Leu Lys Ala Asn Cys
                   325                 330                 335
   Gly Met Lys Val Ser Ile Pro Thr Lys Ala Leu Glu Leu Met Asp Met
   340 345 350
   Gln Thr Phe Lys Ala Glu Pro Pro Glu Lys Pro Ser Ala Phe Glu Pro
   355 360 365
   Ala Ile Glu Met Gln Lys Ser Val Pro Asn Lys Ala Leu Glu Leu Lys
   370 375 380
   Asn Glu Gln Thr Leu Arg Ala Asp Glu Ile Leu Pro Ser Glu Ser Lys
   385 390 395 400
   Gln Lys Asp Tyr Glu Glu Ser Ser Trp Asp Ser Glu Ser Leu Cys Glu
   405 410 415
   Thr Val Ser Gln Lys Asp Val Cys Leu Pro Lys Ala Xaa His Gln Lys
   420 425 430
   Glu Ile Asp Lys Ile Asn Gly Lys Leu Glu Glu Ser Pro Asp Asn Asp
   435 440 445
   Gly Phe Leu Lys Ala Pro Cys Arg Met Lys Val Ser Ile Pro Thr Lys
   450 455 460
   Ala Leu Glu Leu Met Asp Met Gln Thr Phe Lys Ala Glu Pro Pro Glu
   465 470 475 480
   Lys Pro Ser Ala Phe Glu Pro Ala Ile Glu Met Gln Lys Ser Val Pro
   485 490 495
   Asn Lys Ala Leu Glu Leu Lys Asn Glu Gln Thr Leu Arg Ala Asp Gln
   500 505 510
   Met Phe Pro Ser Glu Ser Lys Gln Lys Xaa Val Glu Glu Asn Ser Trp
   515 520 525
   Asp Ser Glu Ser Leu Arg Glu Thr Val Ser Gln Lys Asp Val Cys Val
   530 535 540
   Pro Lys Ala Thr His Gln Lys Glu Met Asp Lys Ile Ser Gly Lys Leu
   545 550 555 560
   Glu Asp Ser Thr Ser Leu Ser Lys Ile Leu Asp Thr Val His Ser Cys
   565 570 575
   Glu Arg Ala Arg Glu Leu Gln Lys Asp His Cys Glu Gln Arg Thr Gly
   580 585 590
   Lys Met Glu Gln Met Lys Lys Lys Phe Cys Val Leu Lys Lys Lys Leu
   595 600 605
   Ser Glu Ala Lys Glu Ile Lys Ser Gln Leu Glu Asn Gln Lys Val Lys
   610 615 620
   Trp Glu Gln Glu Leu Cys Ser Val Arg Phe Leu Thr Leu Met Lys Met
   625 630 635 640
   Lys Ile Ile Ser Tyr Met Lys Ile Ala Cys
   645 650
<210> 26
   <211> 228
   <212> PRT
   <213> Homo sapiens
<400> 26
   Met Ser Pro Ala Lys Glu Thr Ser Glu Lys Phe Thr Trp Ala Ala Lys
                   5                   10                  15
   Gly Arg Pro Arg Lys Ile Ala Trp Glu Lys Lys Glu Thr Pro Val Lys
               20                  25                  30
   Thr Gly Cys Val Ala Arg Val Thr Ser Asn Lys Thr Lys Val Leu Glu
           35                  40                  45
   Lys Gly Arg Ser Lys Met Ile Ala Cys Pro Thr Lys Glu Ser Ser Thr
       50                  55                  60
   Lys Ala Ser Ala Asn Asp Gln Arg Phe Pro Ser Glu Ser Lys Gln Glu
   65                  70                  75                  80
   Glu Asp Glu Glu Tyr Ser Cys Asp Ser Arg Ser Leu Phe Glu Ser Ser
                   85                  90                  95
   Ala Lys Ile Gln Val Cys Ile Pro Glu Ser Ile Tyr Gln Lys Val Met
               100                 105                 110
   Glu Ile Asn Arg Glu Val Glu Glu Pro Pro Lys Lys Pro Ser Ala Phe
           115                 120                 125
   Lys Pro Ala Ile Glu Met Gln Asn Ser Val Pro Asn Lys Ala Phe Glu
       130                 135                 140
   Leu Lys Asn Glu Gln Thr Leu Arg Ala Asp Pro Met Phe Pro Pro Glu
   145                 150                 155                 160
   Ser Lys Gln Lys Asp Tyr Glu Glu Asn Ser Trp Asp Ser Glu Ser Leu
                   165                 170                 175
   Cys Glu Thr Val Ser Gln Lys Asp Val Cys Leu Pro Lys Ala Thr His
               180                 185                 190
   Gln Lys Glu Ile Asp Lys Ile Asn Gly Lys Leu Glu Gly Lys Asn Arg
           195                 200                 205
   Phe Leu Phe Lys Asn Gln Leu Thr Glu Tyr Phe Ser Lys Leu Met Arg
       210                 215                 220
   Arg Asp Ile Leu
   225
<210> 27
   <211> 154
   <212> PRT
   <213> Homo sapiens
<220>
   <221> unsure
   <222> (148)
   <223> Xaa = Any Amino Acid
<400> 27
   Met Arg Leu His Pro Trp Arg Lys Glu His Leu Thr Gln Leu Lys Ala
   5 10 15
   Trp Trp Lys Lys His Leu Met Arg Leu His Pro Trp Trp Lys Glu His
   20 25 30
   Leu Thr Arg Leu Lys Ala Trp Trp Lys Lys His Leu Met Arg Leu His
   35 40 45
   Pro Trp Trp Arg Glu His Leu Thr Lys Phe Asn Val Trp Arg Lys Arg
   50 55 60
   His Leu Glu Ser Ser Asn Ser Gln Gln Lys Lys His Leu Gly Lys Leu
   65 70 75 80
   Arg Val Leu Gln Lys Lys His Leu Arg Asn Leu Arg Gly Gln Gln Lys
   85 90 95
   Glu Asp Leu Gly Arg Ser His Gly Arg Lys Lys Met Thr Gln Leu Arg
   100 105 110
   Gln Lys Lys Lys Lys Lys Lys Lys Lys Lys Lys Lys Lys Lys Lys Lys
   115 120 125
   Lys Lys Lys Lys Lys Lys Lys Lys Lys Lys Lys Lys Lys Lys Lys Lys
   130 135 140
   Lys Lys Lys Xaa Lys Lys Lys Lys Lys Lys
   145 150
<210> 28
   <211> 466
   <212> PRT
   <213> Homo sapiens
<220>
   <221> unsure
   <222> (329)
   <223> Xaa = Any Amino Acid
<400> 28
   Met Ser Pro Ala Lys Glu Thr Ser Glu Lys Phe Thr Trp Ala Ala Lys
   5 10 15
   Gly Arg Pro Arg Lys Ile Ala Trp Glu Lys Lys Glu Thr Pro Val Lys
   20 25 30
   Thr Gly Cys Val Ala Arg Val Thr Ser Asn Lys Thr Lys Val Leu Glu
   35 40 45
   Lys Gly Arg Ser Lys Met Ile Ala Cys Pro Thr Lys Glu Ser Ser Thr
   50 55 60
   Lys Ala Ser Ala Asn Asp Gln Arg Phe Pro Ser Glu Ser Lys Gln Glu
   65 70 75 80
   Glu Asp Glu Glu Tyr Ser Cys Asp Ser Arg Ser Leu Phe Glu Ser Ser
   85 90 95
   Ala Lys Ile Gln Val Cys Ile Pro Glu Ser Ile Tyr Gln Lys Val Met
   100 105 110
   Glu Ile Asn Arg Glu Val Glu Glu Pro Pro Lys Lys Pro Ser Ala Phe
   115 120 125
   Lys Pro Ala Ile Glu Met Gln Asn Ser Val Pro Asn Lys Ala Phe Glu
   130 135 140
   Leu Lys Asn Glu Gln Thr Leu Arg Ala Asp Pro Met Phe Pro Pro Glu
   145 150 155 160
   Ser Lys Gln Lys Asp Tyr Glu Glu Asn Ser Trp Asp Ser Glu Ser Leu
   165 170 175
   Cys Glu Thr Val Ser Gln Lys Asp Val Cys Leu Pro Lys Ala Thr His
   180 185 190
   Gln Lys Glu Ile Asp Lys Ile Asn Gly Lys Leu Glu Glu Ser Pro Asn
   195 200 205
   Lys Asp Gly Leu Leu Lys Ala Thr Cys Gly Met Lys Val Ser Ile Pro
   210 215 220
   Thr Lys Ala Leu Glu Leu Lys Asp Met Gln Thr Phe Lys Ala Glu Pro
   225 230 235 240
   Pro Gly Lys Pro Ser Ala Phe Glu Pro Ala Thr Glu Met Gln Lys Ser
   245 250 255
   Val Pro Asn Lys Ala Leu Glu Leu Lys Asn Glu Gln Thr Leu Arg Ala
   260 265 270
   Asp Glu Ile Leu Pro Ser Glu Ser Lys Gln Lys Asp Tyr Glu Glu Asn
   275 280 285
   Ser Trp Asp Thr Glu Ser Leu Cys Glu Thr Val Ser Gln Lys Asp Val
   290 295 300
   Cys Leu Pro Lys Ala Ala His Gln Lys Glu Ile Asp Lys Ile Asn Gly
   305 310 315 320
   Lys Leu Glu Gly Ser Pro Gly Lys Xaa Gly Leu Leu Lys Ala Asn Cys
   325 330 335
   Gly Met Lys Val Ser Ile Pro Thr Lys Ala Leu Glu Leu Met Asp Met
   340 345 350
   Gln Thr Phe Lys Ala Glu Pro Pro Glu Lys Pro Ser Ala Phe Glu Pro
   355 360 365
   Ala Ile Glu Met Gln Lys Ser Val Pro Asn Lys Ala Leu Glu Leu Lys
   370 375 380
   Asn Glu Gln Thr Leu Arg Ala Asp Glu Ile Leu Pro Ser Glu Ser Lys
   385 390 395 400
   Gln Lys Asp Tyr Glu Glu Ser Ser Trp Asp Ser Glu Ser Leu Cys Glu
   405 410 415
   Thr Val Ser Gln Lys Asp Val Cys Leu Pro Lys Ala Ala His Gln Lys
   420 425 430
   Glu Ile Asp Lys Ile Asn Gly Lys Leu Glu Gly Lys Asn Arg Phe Leu
   435 440 445
   Phe Lys Asn His Leu Thr Lys Tyr Phe Ser Lys Leu Met Arg Lys Asp
   450 455 460
   Ile Leu
   465
<210> 29
   <211> 445
   <212> PRT
   <213> Homo sapiens
<400> 29
   Lys Glu Ile Asp Lys Ile Asn Gly Lys Leu Glu Gly Ser Pro Val Lys
   5 10 15
   Asp Gly Leu Leu Lys Ala Asn Cys Gly Met Lys Val Ser Ile Pro Thr
   20 25 30
   Lys Ala Leu Glu Leu Met Asp Met Gln Thr Phe Lys Ala Glu Pro Pro
   35 40 45
   Glu Lys Pro Ser Ala Phe Glu Pro Ala Ile Glu Met Gln Lys Ser Val
   50 55 60
   Pro Asn Lys Ala Leu Glu Leu Lys Asn Glu Gln Thr Leu Arg Ala Asp
   65 70 75 80
   Glu Ile Leu Pro Ser Glu Ser Lys Gln Lys Asp Tyr Glu Glu Ser Ser
   85 90 95
   Trp Asp Ser Glu Ser Leu Cys Glu Thr Val Ser Gln Lys Asp Val Cys
   100 105 110
   Leu Pro Lys Ala Ala His Gln Lys Glu Ile Asp Lys Ile Asn Gly Lys
   115 120 125
   Leu Glu Glu Ser Pro Asp Asn Asp Gly Phe Leu Lys Ala Pro Cys Arg
   130 135 140
   Met Lys Val Ser Ile Pro Thr Lys Ala Leu Glu Leu Met Asp Met Gln
   145                 150                 155                 160
   Thr Phe Lys Ala Glu Pro Pro Glu Lys Pro Ser Ala Phe Glu Pro Ala
                   165                 170                 175
   Ile Glu Met Gln Lys Ser Val Pro Asn Lys Ala Leu Glu Leu Lys Asn
               180                 185                 190
   Glu Gln Thr Leu Arg Ala Asp Gln Met Phe Pro Ser Glu Ser Lys Gln
           195                 200                 205
   Lys Lys Val Glu Glu Asn Ser Trp Asp Ser Glu Ser Leu Arg Glu Thr
       210                 215                 220
   Val Ser Gln Lys Asp Val Cys Val Pro Lys Ala Thr His Gln Lys Glu
   225                 230                 235                 240
   Met Asp Lys Ile Ser Gly Lys Leu Glu Asp Ser Thr Ser Leu Ser Lys
                   245                 250                 255
   Ile Leu Asp Thr Val His Ser Cys Glu Arg Ala Arg Glu Leu Gln Lys
               260                 265                 270
   Asp His Cys Glu Gln Arg Thr Gly Lys Met Glu Gln Met Lys Lys Lys
           275                 280                 285
   Phe Cys Val Leu Lys Lys Lys Leu Ser Glu Ala Lys Glu Ile Lys Ser
       290                 295                 300
   Gln Leu Glu Asn Gln Lys Val Lys Trp Glu Gln Glu Leu Cys Ser Val
   305                 310                 315                 320
   Arg Leu Thr Leu Asn Gln Glu Glu Glu Lys Arg Arg Asn Ala Asp Ile
                   325                 330                 335
   Leu Asn Glu Lys Ile Arg Glu Glu Leu Gly Arg Ile Glu Glu Gln His
               340                 345                 350
   Arg Lys Glu Leu Glu Val Lys Gln Gln Leu Glu Gln Ala Leu Arg Ile
           355                 360                 365
   Gln Asp Ile Glu Leu Lys Ser Val Glu Ser Asn Leu Asn Gln Val Ser
       370                 375                 380
   His Thr His Glu Asn Glu Asn Tyr Leu Leu His Glu Asn Cys Met Leu
   385                 390                 395                 400
   Lys Lys Glu Ile Ala Met Leu Lys Leu Glu Ile Ala Thr Leu Lys His
                   405                 410                 415
   Gln Tyr Gln Glu Lys Glu Asn Lys Tyr Phe Glu Asp Ile Lys Ile Leu
               420                 425                 430
   Lys Glu Lys Asn Ala Glu Leu Gln Met Thr Pro Arg Ala
           435                 440                 445
<210> 30
   <211> 578
   <212> DNA
   <213> Human
<400> 30
   cttgccttct cttaggcttt gaagcatttt tgtctgtgct ccctgatctt caggtcacca    60
   ccatgaagtt cttagcagtc ctggtactct tgggagtttc catctttctg gtctctgccc   120
   agaatccgac aacagctgct ccagctgaca cgtatccagc tactggtcct gctgatgatg   180
   aagcccctga tgctgaaacc actgctgctg caaccactgc gaccactgct gctcctacca   240
   ctgcaaccac cgctgcttct accactgctc gtaaagacat tccagtttta cccaaatggg   300
   ttggggatct cccgaatggt agagtgtgtc cctgagatgg aatcagcttg agtcttctgc   360
   aattggtcac aactattcat gcttcctgtg atttcatcca actacttacc ttgcctacga   420
   tatccccttt atctctaatc agtttatttt ctttcaaata aaaaataact atgagcaaca   480
   aaaaaaaaaa aaaaaaaaaa aaaaaaaaaa aaaaaaaaaa aaaaaaaaaa aaaaaaaaaa   540
   aaaaaaaaaa aaaaaaaaaa aaaaaaaaaa aaaaaaaa                           578
<210> 31
   <211> 90
   <212> PRT
   <213> Homo sapien
<400> 31
   Met Lys Phe Leu Ala Val Leu Val Leu Leu Gly Val Ser Ile Phe Leu
    1               5                  10                  15
   Val Ser Ala Gln Asn Pro Thr Thr Ala Ala Pro Ala Asp Thr Tyr Pro
               20                  25                  30
   Ala Thr Gly Pro Ala Asp Asp Glu Ala Pro Asp Ala Glu Thr Thr Ala
           35                  40                  45
   Ala Ala Thr Thr Ala Thr Thr Ala Ala Pro Thr Thr Ala Thr Thr Ala
       50                  55                  60
   Ala Ser Thr Thr Ala Arg Lys Asp Ile Pro Val Leu Pro Lys Trp Val
   65                  70                  75                  80
   Gly Asp Leu Pro Asn Gly Arg Val Cys Pro
                   85                  90
<210> 32
   <211> 3101
   <212> DNA
   <213> Homo sapien
<400> 32
   tgttggggcc tcagcctccc aagtagctgg gactacaggt gcctgccacc acgcccagct    60
   aattttttgt atatttttta gtagagacgg ggtttcaccg tggtctcaat ctcctgacct   120
   cgtgatctgc cagccttggc ctcccaaagt gtattctctt tttattatta ttattatttt   180
   tgagatggag tctgtctctg tcgcccaggc tggagtgcag tggtgcgatc tctgctcact   240
   gcaagctccg cctcctgggt tcatgccatt ctcctgcctc agcctcccga gtagctggga   300
   ctacaggccc ctgccaccac acccggctaa ttttttgtat ttttagtaga gacagggttt   360
   caccatgtta gccagggtgg tctctatctt ctgacctcgt gatccgcctg cctcagtctc   420
   tcaaagtgct gggattacag gcgtgagcca ccgcgaccag ccaactattg ctgtttattt   480
   ttaaatatat tttaaagaaa caattagatt tgttttcttt ctcattcttt tacttctact   540
   cttcatgtat gtataattat atttgtgttt tctattacct tttctccttt tactgtattg   600
   gactataata attgtgctca ctaatttctg ttcactaata ttatcagctt agataatact   660
   ttaattttta acttatatat tgagtattaa attgatcagt tttatttgta attatctatc   720
   ttccgcttgg ctgaatataa cttcttaagc ttataacttc ttgttctttc catgttattt   780
   ttttcttttt tttaatgtat tgaatttctt ctgacactca ttctagtaac ttttttctcg   840
   gtgtgcaacg taagttataa tttgtttctc agatttgaga tctgccataa gtttgaggct   900
   ttattttttt tttttatttg ctttatggca agtcggacaa cctgcatgga tttggcatca   960
   atgtagtcac ccatatctaa gagcagcact tgcttcttag catgatgagt tgtttctgga    1020
   ttgtttcttt attttactta tattcctggt agattcttat attttccctt caactctatt    1080
   cagcatttta ggaattctta ggactttctg agaattttag ctttctgtat taaatgtttt    1140
   taatgagtat tgcattttct caaaaagcac aaatatcaat agtgtacaca tgaggaaaac    1200
   tatatatata ttctgttgca gatgacagca tctcataaca aaatcctagt tacttcattt    1260
   aaaagacagc tctcctccaa tatactatga ggtaacaaaa atttgtagtg tgtaattttt    1320
   ttaatattag aaaactcatc ttacattgtg cacaaatttc tgaagtgata atacttcact    1380
   gtttttctat agaagtaact taatattggc aaaattactt atttgaattt aggttttggc    1440
   tttcatcata tacttcctca ttaacatttc cctcaatcca taaatgcaat ctcagtttga    1500
   atcttccatt taacccagaa gttaattttt aaaaccttaa taaaatttga atgtagctag    1560
   atattatttg ttggttacat attagtcaat aatttatatt acttacaatg atcagaaaat    1620
   atgatctgaa tttctgctgt cataaattca ataacgtatt ttaggcctaa acctttccat    1680
   ttcaaatcct tgggtctggt aattgaaaat aatcattatc ttttgttttc tggccaaaaa    1740
   tgctgcccat ttatttctat ccctaattag tcaaactttc taataaatgt atttaacgtt    1800
   aatgatgttt atttgcttgt tgtatactaa aaccattagt ttctataatt taaatgtcac    1860
   ctaatatgag tgaaaatgtg tcagaggctg gggaagaatg tggatggaga aagggaaggt    1920
   gttgatcaaa aagtacccaa gtttcagtta cacaggaggc atgagattga tctagtgcaa    1980
   aaaatgatga gtataataaa taataatgca ctgtatattt tgaaattgct aaaagtagat    2040
   ttaaaattga tttacataat attttacata tttataaagc acatgcaata tgttgttaca    2100
   tgtatagaat gtgcaacgat caagtcaggg tatctgtggt atccaccact ttgagcattt    2160
   atcgattcta tatgtcagga acatttcaag ttatctgttc tagcaaggaa atataaaata    2220
   cattatagtt aactatggcc tatctacagt gcaactaaac actagatttt attcctttcc    2280
   aactgtgggt ttgtattcat ttaccaccct cttttcattc cctttctcac ccacacactg    2340
   tgccgggcct caggcatata ctattctact gtctgtctct gtaaggatta tcattttagc    2400
   ttccacatat gagagaatgc atgcaaagtt tttctttcca tgtctggctt atttcactta    2460
   acaaaatgac ctccgcttcc atccatgtta tttatattac ccaatagtgt tcataaatat    2520
   atatacacac atatatacca cattgcattt gtccaattat tcattgacgg aaactggtta    2580
   atgttatatc gttgctattg tgaatagtgc tgcaataaac acgcaagtgg ggatataatt    2640
   tgaagagttt ttttgttgat gttccataca aattttaaga ttgttttgtc tatgtttgtg    2700
   aaaatggcgt tagtattttc atagagattg cattgaatct gtagattgct ttgggtaagt    2760
   atggttattt tgatggtatt aattttttca ttccatgaag atgagatgtc tttccatttg    2820
   tttgtgtcct ctacattttc tttcatcaaa gttttgttgt atttttgaag tagatgtatt    2880
   tcaccttata gatcaagtgt attccctaaa tattttattt ttgtagctat tgtagatgaa    2940
   attgccttct cgatttcttt ttcacttaat tcattattag tgtatggaaa tgttatggat    3000
   ttttatttgt tggtttttaa tcaaaaactg tattaaactt agagtttttt gtggagtttt    3060
   taagtttttc tagatataag atcatgacat ctaccaaaaa a                        3101
<210> 33
   <211> 16
   <212> DNA
   <213> Artificial Sequence
<220>
   <223> PCR primer
<400> 33
   tgcccctccg gaagct 16
<210> 34
   <211> 23
   <212> DNA
   <213> Artificial Sequence
<220>
   <223> PCR primer
<400> 34
   cgtttctgaa gggacatctg atc 23
<210> 35
   <211> 30
   <212> DNA
   <213> Artificial Sequence
<220>
   <223> PCR primer
<400> 35
   ttgcagccaa gttaggagtg aagagatgca 30
<210> 36
   <211> 24
   <212> DNA
   <213> Artificial Sequence
<220>
   <223> PCR primer
<400> 36
   aagcctcaga gtccttccag tatg 24
<210> 37
   <211> 35
   <212> DNA
   <213> Artificial Sequence
<220>
   <223> PCR primer
<400> 37
   ttcaaatata agtgaagaaa aaattagtag atcaa 35
<210> 38
   <211> 37
   <212> DNA
   <213> Artificial Sequence
<220>
   <223> PCR primer
<400> 38
   aatccattgt atcttagaac cgagggattt gtttaga 37
<210> 39
   <211> 22
   <212> DNA
   <213> Artificial Sequence
<220>
   <223> PCR primer
<400> 39
   aaagcagatg gtggttgagg tt 22
<210> 40
   <211> 22
   <212> DNA
   <213> Artificial Sequence
<220>
   <223> PCR primer
<400> 40
   cctgagacca aatggcttct tc 22
<210> 41
   <211> 24
   <212> DNA
   <213> Artificial Sequence
<220>
   <223> PCR primer
<400> 41
   attccatgcc ggctgcttct tctg 24
<210> 42
   <211> 30
   <212> DNA
   <213> Artificial Sequence
<220>
   <223> PCR primer
<400> 42
   tctggttttc tcattcttta ttcatttatt 30
<210> 43
   <211> 20
   <212> DNA
   <213> Artificial Sequence
<220>
   <223> PCR primer
<400> 43
   tgccaaggag cggattatct 20
<210> 44
   <211> 30
   <212> DNA
   <213> Artificial Sequence
<220>
   <223> PCR primer
<400> 44
   caaccacgtg acaaacactg gaattacagg 30
<210> 45
   <211> 21
   <212> DNA
   <213> Artificial Sequence
<220>
   <223> PCR primer
<400> 45
   actggaacgg tgaaggtgac a 21
<210> 46
   <211> 20
   <212> DNA
   <213> Artificial Sequence
<220>
   <223> PCR primer
<400> 46
   cggccacatt gtgaactttg 20
<210> 47
   <211> 23
   <212> DNA
   <213> Artificial Sequence
<220>
   <223> PCR primer
<400> 47
   cagtcggttg gagcgagcat ccc 23
<210> 48
   <211> 24
   <212> DNA
   <213> Artificial Sequence
<220>
   <223> PCR primer
<400> 48
   tgccatagat gaattgaagg aatg 24
<210> 49
   <211> 29
   <212> DNA
   <213> Artificial Sequence
<220>
   <223> PCR primer
<400> 49
   tgtcatatat taattgcata aacacctca 29
<210> 50
   <211> 32
   <212> DNA
   <213> Artificial Sequence
<220>
   <223> PCR primer
<400> 50
   tcttaaccaa acggatgaaa ctctgagcaa tg 32
<210> 51
   <211> 28
   <212> DNA
   <213> Artificial Sequence
<220>
   <223> PCR primer
<400> 51
   atcattgaaa attcaaatat aagtgaag 28
<210> 52
   <211> 30
   <212> DNA
   <213> Artificial Sequence
<220>
   <223> PCR primer
<400> 52
   gtagttgtgc attgaaataa ttatcattat 30
<210> 53
   <211> 20
   <212> DNA
   <213> Artificial Sequence
<220>
   <223> PCR Primer
<400> 53
   caattttggt ggagaacccg 20
<210> 54
   <211> 20
   <212> DNA
   <213> Artificial Sequence
<220>
   <223> PCR Primer
<400> 54
   gctgtcggag gtatatggtg 20
<210> 55
   <211> 28
   <212> DNA
   <213> Artificial Sequence
<220>
   <223> PCR Primer
<400> 55
   catttcagag agtaacatgg actacaca 28
<210> 56
   <211> 21
   <212> DNA
   <213> Artificial Sequence
<220>
   <223> PCR Primer
<400> 56
   tctgataaag gccgtacaat g 21
<210> 57
   <211> 22
   <212> DNA
   <213> Artificial Sequence
<220>
   <223> PCR Primer
<400> 57
   tcacgacttg ctgtttttgc tc 22
<210> 58
   <211> 30
   <212> DNA
   <213> Artificial Sequence
<220>
   <223> PCR Primer
<400> 58
   atcaaaaaac aagcatggcc tcacaccact 30
<210> 59
   <211> 21
   <212> DNA
   <213> Artificial Sequence
<220>
   <223> PCR Primer
<400> 59
   gcaagtgcca atgatcagag g 21
<210> 60
   <211> 23
   <212> DNA
   <213> Artificial Sequence
<220>
   <223> PCR Primer
<400> 60
   atatagactc aggtatacac act 23
<210> 61
   <211> 30
   <212> DNA
   <213> Artificial Sequence
<220>
   <223> PCR Primer
<400> 61
   tcccatcaga atccaaacaa gaggaagatg 30
<210> 62
   <211> 34
   <212> DNA
   <213> Artificial Sequence
<220>
   <223> PCR Primer
<400> 62
   aatccattgt atcttagaac cgagggattt gttt 34
<210> 63
   <211> 24
   <212> DNA
   <213> Artificial Sequence
<220>
   <223> PCR Primer
<400> 63
   ccgcttctga caacactaga gate 24
<210> 64
   <211> 32
   <212> DNA
   <213> Artificial Sequence
<220>
   <223> PCR Primer
<400> 64
   cctataaaga tgttatgtac caaaaatgaa gt 32
<210> 65
   <211> 22
   <212> DNA
   <213> Artificial Sequence
<220>
   <223> PCR Primer
<400> 65
   cccctccctc agggtatggc cc 22
<210> 66
   <211> 22
   <212> DNA
   <213> Artificial Sequence
<220>
   <223> PCR Primer
<400> 66
   ccctttctca cccacacact gt 22
<210> 67
   <211> 24
   <212> DNA
   <213> Artificial Sequence
<220>
   <223> PCR Primer
<400> 67
   tgcattctct catatgtgga agct 24
<210> 68
   <211> 33
   <212> DNA
   <213> Artificial Sequence
<220>
   <223> PCR Primer
<400> 68
   ccgggcctca ggcatatact attctactgt ctg 33
<210> 69
   <211> 24
   <212> DNA
   <213> Artificial Sequence
<220>
   <223> PCR Primer
<400> 69
   gacattccag ttttacccaa atgg 24
<210> 70
   <211> 23
   <212> DNA
   <213> Artificial Sequence
<220>
   <223> PCR Primer
<400> 70
   tgcagaagac tcaagctgat tcc 23
<210> 71
   <211> 28
   <212> DNA
   <213> Artificial Sequence
<220>
   <223> PCR Primer
<400> 71
   tctcagggac acactctacc attcggga 28
<210> 72
   <211> 30
   <212> DNA
   <213> Artificial Sequence
<220>
   <223> PCR Primer
<400> 72
   aaatataagt gaagaaaaaa attagtagat 30
<210> 73
   <211> 503
   <212> DNA
   <213> Homo sapiens
<400> 73
   gacagcggct tccttgatcc ttgccacccg cgactgaaca ccgacagcag cagcctcacc      60
   atgaagttgc tgatggtcct catgctggcg gccctctccc agcactgcta cgcaggctct     120
   ggctgcccct tattggagaa tgtgatttcc aagacaatca atccacaagt gtctaagact     180
   gaatacaaag aacttcttca agagttcata gacgacaatg ccactacaaa tgccatagat     240
   gaattgaagg aatgttttct taaccaaacg gatgaaactc tgagcaatgt tgaggtgttt     300
   ctgcaattaa tatatgacag cagtctttgt gatttatttt aactttctgc aagacctttg     360
   gctcacagaa ctgcagggta tggtgagaaa ccaactacgg attgctgcaa accacacctt     420
   ctctttctta tgtcttttta ctacaaacta caagacaatt gttgaaacct gctatacatg     480
   tttattttaa taaattgatg gca                                             503
<210> 74
   <211> 301
   <212> DNA
   <213> Homo sapiens
<400> 74
   cactgctacg caggctctgg ctgcccctta ttggagaatg tgatttccaa gacaatcaat      60
   ccacaagtgt ctaagactga atacaaagaa cttcttcaag agttcataga cgacaatgcc     120
   actacaaatg ccatagatga attgaaggaa tgttttctta accaaacgga tgaaactctg     180
   agcaatgttg aggtgtttat gcaattaata tatgacagca gtctttgtga tttatttggc     240
   ggccatcacc atcaccatca ctaaggtccc gagctcgaat tctgcagata tccatcacac     300
   t                                                                     301
<210> 75
   <211> 3282
   <212> DNA
   <213> Homo sapiens
<400> 75
   gggacagggc tgaggatgag gagaaccctg gggacccaga agaccgtgcc ttgcccggaa      60
   gtcctgcctg taggcctgaa ggacttgccc taacagagcc tcaacaacta cctggtgatt     120
   cctacttcag ccccttggtg tgagcagctt ctcaacatga actacagcct ccacttggcc     180
   ttcgtgtgtc tgagtctctt cactgagagg atgtgcatcc aggggagtca gttcaacgtc     240
   gaggtcggca gaagtgacaa gctttccctg cctggctttg agaacctcac agcaggatat     300
   aacaaatttc tcaggcccaa ttttggtgga gaacccgtac agatagcgct gactctggac     360
   attgcaagta tctctagcat ttcagagagt aacatggact acacagccac catatacctc     420
   cgacagcgct ggatggacca gcggctggtg tttgaaggca acaagagctt cactctggat     480
   gcccgcctcg tggagttcct ctgggtgcca gatacttaca ttgtggagtc caagaagtcc     540
   ttcctccatg aagtcactgt gggaaacagg ctcatccgcc tcttctccaa tggcacggtc     600
   ctgtatgccc tcagaatcac gacaactgtt gcatgtaaca tggatctgtc taaatacccc     660
   atggacacac agacatgcaa gttgcagctg gaaagctggg gctatgatgg aaatgatgtg     720
   gagttcacct ggctgagagg gaacgactct gtgcgtggac tggaacacct gcggcttgct     780
   cagtacacca tagagcggta tttcacctta gtcaccagat cgcagcagga gacaggaaat     840
   tacactagat tggtcttaca gtttgagctt cggaggaatg ttctgtattt cattttggaa     900
   acctacgttc cttccacttt cctggtggtg ttgtcctggg tttcattttg gatctctctc      960
   gattcagtcc ctgcaagaac ctgcattgga gtgacgaccg tgttatcaat gaccacactg    1020
   atgatcgggt cccgcacttc tcttcccaac accaactgct tcatcaaggc catcgatgtg    1080
   tacctgggga tctgctttag ctttgtgttt ggggccttgc tagaatatgc agttgctcac    1140
   tacagttcct tacagcagat ggcagccaaa gataggggga caacaaagga agtagaagaa    1200
   gtcagtatta ctaatatcat caacagctcc atctccagct ttaaacggaa gatcagcttt    1260
   gccagcattg aaatttccag cgacaacgtt gactacagtg acttgacaat gaaaaccagc    1320
   gacaagttca agtttgtctt ccgagaaaag atgggcagga ttgttgatta tttcacaatt    1380
   caaaacccca gtaatgttga tcactattcc aaactactgt ttcctttgat ttttatgcta    1440
   gccaatgtat tttactgggc atactacatg tatttttgag tcaatgttaa atttcttgca    1500
   tgccataggt cttcaacagg acaagataat gatgtaaatg gtattttagg ccaagtgtgc    1560
   acccacatcc aatggtgcta caagtgactg aaataatatt tgagtctttc tgctcaaaga    1620
   atgaagctcc aaccattgtt ctaagctgtg tagaagtcct agcattatag gatcttgtaa    1680
   tagaaacatc agtccattcc tctttcatct taatcaagga cattcccatg gagcccaaga    1740
   ttacaaatgt actcagggct gtttattcgg tggctccctg gtttgcattt acctcatata    1800
   aagaatggga aggagaccat tgggtaaccc tcaagtgtca gaagttgttt ctaaagtaac    1860
   tatacatgtt ttttactaaa tctctgcagt gcttataaaa tacattgttg cctatttagg    1920
   gagtaacatt ttctagtttt tgtttctggt taaaatgaaa tatgggctta tgtcaattca    1980
   ttggaagtca atgcactaac tcaataccaa gatgagtttt taaataatga atattattta    2040
   ataccacaac agaattatcc ccaatttcca ataagtccta tcattgaaaa ttcaaatata    2100
   agtgaagaaa aaattagtag atcaacaatc taaacaaatc cctcggttct aagatacaat    2160
   ggattcccca tactggaagg actctgaggc tttattcccc cactatgcat atcttatcat    2220
   tttattatta tacacacatc catcctaaac tatactaaag cccttttccc atgcatggat    2280
   ggaaatggaa gatttttttg taacttgttc tagaagtctt aatatgggct gttgccatga    2340
   aggcttgcag aattgagtcc attttctagc tgcctttatt cacatagtga tggggtacta    2400
   aaagtactgg gttgactcag agagtcgctg tcattctgtc attgctgcta ctctaacact    2460
   gagcaacact ctcccagtgg cagatcccct gtatcattcc aagaggagca ttcatccctt    2520
   tgctctaatg atcaggaatg atgcttatta gaaaacaaac tgcttgaccc aggaacaagt    2580
   ggcttagctt aagtaaactt ggctttgctc agatccctga tccttccagc tggtctgctc    2640
   tgagtggctt atcccgcatg agcaggagcg tgctggccct gagtactgaa ctttctgagt    2700
   aacaatgaga cacgttacag aacctatgtt caggttgcgg gtgagctgcc ctctccaaat    2760
   ccagccagag atgcacattc ctcggccagt ctcagccaac agtaccaaaa gtgatttttg    2820
   agtgtgccag ggtaaaggct tccagttcag cctcagttat tttagacaat ctcgccatct    2880
   ttaatttctt agcttcctgt tctaataaat gcacggcttt acctttcctg tcagaaataa    2940
   accaaggctc taaaagatga tttcccttct gtaactccct agagccacag gttctcattc    3000
   cttttcccat tatacttctc acaattcagt ttctatgagt ttgatcacct gattttttta     3060
   acaaaatatt tctaacggga atgggtggga gtgctggtga aaagagatga aatgtggttg    3120
   tatgagccaa tcatatttgt gattttttaa aaaaagttta aaaggaaata tctgttctga    3180
   aaccccactt aagcattgtt tttatataaa aacaatgata aagatgtgaa ctgtgaaata    3240
   aatataccat attagctacc caccaaaaaa aaaaaaaaaa aa                       3282
<210> 76
   <211> 463
   <212> DNA
   <213> Homo sapiens
<400> 76
   tagaattcag cggccgctta attctagaag tccaaatcac tcattgtttg tgaaagctga      60
   gctcacagca aaacaagcca ccatgaagct gtcggtgtgt ctcctgctgg tcacgctggc     120
   cctctgctgc taccaggcca atgccgagtt ctgcccagct cttgtttctg agctgttaga     180
   cttcttcttc attagtgaac ctctgttcaa gttaagtctt gccaaatttg atgcccctcc     240
   ggaagctgtt gcagccaagt taggagtgaa gagatgcacg gatcagatgt cccttcagaa     300
   acgaagcctc attgcggaag tcctggtgaa aatattgaag aaatgtagtg tgtgacatgt     360
   aaaaactttc atcctggttt ccactgtctt tcaatgacac cctgatcttc actgcagaat     420
   gtaaaggttt caacgtcttg ctttaataaa tcacttgctc tac                       463
<210> 77
   <211> 90
   <212> PRT
   <213> Homo sapiens
<400> 77
   Met Lys Leu Ser Val Cys Leu Leu Leu Val Thr Leu Ala Leu Cys Cys
    1               5                  10                  15
   Tyr Gln Ala Asn Ala Glu Phe Cys Pro Ala Leu Val Ser Glu Leu Leu
               20                  25                  30
   Asp Phe Phe Phe Ile Ser Glu Pro Leu Phe Lys Leu Ser Leu Ala Lys
           35                  40                  45
   Phe Asp Ala Pro Pro Glu Ala Val Ala Ala Lys Leu Gly Val Lys Arg
       50                  55                  60
   Cys Thr Asp Gln Met Ser Leu Gln Lys Arg Ser Leu Ile Ala Glu Val
   65                  70                  75                  80
   Leu Val Lys Ile Leu Lys Lys Cys Ser Val
                   85                  90